(19) Europäisches Patentamt
European Patent Office
Office européen des brevets

(11) Veröffentlichungsnummer: **0 096 654**
**B1**

(12) # EUROPÄISCHE PATENTSCHRIFT

(45) Veröffentlichungstag der Patentschrift: **28.10.87**

(21) Anmeldenummer: **83810216.8**

(22) Anmeldetag: **20.05.83**

(51) Int. Cl.⁴: **D 21 H 3/02,** C 07 C 69/00, C 07 C 93/18, C 07 C 101/04, C 07 C 103/00, C 07 C 121/417, C 07 C 127/15, C 07 C 141/02, C 07 C 143/14, C 07 C 143/44, C 07 D 251/34

(54) **Verfahren zum Leimen von Papier mit anionischen, hydrophoben Leimungsmitteln und kationischen Retentionsmitteln.**

(30) Priorität: **28.05.82 CH 3315/82**
**25.02.83 CH 1060/83**
**30.03.83 CH 1754/83**
**30.03.83 CH 1755/83**
**30.03.83 CH 1756/83**

(43) Veröffentlichungstag der Anmeldung:
**21.12.83 Patentblatt 83/51**

(45) Bekanntmachung des Hinweises auf die Patenterteilung:
**28.10.87 Patentblatt 87/44**

(84) Benannte Vertragsstaaten:
**AT BE CH DE FR GB IT LI NL SE**

(56) Entgegenhaltungen:
**EP - A - 0 037 379**
**DE - A - 1 621 698**
**DE - A - 2 459 165**
**DE - B - 1 012 165**
**DE - B - 1 030 671**
**DE - C - 916 927**
**FR - A - 895 568**
**FR - A - 900 239**
**FR - A - 900 508**
**US - A - 2 772 967**
**US - A - 3 796 735**
**US - A - 4 175 047**
**FETTE-SEIFEN-ANSTRICHMITTEL; 80. Jahrgang, Nr. 8, 1978 R. NEISSNER: "Herstellung, Analyse und DC-Trennung von Fettsäurepartialestern mehrwertiger Alkohole", Seiten 303-311.**
**FETTE-SEIFEN-ANSTRICHMITTEL; 79. Jahrgang, Nr. 3, 1977 R. NEISSNER: "Qualitative DC-Trennung von acetylierten Pentaerythritt-Fettsäureestern", Seiten 103-107.**
**TETRAHEDRON, Band 34, 1978 OXFORD (GB) Pergamon Press K.C. TANG et al.: "The synthesis of phosphonic acid and phosphate analogues of glycerol-3-phosphate and related metabolites", pages 2873-2878.**

(56) Entgegenhaltungen: (Fortsetzung)
**CHEMICAL ABSTRACTS, Band 90, Nr. 26, 25. Juni, 1979, Seite 405, Zusammenfassung 210202b COLUMBUS OHIO (US) V.S. KRIVICH: "Gas-chromatographic method for the separation of pentaerythritol esters".**
**CHEMICAL ABSTRACTS, Band 87, Nr. 8, 22. August 1977, Seite 75, Zusammenfassung 54604q COLUMBUS OHIO (US)**
**CHEMICAL ABSTRACTS, Band 70, Nr. 22, 2. Juni 1969, Seite 94, Zusammenfassung 98157z COLUMBUS OHIO (US) A.A. PETROV et al.: "Study of hydroxyethylated derivatives of glycerol and mono- and triethanolamine. II. Synthesis of polyglycol esters of glycerol and triethanolamine derivatives".**
**CHEMICAL ABSTRACTS, Band 91, Nr. 10, 3. September 1979, Seite 32, Zusammenfassung 75324w COLUMBUS OHIO (US)**
**CHEMICAL ABSTRACTS, Band 88, Nr. 26, 26. Juni 1978, Seite 42, Zusammenfassung 192092t COLUMBUS OHIO (US)**
**CHEMICAL ABSTRACTS, Band 91, Nr. 4, 23. Juli 1979, Seite 50, Zusammenfassung 21810h COLUMBUS OHIO (US)**
**CHEMICAL ABSTRACTS, Band 94, Nr. 2, 12. Januar 1981, Seite 476, Zusammenfassung 10740w COLUMBUS OHIO (US) M. SESTRIENKOVA et al.: "Analysis of pentaerythritol esters".**

(73) Patentinhaber: **CIBA-GEIGY AG, Klybeckstrasse 141, CH-4002 Basel (CH)**

(72) Erfinder: **Töpfl, Rosemarie, Dorneckstrasse 68, CH-4143 Dornach (CH)**
Erfinder: **Bernheim, Michael, Dr., Angensteinerweg 15, CH-4144 Arlesheim (CH)**
Erfinder: **Meindl, Hubert, Dr., Kornfeldstrasse 77, CH-4125 Riehen (CH)**
Erfinder: **Wegmüller, Hans, Dr., Kornfeldstrasse 18, CH-4125 Riehen (CH)**
Erfinder: **Rohringer, Peter, Sechsjuchartenstrasse 1, CH-4124 Schönenbuch (CH)**
Erfinder: **Werthemann, Dieter, Dr., Scherkesselweg 15, CH-4052 Basel (CH)**

**EP 0 096 654 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Der vorliegenden Erfindung liegt die Aufgabe zu Grunde, dem Papierhersteller leicht zugängliche und auf einfache Art erhältliche Leimungsmittel zur Verfügung zu stellen, die unter Mitverwendung von herkömmlichen, kationischen Retentionsmitteln geeignet sind, eine gute Leimung bei der Papierherstellung aus Faserstoffsuspensionen zu bewirken.

Diese Aufgabe wird erfindungsgemäss so gelöst, dass bei der Papierherstellung unter Mitverwendung von polymeren, kationischen Retentionsmitteln, wie z.B. Polyalkyleniminen, Leimungsmittel eingesetzt werden, die mindestens zwei langkettige, hydrophobe Substituenten und mindestens eine anionische oder acide (saure), gegebenenfalls als Salz vorliegende Gruppe aufweisen.

Aus DE-A-1 621 698 sind Leimungsmittel bekannt, die unter Mitverwendung eines Polyalkylenimins als polymeres, kationisches Retentionsmittel bei der Papierherstellung eingesetzt werden. Die bekannten Leimungsmittel weisen jedoch eine einzige hydrophobe Gruppe und keine anionische oder acide Gruppe auf. In ihrer bevorzugten Ausführungsform stellen die bekannten Leimungsmittel 2-Chlor-niederalkylen-sulfone, -sulfoxide oder -sulfide dar, die am Schwefelatom mit der hydrophoben Gruppe substituiert sind.

Gegenstand der vorliegenden Erfindung ist somit ein Verfahren zum Leimen von Papier oder Karton, bei welchem man zu wässrigen, gegebenenfalls füllmittelhaltigen Faserstoffsuspensionen mindestens
(A) ein Leimungsmittel
und
(B) ein polymeres, kationisches Retentionsmittel in beliebiger Reihenfolge oder gleichzeitig hinzugibt, welches dadurch gekennzeichnet ist, dass das erfindungsgemäss eingesetzte Leimungsmittel im Gegensatz zum bekannten Leimungsmittel als anionische oder acide, gegebenenfalls in Salzform vorliegende Gruppe mindestens eine saure Phosphat-, Sulfat-, Carboxyl-, Methylen- oder Methingruppe und mindestens zwei hydrophobe, nur Kohlenstoff- und Wasserstoffatome enthaltende Substituenten mit jeweils 5 bis 22 Kohlenstoffatomen enthält, wobei mindestens einer der hydrophoben Substituenten mindestens 8 Kohlenstoffatome oder vorzugsweise alle hydrophoben Gruppen jeweils mindestens 8 Kohlenstoffatome aufweisen, und mindestens zwei der nächst benachbarten hydrophoben Substituenten miteinander über ein Verbindungsglied verknüpft sind, das mindestens 2 Heteroatome und mindestens 1 Kohlenstoffatom aufweist.

Bevorzugt weisen die erfindungsgemäss verwendeten Leimungsmittel (A) 1 bis 6, vor allem 1 bis 4, vorzugsweise 1 oder 2, insbesondere eine anionische oder acide Gruppe auf, die in der Regel je eine oder zwei negative Ladungen enthalten. Beispielsweise weisen saure Phosphate

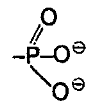 bzw. 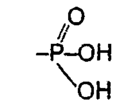

als acide Gruppen 2 negative Ladungen auf, während saure Sulfate $-SO_3^{\ominus}$ bzw. $-SO_3H$ oder Carboxylgruppen $-COO^{\ominus}$ bzw. COOH nur eine negative Ladung besitzen. In einer weiteren Ausführungsform weisen die Leimungsmittel (A) vorzugsweise 1 oder 2 potentiell anionische, acide Methylen oder Methingruppen auf. Die Fähigkeit der anionischen oder sauren Gruppen, in wässrigem Medium Anionen aufzuweisen oder bilden zu können, ist ein weiteres, wesentliches Kennzeichen des Leimungsmittels. Die Bildung von Anionen findet bei den üblicherweise bei der Papierherstellung vorliegenden pH-Werten der Faserstoffsuspensionen statt.

Bei den genannten Bedingungen können die kationischen Retentionsmittel (B) ebenfalls Kationen bilden. Die Fähigkeit der Leimungsmittel und der Retentionsmittel, bei den Bedingungen der Papierherstellung Anionen bzw. Kationen zu bilden, kann auch als anionaktiv bzw. kationaktiv bezeichnet werden. Somit können die anionischen Leimungsmittel und die kationischen Retentionsmittel auch anionaktive Leimungsmittel bzw. kationaktive Retentionsmittel genannt werden.

Als weiteres kennzeichnendes Merkmal weisen die Leimungsmittel (A) bevorzugt 2 bis 10, vorzugsweise 2 bis 6, insbesondere 2 oder 3 hydrophobe, nur Kohlenstoff- und Wasserstoffatome enthaltende Substituenten mit je 5 bis 22, vorzugsweise 8 bis 22, insbesondere 16 bis 20 Kohlenstoffatome auf, z.B. $C_5$–$C_{12}$-Cycloalkyl-, $C_6$–$C_{10}$-Aryl-, ferner -Alkaryl- oder -Aralkylreste auf. Bevorzugte Substituenten sind indessen offenkettige Alkyl- oder Alkenylreste, welche sich in der Regel von ungesättigten oder vor allem gesättigten Fettalkoholen, Fettaminen oder Fettsäuren mit 8 bis 22 Kohlenstoffatomen ableiten.

Die Verknüpfungsart, mit welcher diese hydrophoben Substituenten miteinander verbunden sind, bildet ein weiteres Kennzeichen der Leimungsmittel (A). Die Verbindungsglieder, über welche mindestens zwei der hydrophoben Substituenten verbunden sind, weisen nämlich in einer besonderen Ausführungsform der Leimungsmittel (A) vorzugsweise 4 bis 15 Kohlenstoffatome und mindestens 2 Heteroatome, vorzugsweise mindestens zwei Sauerstoff- und/oder Stickstoffatome auf.

Bei möglichen Carboxyl- oder Amidresten

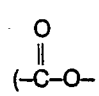 oder 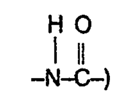

sind als Heteroatome beispielsweise ein Sauerstoff- oder ein Stickstoffatom Teil eines Verbindungsgliedes.

Je nach Anzahl der hydrophoben Substituenten enthalten die Leimungsmittel 1 bis 5, vorzugsweise 1 bis 3 Verbindungsglieder der angegebenen Art.

Bevorzugte Verbindungsglieder entsprechen im allgemeinen einer der Formeln

(1) $-Q_1-(O)_{n-1}-A_1-(O)_{m-1}-Q_2-$ oder

(2) $-O-A_2-O-$,

worin n und m je 1 oder 2, $A_1$ einen zweiwertigen, aliphatischen oder cycloaliphatischen Rest, $A_2$ einen zweiwertigen aromatischen Rest und $Q_1$ und $Q_2$ voneinander verschieden oder vorzugsweise gleich sind und,

sofern n und m 1 sind, $-NH-$, $-N<$, oder 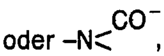,

oder, sofern n und m 2 sind, insbesondere 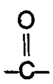 bedeuten, wobei n und m vorzugsweise für 2 stehen.

In Formel (1) bildet der Rest $A_1$ ein Teil eines aromatischen, vorzugsweise aliphatischen oder cycloaliphatischen Brückengliedes, welches vorzugsweise 1 oder 2, insbesondere eine anionische oder acide Gruppe und gegebenenfalls 1 bis 5, vorzugsweise 1, 2 oder 3 Stickstoffatome aufweist. Als Beispiele seien Brückenglieder der Formeln

(3) 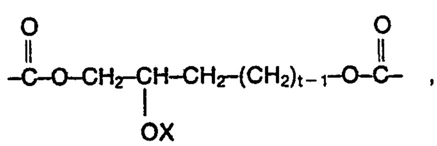,

(4) 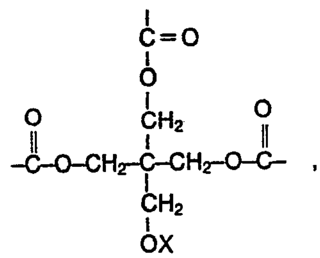,

(5) 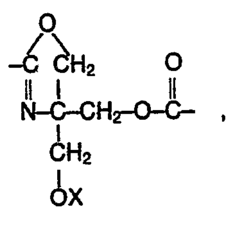,

(6) 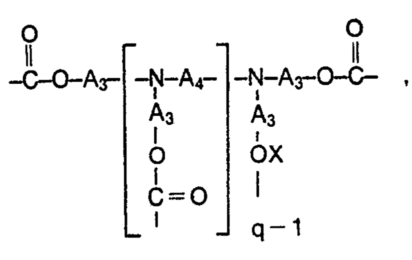,

(7) 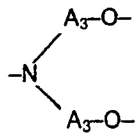

(8) 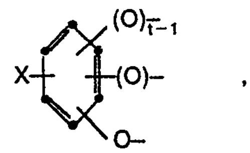,

(9) 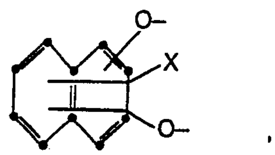,

(10) 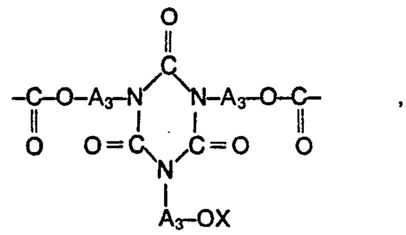,

(11) 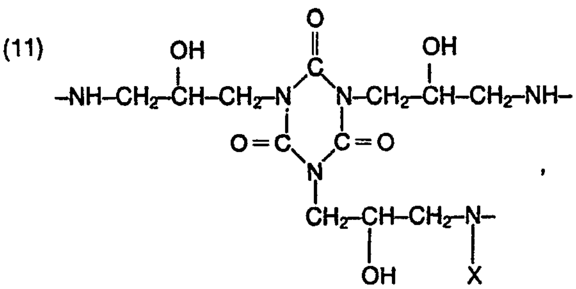,

(12) 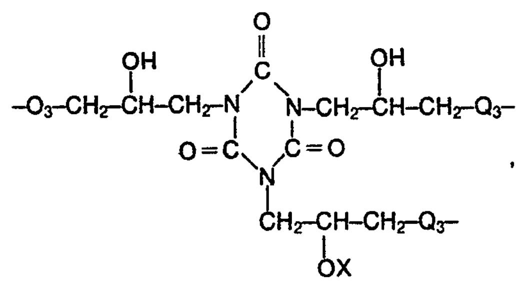,

(13) 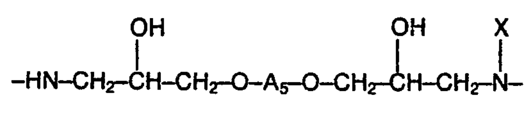

oder

(14) 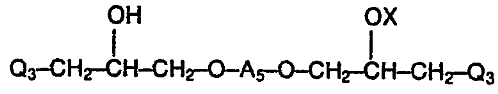

erwähnt, worin t 1 oder 2, q eine ganze Zahl von 1 bis 5, vorzugsweise 1 bis 3, $A_3$ und $A_4$ je Propylen, Isopropylen oder Äthylen, $A_5$ gegebenenfalls verzweigtes Alkylen mit 1 bis 6 Kohlenstoffatomen, X eine anionische oder acide Gruppe und

$Q_3$ 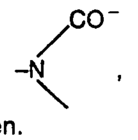, 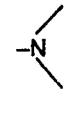 oder 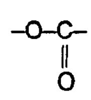

bedeuten.

Von besonderer Bedeutung als Leimungsmittel (A) sind solche, die durch chemische Umsetzung von

(a) einem aliphatischen Alkohol mit 3 bis 26 Kohlenstoffatomen und 2 bis 6, vorzugsweise 2 bis 4 Hydroxyl- oder Hydroxy-$C_1$–$C_4$-alkylgruppen, der gegebenenfalls 1 bis 5, vorzugsweise 1, 2 oder 3 Stickstoffatome und bei Anwesenheit von 2 Hydroxylgruppen gegebenenfalls einen $C_6$–$C_{22}$-, vorzugsweise $C_8$–$C_{22}$-, insbesondere $C_{16}$–$C_{20}$-Fettaminrest aufweist, einem heterocyclischen Alkohol oder Glycid mit vorzugsweise 3 Stickstoffatomen im Heteroring und 3 Hydroxy-$C_1$–$C_4$-alkyl- oder Glycidgruppen, einem Alkandioldiglycid mit 2 bis 6 Kohlenstoffatomen im Alkanrest oder einem Di- oder Triphenol oder einem Dihydroxynaphthalin, mit

(b) einer gegebenenfalls ungesättigten Fettsäure oder deren Halogeniden oder einem primären oder sekundären Fettamin mit 6 bis 22, vorzugsweise 8 bis 22, insbesondere 16 bis 20 Kohlenstoffatomen im Fettrest und hierauf mit

(c) einer mehrbasischen anorganischen oder organischen Säure mit 2 bis 18, vorzugsweise 4 bis 9 Kohlenstoffatomen oder deren Anhydriden erhältlich sind.

Als Beispiele von spezifischen Vertretern der Komponente (a), aus welcher das Leimungsmittel (A) erhältlich ist, seien als Dihydroxynaphthaline und Di- oder Triphenole, 1,5-, 1,8-, 2,3- und 2,7-Dihydronaphthalin, Pyrogallol, Hydroxyhydrochinon, Phloroglucin, Hydrochinon und vor allem Brenzcatechin und Resorcin, als heterocyclische Alkohole oder Glycide vor allem Tris(hydroxyäthyl)-isocyanurat und Isocyanursäuretriglycid, als Alkandioldiglycide vor allem Butan-1,4-dioldiglycid, als aliphatische Alkohole Sorbit, Sorbitan (d.h. cyclische Anhydrosorbite, erhalten aus Sorbit unter Abspaltung von Wasser), vor allem Butan-1,2,4-triol, Glycerin, Pentaerythrit, Tris(hydroxymethyl)-amino-methan, Trialkanolamine wie z.B. Triäthanolamin, $C_6$–$C_{22}$-Fettamindialkoxylate wie z.B. Laurylamindiäthoxylat und Polyhydroxyalkyl-polyalkylen-polyamine wie z.B. N,N,N',N'-Tetrakis(2-hydroxypropyl)-äthylendiamin genannt.

Aliphatische Alkohole sind als Komponente (a) gegenüber den Dihydroxynaphthalinen, den Di- oder Triphenolen, den heterocyclischen Alkoholen oder Glyciden oder den Alkandiodiglyciden bevorzugt.

Leimungsmittel (A) mit einem Brückenglied der Formel (3) sind aus Butan-1,2,4-triol oder Glycerin, mit einem Brückenglied der Formel (4) aus Pentaerythrit, mit einem Brückenglied der Formel (5) aus Tris-(hydroxymethyl)-amino-methan, mit einem Brückenglied der Formel (6) aus einem Fettamin-dialkoxylat oder Polyhydroxyalkyl-polyalkylen-polyamin, mit einem Brückenglied der Formel (7) aus einem Trialkanolamin, mit einem Brückenglied der Formel (8) aus einem Di- oder Triphenol, mit einem Brückenglied der Formel (9) aus einem Dihydroxynaphthalin, mit einem Brükkenglied der Formel (10) aus einem Tris-(hydroxyalkyl)-isocyanurat, mit einem Brückenglied der Formel (11) oder (12) aus Isocyanursäuretriglycid und mit einem Brückenglied der Formel (13) oder (14) aus einem Alkandioldiglycid als Komponente (a) erhältlich.

Als Komponente (b), aus welcher das Leimungsmittel (A) erhältlich ist, kommen vor allem gegebenenfalls ungesättigte Fettsäuren mit 6 bis 22, vorzugsweise 8 bis 22, insbesondere 16 bis 20 Kohlenstoffatomen, deren Halogenide oder, als sekundäre oder vor allem primäre Fettamine, Mono- oder Dialkylamine oder Mono- oder Dialkenylamine mit jeweils 6 bis 22, vorzugsweise 8 bis 22, insbesondere 16 bis 20 Kohlenstoffatomen im Alkyl- oder Alkenylrest, d.h. im Fettrest in Betracht. Bei den ungesättigten oder gesättigten $C_6$–$C_{22}$-, vorzugsweise $C_8$–$C_{22}$-, insbesondere $C_{16}$–$C_{20}$-Fettsäuren als Komponente (b) handelt es sich z.B. um Capron-, vorzugsweise Capryl-, Caprin- und Arachinsäure, insbesondere Laurin-, Myristin-, Palmitin-, Stearin- und Behensäure oder Myristolein, Palmitolein-, Elaeostearin-, Clupanodonsäure, insbesondere Öl-, Elaidin-, Eruka-, Linol- und Linolensäure. Hierbei kommt der Laurin-, Palmitin-, Stearin-, Öl- und Behensäure eine besondere Bedeutung zu, wobei Stearinsäure im Vordergrund des Interesses steht. Auch technische, gut zugängliche Gemische der soeben erwähnten Säuren kommen in Betracht. Die ungesättigten oder vorzugsweise gesättigten Fettsäurehalogenide, d.h. Alkyl- oder Alkenylhalogenide, die Mono- oder Dialkylamine oder Mono- oder Dialkenylamine, leiten sich strukturell von den soeben erwähnten Fettsäuren ab. Als Alkyl- oder Alkenylhalogenide kommen vor allem die Chloride oder insbesondere die Bromide in Betracht. Als spezifische Vertreter von Mono- oder Dialkylaminen mit $C_{16}$–$C_{20}$-Alkylresten oder von Alkylhalogeniden seien ihrer guten Zugänglichkeit wegen Dioctadecylamin, vor allem Octadecylamin und Octadecylbromid erwähnt. Auch technische Gemische der Fettamine bzw. der Alkylhalogenide der angegebenen Art kommen in Betracht.

Als Beispiele spezifischer Vertreter der Komponente (c) seien z.B. Trimellithsäureanhydrid, Glutarsäureanhydrid, vor allem Schwefeltrioxid, Schwefelsäure, Phosphorsäure, Phthalsäureanhydrid, insbesondere Chlorsulfonsäure, Phosphorpentoxyd, Bernsteinsäureanhydrid und Maleinsäureanhydrid erwähnt. Somit kommen für X in den Formeln (3) bis (14) vor allem die aciden Gruppen $-CO-C_6C_4-COOH$, $-CO-(CH_2)_2-COOH$, $-CO-CH=CH-COOH$,

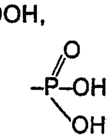

oder $-SO_3H$ in Frage.

Die Umsetzungsprodukte aus den Komponenten (a) und (b) stellen Zwischenprodukte dar, aus welchen die Leimungsmittel (A) unter Mitverwendung der Komponente (c) erhältlich sind. Ob eine Fettsäure, ein Fettamin, ein Fettsäureanhydrid oder ein Alkyl- oder Alkenylhalogenid als Komponente (b) eingesetzt werden muss, ergibt sich für den Fachmann aus der Art der eingesetzten Komponente (a). Bei Verwendung eines Di- oder Triphenols oder eines Dihydroxynaphthalins als Komponente (a) wird ein Alkyl- oder Alkenylhalogenid als Komponente (b) eingesetzt, während bei Verwendung eines aliphatischen oder heterocyclischen Alkohols als Komponente (a) in der Regel eine Fettsäure als Komponente (b) eingesetzt wird. Hingegen ist bei Verwendung eines heterocyclischen Glycids oder eines Alkandioldiglycids als Komponente (a) der Einsatz sowohl von Fettaminen als auch von Fettsäuren möglich.

Bei Verwendung eines Di- oder Triphenols oder eines Dihydroxynaphthalins als Komponente (a) werden vorzugsweise [h] Mol Alkyl- oder Alkenylhalogenid als Komponente (b) pro Mol Komponente (a) eingesetzt, wobei [h] die Anzahl Hydroxylgruppen der Komponente (a) bedeutet. Somit sind in diesem Fall hydroxylgruppenfreie aromatische Di- oder -triäther als Zwischenprodukte enthältlich, aus welchen durch Einsatz von 1 bis 2, vorzugsweise 1 bis 1,5, insbesondere 1 Mol Komponente (c) pro Mol Ausgangskomponente (a) die Leimungsmittel (A) erhältlich sind. Da die Umsetzung der Komponente (c) am aromatischen Kern der Fettsäuredi- oder -triäther stattfindet, empfiehlt es sich, als Komponente (c) Schwefelsäure, Schwefeltrioxyd oder insbesondere Chlorsulfonsäure einzusetzen. Demgemäss sind Leimungsmittel (A) in einer ihrer bevorzugten Ausführungsformen durch Umsetzung von mindestens

(a) 1 Mol eines Di- oder Triphenols oder eines Dihydroxynaphthalins mit

(b) [h] Mol eines Alkyl- oder Alkenylhalogenids mit 6 bis 22, vorzugsweise 8 bis 22, insbesondere 16 bis 20 Kohlenstoffatomen, wobei [h] die Anzahl Hydroxylgruppen der Komponente (a) bedeutet und hierauf mit

(c) 1 bis 2, vorzugsweise 1 bis 1,5, insbesondere 1 Mol Chlorsulfonsäure

erhältlich, wobei die Komponente (c) zuletzt eingesetzt wird.

Bei Verwendung eines heterocyclischen Glycids oder eines Alkandioldiglycids als Komponente (a) werden vorzugsweise [h'] Mol einer Fettsäure und/oder eines primären oder sekundären Fettamins eingesetzt, wobei [h'] die Anzahl Glycidgruppen der Komponente (a) bedeutet. Somit sind in diesem Fall heterocyclische Verbindungen oder Alkane als Zwischenprodukte erhältlich, welche

β-Hydroxy-γ-fettsäure-propylgruppen,
primäre oder sekundäre
β-Hydroxy-γ-fettamino-propylgruppen oder
β-Hydroxy-γ-bis(fettamino)-propylgruppen
oder sekundäre β-Hydroxy-γ-fettsäureamido-propylgruppen

aufweisen. Heterocyclische Verbindungen weisen als Zwischenprodukte vorzugsweise 3 solche Gruppen und Alkane als Zwischenprodukte 2 solche Gruppen auf, welche z.B. in den Brückengliedern der Formel (11) bis (14) enthalten sind. Aus solchen Zwischenprodukten sind Leimungsmittel (A) durch Einsatz von 1 bis 2, vorzugsweise 1 bis 1,5, insbesondere 1 Mol Komponente (c) pro Mol Ausgangskomponente (a) erhältlich, wobei alle vorstehend erwähnten, spezifischen Vertreter der mehrbasischen, anorganischen oder organischen Säuren mit 2 bis 8 Kohlenstoffatomen oder deren Anhydride in Frage kommen.

Sofern das Zwischenprodukt aus der Komponente (a) mit einem primären Fettamin als Komponente (b) erhältlich ist und deshalb primäre β-Hydroxy-γ-fettamino-propylgruppen aufweist, findet die Umsetzung der Komponente (c) im allgemeinen mit dem Wasserstoffatom des –NH-Restes solcher Gruppen statt. Falls hingegen das Zwischenprodukt aus der Komponente (a) mit einer Fettsäure oder einem sekundären Fettamin, d.h. einem Dialkyl- oder Dialkenylamin mit 6 bis 22 Kohlenstoffatomen, oder aus einem primären Fettamin, d.h. einem $C_6$–$C_{22}$-Alkyl- oder -Alkenylamin erhältlich ist, und sekundäre

β-Hydroxy-γ-fettsäureester-propylgruppen,
β-Hydroxy-γ-bis(fettamino)-propylgruppen oder
β-Hydroxy-γ-fettsäureamido-propylgruppen

aufweist, findet die Umsetzung der Komponente (c) im allgemeinen mit dem Wasserstoffatom des β-Hydroxylrestes solcher Gruppen statt.

Leimungsmittel (A) in einer weiteren bevorzugten Ausführungsform sind somit durch Umsetzung von mindestens

(a) 1 Mol eines heterocyclischen Glycids mit vorzugsweise 3 Stickstoffatomen im Heteroring und 3 Glycidgruppen, insbesondere Isocyanursäuretriglycid, oder eines Alkandioldiglycids mit vorzugsweise 2 bis 6 Kohlenstoffatomen im Alkanrest, insbesondere Butan-1,4-diol-diglycid, mit

(b) [h'] Mol einer Fettsäure oder eines primären oder sekundären Fettamins mit je 6 bis 22, vorzugsweise 8 bis 22, insbesondere 16 bis 20 Kohlenstoffatomen im Fettrest, wobei [h'] die Anzahl Glycidgruppen Komponente (a) bedeutet, und hierauf mit

(c) 1 bis 2, vorzugsweise 1 bis 1,5, insbesondere 1 Mol einer mehrbasischen, anorganischen oder organischen Säure mit 2 bis 8 Kohlenstoffatomen oder deren Anhydriden

erhältlich, wobei die Komponente (c) zuletzt eingesetzt wird.

Bei bevorzugter Verwendung eines heterocyclischen oder aliphatischen Alkohols als Komponente (a) werden vorzugsweise [h–l] Mol einer Fettsäure eingesetzt, wobei [h] die Anzahl Hydroxylgruppen der Komponente (a) bedeutet. Wird beispielsweise ein aliphatischer Alkohol mit 2 Hydroxylgruppen und einem Fettaminrest als Komponente (a) verwendet, so wird 1 Mol Fettsäure als Komponente (b) pro Mol Komponente (a) eingesetzt. Wird hingegen ein heterocyclischer Alkohol mit 3 Heteroatomen als Komponente (a) eingesetzt, so werden 2 Mol Fettsäure als Komponente (b) pro Mol Komponente (a) eingesetzt. Bei Verwendung von aliphatischen Alkoholen mit vorzugsweise 3 oder 4 Hydroxylgruppen als Komponente (a) werden 2 oder 3 Mol Fettsäure als Komponente (b) pro Mol Komponente (a) eingesetzt. Bei den aus den Komponenten (a) und (b) erhältlichen Zwischenprodukten handelt es sich, sofern Alkohole der angegebenen Art als Komponente (a) eingesetzt werden, um partiell veresterte Verbindungen, d.h. um partielle Ester, worin eine

unveresterte Hydroxylgruppe noch vorhanden ist. Aus diesen Produkten sind durch Einsatz von 1 bis 2 Mol, vorzugsweise 1 bis 1,5 Mol, insbesondere 1 Mol Komponente (c) pro Mol Ausgangskomponente (a) die Leimungsmittel (A) erhältlich, wobei die Komponente (c) mit der Hydroxylgruppe der Zwischenprodukte aus (a) und (b) saure Ester bildet und alle vorstehend erwähnten, spezifischen Vertreter der mehrbasischen anorganischen oder organischen Säuren mit 2 bis 8 Kohlenstoffatomen oder deren Anhydride als Komponente (c) einsetzbar sind.

In ihrer besonders bevorzugten Ausführungsart sind somit Leimungsmittel (A) durch Umsetzung von mindestens

(a) 1 Mol eines aliphatischen Alkohols, der 3 bis 26 Kohlenstoffatome, 2 bis 6, vorzugsweise 2 bis 4 Hydroxylgruppen und gegebenenfalls 1 bis 5, vorzugsweise 1, 2 oder 3 Stickstoffatome und, bei Anwesenheit von 2 Hydroxylgruppen, gegebenenfalls einen $C_6$–$C_{22}$-, vorzugsweise $C_8$–$C_{22}$-, insbesondere $C_{16}$–$C_{20}$-Fettaminrest aufweist, mit

(b) [h–l] Mol einer ungesättigten oder vorzugsweise gesättigten Fettsäure mit 6 bis 22, vorzugsweise 8 bis 22, insbesondere 16 bis 20 Kohlenstoffatomen oder deren Gemischen, wobei [h] die Anzahl Hydroxylgruppen der Komponente (a) bedeutet, und hierauf mit

(c) 1 bis 2, vorzugsweise 1 bis 1,5, insbesondere 1 Mol einer mehrbasischen, anorganischen oder organischen Säure mit 2 bis 18 Kohlenstoffatomen oder deren Anhydriden,

erhältlich, wobei die mehrbasische Säure als Komponente (c) zuletzt eingesetzt wird und mit den vorhandenen Hydroxylgruppen der Zwischenprodukte aus den Komponenten (a) und (b) saure Ester bildet.

Den sauren Estern als Leimungsmittel (A), die durch Umsetzung von Zwischenprodukten aus 1 Mol Laurylamindiäthoxylat und 1 Mol Behensäure, aus 1 Mol Glycerin, Triäthanolamin oder Tris-(hydroxymethyl)-amino-methan und 2 Mol Stearinsäure oder einem äquimolaren Gemisch aus Stearin- und Palmitinsäure oder aus 1 Mol Pentaerythrit oder N,N,N',N'-Tetrakis(2-hydroxypropyl)-äthylendiamin und 3 Mol Stearinsäure mit je 1 bis 1,5 Mol Chlorsulfonsäure, Phosphorpentoxid oder Maleinsäureanhydrid erhältlich sind, kommen eine besondere Bedeutung zu.

Im Vordergrund des Interesses stehen Leimungsmittel (A), die durch Umsetzung von

(a) 1 Mol Triäthanolamin oder insbesondere 1 Mol Glycerin mit
(b) 2 Mol Stearinsäure und hierauf mit
(c) 1 Mol Phosphorpentoxid

oder vor allem von

(a) 1 Mol Tris(hydroxymethyl)-amino-methan mit
(b) 2 Mol Stearinsäure und hierauf mit
(c) 1 bis 1,5 Mol Maleinsäureanhydrid

erhältlich sind.

Weitere Leimungsmittel von Interesse sind durch Umesterung von Tensiden auf Basis von Sulfobernsteinsäure, z.B. Sulfobernsteinsäureisooctylester, mit einem Fettalkohol erhältlich.

Bevorzugte, aus den Komponenten (a), (b) und (c) oder aus Tensiden der angegebenen Art erhältliche Leimungsmittel (A) weisen Molekulargewichte von etwa 400 bis 3000, vorzugsweise von etwa 600 bis 1500 und eine Säurezahl (mg KOH/g Substanz) von etwa 15 bis etwa 150, vorzugsweise etwa 35 bis etwa 125 auf.

Im allgemeinen werden die Verfahren zur Herstellung der Zwischenprodukte aus den Komponenten (a) und (b) und der sauren Ester durch weitere Umsetzung mit der Komponente (c) in Gegenwart von Lösungsmitteln durchgeführt, die sowohl jeder der Ausgangskomponenten (a), (b) und (c) als auch jedem der Zwischen- und Endprodukte, d.h. den Umsetzungsprodukten aus (a) und (b) und aus (a), (b) und (c) gegenüber inert sein müssen. Als Lösungsmittel kommen vor allem gegebenenfalls halogenierte Kohlenwasserstoffe in Betracht, die einen Siedepunkt von höchstens 140 °C aufweisen. Bei der Umsetzung der Komponenten (a) und (b) werden Kohlenwasserstoffe mit Siedepunkten zwischen etwa 110 °C und etwa 140 °C wie beispielsweise Toluol, Chlorbenzol, o-, m- und p-Xylol, ein technisches Xylolgemisch oder auch Gemische der erwähnten Kohlenwasserstoffe, bevorzugt. Bei der weiteren Umsetzung der Zwischenprodukte aus den Komponenten (a) und (b) mit der Komponente (c) kommen neben den Kohlenwasserstoffen der angegebenen Art auch tiefer siedende, z.B. bei etwa 40 °C bis etwa 80 °C siedende, vorzugsweise halogenierte Kohlenwasserstoffe, beispielsweise Dichloräthan oder Tetrachlorkohlenstoff, in Betracht. Als erhöhte Temperaturen bei der Umsetzung der Komponenten (a) und (b) eignen sich Temperaturen von 100 °C bis 140 °C und bei der weiteren Umsetzung mit der Komponente (c) die Raumtemperatur (15 bis 25 °C) bis erhöhte Temperaturen von etwa 40 bis 80 °C besonders gut.

Ferner ist es vorteilhaft, vor allem die Umsetzung der Komponenten (a) und (b) in Gegenwart eines Katalysators durchzuführen, der nötigenfalls die Veresterungsreaktion beschleunigt.

Als Katalysatoren kommen anorganische Säuren wie z.B. Salzsäure, Schwefelsäure, Phosphorsäure, vor allem organische Säuren wie z.B. organische Sulfonsäuren und insbesondere p-Toluolsulfonsäure in Betracht. Zweckmässig werden z.B. 1 bis 4% dieser Katalysatoren pro Mol der Komponente (a) eingesetzt.

Zur Verhinderung der Polymerisation bei der Herstellung saurer Ester mit reaktionsfähiger C=C Doppelbindung kann die Veresterung mit der Komponente (c) vorteilhaft in inerter Stickstoffatmosphäre vorgenommen werden. Dies trifft vor allem beim Einsatz besonders reaktionsfähiger Komponenten, wie z.B. Maleinsäure, zu. In diesem Fall oder auch beim Einsatz ungesättigter Komponenten (b), wie z.B. Ölsäure, ist es vorteilhaft, vor allem bei höheren Temperaturen, einen Polymerisationsinhibitor einzusetzen. Als Beispiele solcher Inhibitoren seien Methylenblau, Benzthiazin oder insbesondere Hydrochinon genannt. Zweckmässig werden z.B. 0,1 bis 0,3% dieser Inhibitoren pro Mol der ungesättigten Komponenten (b) oder (c) eingesetzt.

In einer anderen bevorzugten Ausführungsform enthalten die erfindungsgemäss verwendeten Leimungsmittel (A) neben z.B. 1 bis 4 sauren Phosphaten oder Sulfaten oder Carboxylgruppen als anionische oder acide Gruppen und vorzugsweise 2 bis 5, insbesondere 2 hydrophoben Substituenten der angegebenen Art zweiwertige Verbindungsglieder, über welche mindestens zwei der nächst benachbarten hydrophoben Substituenten verknüpft sind, die vorzugsweise 1 bis 15, insbesondere 2 bis 4 Kohlenstoffatome und mindestens 2 Heteroatome, vorzugsweise 2 bis 6, insbesondere 2 Stickstoffatome aufweisen. Je nach Anzahl der hydrophoben Substituenten enthalten solche Leimungsmittel 1 bis 5, vorzugsweise 1 bis 3, insbesondere 1 Verbindungsglied der angegebenen Art.

Bevorzugte Verbindungsglieder entsprechen in ihrer einfachsten Ausführungsform der Formel

(15) 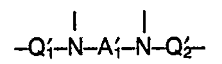

worin $A_1'$ Äthylen, Propylen oder Butylen und $Q_1'$ und $Q_2'$ je die direkte Bindung, –CO– oder –CO–NH– bedeuten.

Verbindungsglieder der Formel (15) sind Teile von Brückengliedern, welche mindestens eine an einem Stickstoffatom gebundene, anionische oder acide Gruppe aufweisen, und der Formel

(16) 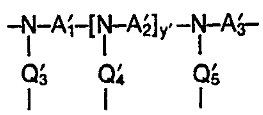

entsprechen, worin $A_1'$, $A_2'$ und $A_3'$ je Äthylen, Propylen oder Butylen, mindestens einer der Reste $Q_3'$, $Q_4'$ und $Q_5'$ –X', mindestens zwei der Reste $Q_3'$, $Q_4'$ und $Q_5'$ –CO–, –CO–NH– oder die direkte Bindung, X' eine anionische oder acide Gruppe der angegebenen Art oder einen, eine solche Gruppe tragenden Rest und y' eine ganze Zahl von 1 bis 5 bedeuten.

In Formel (16) sind 2 und vor allem 1 als Bedeutungen von y' bevorzugt. Zudem haben $A_1'$, $A_2'$ und $A_3'$ vorzugsweise die gleichen Bedeutungen und stehen vor allem für Propylen und insbesondere für Äthylen.

Brückenglieder, die im Vordergrund des Interesses stehen, entsprechen somit der Formel

(17) 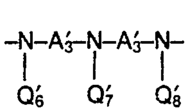 ,

worin $A_3'$ Äthylen oder Propylen sind und zwei der $Q_6'$, $Q_7'$ und $Q_8'$ Reste für –CO– oder –CO–NH– und einer der Reste $Q_6'$, $Q_7'$ und $Q_8'$ für –X' stehen, wobei –X' die angegebenen Bedeutungen hat.

Von besonderer Bedeutung als Leimungsmittel (A) sind solche, die durch chemische Umsetzung von

(a') einem gegebenenfalls mit N–$C_6$–$C_{22}$-Alkyl oder -Alkenyl mono-substituierten Polyalkylenpolyamin mit 3 bis 6 Stickstoffatomen und 4 bis 40 Kohlenstoffatomen mit

(b') einer Fettsäure oder einem Alkyl- oder Alkenylisocyanat mit mindestens 5, vorzugsweise 6 bis 22, vor allem 16 bis 20 Kohlenstoffatomen im Alkyl- oder Alkenylrest und hierauf

(c') mit dem Anhydrid einer mehrbasischen, vorzugsweise dreibasischen, insbesondere zweibasischen, anorganischen oder organischen Säure mit 2 bis 18, vorzugsweise 2 bis 8 Kohlenstoffatomen oder einer α- oder β-Halogencarbonsäure mit 2 bis 6 Kohlenstoffatomen oder einem Sulton erhältlich sind, wobei die Komponente (c') zuletzt eingesetzt wird.

Als Beispiele spezifischer Vertreter der Komponente (a'), aus welchen das Leimungsmittel erhältlich ist, seien N,N'-Bis(3-aminopropyl)-1,4-diamino-butan, N-(3-aminopropyl)-1,4-diaminobutan, 1,2-Bis(3-aminopropyl-amino)-äthan, Pentaäthylenhexamin, vor allem Tetraäthylenpentamin, Triäthylentetramin und insbesondere Dipropylentriamin und Diäthylentriamin genannt, wobei Diäthylentriamin im Vordergrund des Interesses steht.

Als Komponente (b'), aus welchem das Leimungsmittel (A) erhältlich ist, kommen vor allem gegebenenfalls ungesättigte Fettsäuren, Alkenyl- oder vor allem Alkylisocyanate mit 6 bis 22, vorzugsweise 8 bis 22, insbesondere 16 bis 20 Kohlenstoffatomen im Alkyl- oder Alkenylrest in Betracht. Bei den ungesättigten oder gesättigten $C_6$–$C_{22}$-, vorzugsweise $C_8$–$C_{22}$-, insbesondere $C_{16}$–$C_{20}$-Fettsäuren als Komponente (b) handelt es sich z.B. um Capron-, vorzugsweise Capryl-, Caprin-, Laurin-, Myristin- und Arachinsäure, insbesondere Palmitin-, Stearin- und Behensäure oder Myristolein-, Palmitolein-, Elaeostearin-, Clupanodonsäure, insbesondere Öl-, Elaidin-, Eruka-, Linol- und Linolensäuren. Hierbei kommt der Palmitin-, Stearin-, Öl- und Behensäure eine besondere Bedeutung zu, wobei Palmitin- und vor allem Stearinsäure im Vordergrund des Interesses stehen. Auch technische, gut zugängliche Gemische der soeben erwähnten Säuren kommen in Betracht. Als $C_{16}$–$C_{20}$-Alkylisocyanat sei seiner guten Zugänglichkeit wegen Octadecylisocyanat erwähnt.

Als Komponente (c') sind Anhydride organischer, mehrbasischer Säuren gegenüber denjenigen anorganischer, mehrbasischer Säuren bevorzugt. Als Beispiele spezifischer Vertreter der Anhydride anorganischer, mehrbasischer Säuren seien Schwefeltrioxyd, vor allem Phosphorpentoxyd und insbesondere Chlorsulfonsäure erwähnt. Bei den organischen, mehrbasischen Säureanhydriden handelt es sich beispielsweise um diejenigen der Benzophenontetracarbonsäure, 1,8-Naphthalsäure, Tri- und Pyromellithsäure, Bicyclo-(2,2,1)-hept-5-en-2,3-dicarbonsäure (auch Norbornendicarbonsäure oder «Nadic acid» genannt), Hexa- und Tetrahydrophthalsäure sowie Phthalsäure, Bernstein- und Glutarsäure, Dimethylmaleinsäure, Citracon- und Itaconsäure und insbesondere um Maleinsäure. Bei den Halogencarbonsäuren handelt es sich beispielsweise um

2-Chlorbuttersäure, 2- und 3-Chlorpropionsäure, Bromessigsäure oder Chloressigsäure und deren Alkalimetallsalze. Als Sulton findet vor allem Propansulton Verwendung. Im Vordergrund des Interesses stehen Glutar- und Bernsteinsäureanhydrid, Natriumchloracetat, Citracon- und Itaconsäureanhydrid, Phthalsäure- und Pyromellithsäureanhydrid und insbesondere Trimellithsäureanhydrid und Maleinsäureanhydrid, aus welchen besonders wirksame Leimungsmittel erhältlich sind.

Die Umsetzungsprodukte der Komponenten (a′) und (b′) stellen Zwischenprodukte dar, aus welchen die Leimungsmittel (A) durch weitere Umsetzung dieser Zwischenprodukte mit der Komponente (c′) erhältlich sind.

Die Umsetzungen der Komponenten (a′) und (b′) werden im allgemeinen in der Schmelze bei etwa 120 bis etwa 250 °C, vorzugsweise bis 200 °C durchgeführt. Bei Anwendung von hohen Temperaturen von etwa 200 bis 250 °C können nötigenfalls die erhaltenen Produkte mit Aktivkohle gereinigt werden. Vor allem bei Einsatz von Alkenyl- oder Alkylisocyanaten als Komponente (b′) oder bei der Umsetzung mit der Komponente (c′) ist es jedoch auch möglich, die Reaktionen in Gegenwart mindestens eines Lösungsmittels, das gegenüber jeder der Ausgangs-, Zwischen- und Endprodukte inert sein muss, vorzunehmen. Bei Einsatz solcher Lösungsmittel können die Umsetzungen auch bei tieferen Temperaturen, z.B. 30 bis 120 °C, durchgeführt werden. Als Beispiele möglicher Lösungsmittel seien Aceton, Dioxan oder gegebenenfalls halogenierte Kohlenwasserstoffe wie etwa Dichloräthan, Tetrachlorkohlenstoff, Benzol, Toluol, Chlorbenzol, o-, m- und p-Xylol, ein technisches Xylolgemisch oder auch Gemische der erwähnten Kohlenwasserstoffe erwähnt. Falls halogenierte Carbonsäuren, z.B. Chloressigsäure, als Komponente (c′) umgesetzt wird, empfiehlt es sich, zur Verhinderung der Bildung von Nebenprodukten eine etwa äquimolare Menge (bezogen auf die halogenierte Säure) einer schwachen, stickstoffhaltigen Base, z.B. Pyridin, Isochinolin, Chinolin oder vorzugsweise Triäthylamin, als Säureempfänger mitzuverwenden. Sofern ungesättigte Komponenten (c′) oder vor allem (b′) eingesetzt werden, ist es zudem vorteilhaft, die Umsetzung in inerter Stickstoffatmosphäre und/oder vor allem bei höheren Temperaturen, in Gegenwart eines Polymerisationsinhibitors, z.B. Methylenblau, Benthiazin oder vorzugsweise Hydrochinon, durchzuführen.

Bevorzugte Leimungsmittel (A) der angegebenen Art aus den Komponenten (a′), (b′) und (c′) weisen Molekulargewichte von 400 bis 3000, vorzugsweise etwa 500 bis etwa 3000, insbesondere etwa 600 bis etwa 1500 und infolge ihrer Gehalte an mindestens einer aciden Gruppe, z.B. einer Gruppe $-SO_3H$ oder $-COOH$, eine Säurezahl (mg KOH/g Substanz) von etwa 15 bis etwa 150, vorzugsweise etwa 50 bis etwa 120 auf.

In einer weiteren bevorzugten Ausführungsform enthalten die erfindungsgemäss verwendeten Leimungsmittel (A) neben 1 oder 2 aciden Methylen- oder Methingruppen, die als zwei- oder dreiwertige Reste $-CO-CH_2-CO-$ oder $-CO-\overset{|}{C}H-CO-$ vorliegen und 2 oder 3 hydrophoben Substituenten der angegebenen Art, zweiwertige Verbindungsglieder, über welche mindestens zwei der nächst benachbarten hydrophoben Substituenten verknüpft sind, die 1 bis 15, vorzugsweise 3 bis 8 Kohlenstoffatome und mindestens je 2 Heteroatome, vorzugsweise 2 bis 4 Stickstoff- und/oder Sauerstoffatome oder insbesondere 4 Stickstoffatome oder 2 Sauerstoffatome aufweisen. Verbindungsglieder mit jeweils 3 bis 5 Kohlenstoff- und zwei Sauerstoffatomen sind besonders bevorzugt. Je nach Anzahl der hydrophoben Substituenten enthalten solche Leimungsmittel vorzugsweise 1 oder 2 Verbindungsglieder der angegebenen Art.

Bevorzugte Verbindungsglieder entsprechen im allgemeinen einer der Formeln

(18) 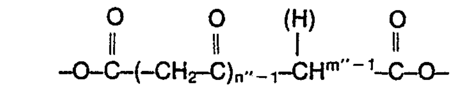

oder

(19) 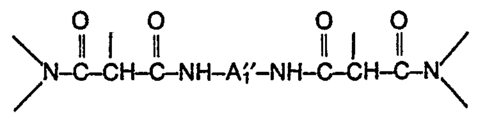 ,

worin n″ und m″ je 1 oder 2 und $A_1''$ verzweigtes oder vor allem geradkettiges Alkylen mit 4 bis 12, insbesondere 6 bis 10 Kohlenstoffatomen, Cycloalkylen mit 6 bis 14 Kohlenstoffatomen oder Arylen mit 6 bis 14, insbesondere 6 bis 8 Kohlenstoffatomen bedeuten. Sofern m″ in Formel (18) 1 ist, weist das Verbindungsglied an der entsprechenden Stelle eine Methingruppe auf. Falls aber m″ 2 ist, so weist das Verbindungsglied an der entsprechenden Stelle eine Methylengruppe auf.

In den Verbindungsgliedern der Formeln (18) oder (19) sind die Methylen- oder Methingruppen Teil eines zwei- bzw. dreiwertigen Restes $-CO-CH_2-CO-$ oder $-CO-\overset{|}{C}H-CO-$, wobei die direkte Verknüpfung der Methylengruppe jeweils zwischen zwei CO-Gruppen oder der Methingruppe zwischen 3 CO-Gruppen oder 2 CO-Gruppen und einer CN-Gruppe die aciden Eigenschaften dieser Methylen- oder Methingruppen bedingen.

Sofern n in Formel (18) für 2 steht, so bedeutet m″ in der Regel 2. 1 als Bedeutung von m″ gilt in der Regel nur insofern n″ auch 1 ist. Besonders bevorzugt ist jedoch das Verbindungsglied der Formel (18), worin n″ für 1 und m″ für 2 stehen.

Von besonderer Bedeutung als Leimungsmittel (A) sind vor allem solche, die durch chemische Umsetzung von

($a_1''$) Malonsäure, einem Malonsäuredihalogenid, Acetondicarbonsäure oder einem $C_1$–$C_4$-Alkylester der Malonsäure, Acetondicarbonsäure oder Methantricarbonsäure mit

($b_1''$) einem Fettalkohol

oder von

($a_2''$) Cyanessigsäure oder deren $C_1$–$C_4$-Alkylester mit

($b_2''$) einem Fettamin und hierauf mit

(c″) einem $C_4$–$C_{12}$-Alkylen-, $C_6$–$C_{14}$-Cycloalkylen- oder $C_6$–$C_{14}$-Arylendiisocyanat erhältlich sind.

Als Komponente ($a_1$″) aus welcher das Leimungsmittel (A) erhältlich ist, kommen vor allem die Dihalogenide, z.B. das Dibromid und insbesondere das Dichlorid der Malonsäure sowie deren Methyl- oder insbesonder Äthylester in Frage. Wegen ihrer grossen Reaktionsfähigkeit ist Maleinsäuredichlorid besonders bevorzugt. Ferner kommen ihrer guten Zugänglichkeit wegen auch der Dimethyl- und vor allem der Diäthylester der Acetondicarbonsäure oder der Malonsäure oder der Trimethyl- und vor allem Triäthylester der Methantricarbonsäure in Betracht.

Bei der Komponente ($a_2$″) handelt es sich vor allem um Cyanessigsäuremethylester und insbesondere um Cyanessigsäureäthylester.

Als Komponenten ($a_1$″) und ($a_2$″) stehen Malonsäuredichlorid und Cyanessigsäureäthylester im Vordergrund des Interesses.

Als Komponente ($b_1$″), aus welcher das Leimungsmittel (A) erhältlich ist, kommen vor allem gegebenenfalls ungesättigte, aliphatische Alkohole mit 6 bis 22, vorzugsweise 8 bis 22, insbesondere 16 bis 20 Kohlenstoffatomen und als Komponente ($b_1$″) vor allem Mono- oder Dialkylamine oder Mono- oder Dialkenylamine mit jeweils 6 bis 22, vorzugsweise 8 bis 22, insbesondere 16 bis 20 Kohlenstoffatomen im Alkyl- oder Alkenylrest in Betracht. Die gesättigten Fettalkohole und die Alkyl- oder Dialkylamine sind gegenüber den ungesättigten Fettalkoholen und den Alkenyl- oder Dialkenylaminen bevorzugt. Primäre Amine sind gegenüber sekundären Aminen ebenfalls bevorzugt. Als spezifische Vertreter von $C_{16}$–$C_{20}$ Fettalkoholen und von Mono- oder Dialkylaminen mit $C_{16}$–$C_{20}$ Alkylresten seien ihrer guten Zugänglichkeit wegen Hexadecanol, Octadecanol, Oleylalkohol, Octadecylamin und Dioctadecylamin erwähnt. Auch technische Gemische der Fettalkohole bzw. der Fettamine der angegebenen Art kommen in Betracht.

Bei den aliphatischen Diisocyanaten als Komponente (c″) handelt es sich um solche, die verzweigte oder vorzugsweise geradkettige Alkylenreste mit etwa 4 bis 12, insbesondere 6 bis 10 Kohlenstoffatome aufweisen. Als spezifische Vertreter solcher Diisocyanate seien Butylendiisocyanat, Dodecylendiisocyanat, vor allem Decylen-1,10-Diisocyanat und insbesondere Hexylen-1,6-Diisocyanat genannt. Bei den cycloaliphatischen Diisocyanaten handelt es sich um solche, die im allgemeinen 6 bis 14 Kohlenstoffatomen im Cycloalkylenrest aufweisen. Als Beispiele seien Cyclohexyl- oder Dicyclohexyl-diisocyanat genannt. Die aromatischen Diisocyanate weisen im allgemeinen 6 bis 14, vorzugsweise 6 bis 8 Kohlenstoffatome im Arylenrest auf. Als Beispiele spezifischer Vertreter seien
Naphthylen-1,5-diisocyanat,
Diphenylmethan-4,4′-diisocyanat,
Phenylen-1,4-diisocyanat und
Toluylen-2,4- und -2,6-diisocyanat
genannt. Ihrer guten Zugänglichkeit wegen seien Hexan-1,6-diisocyanat und Toluylen-2,4- oder 2,6-diisocyanat oder vor allem technische Gemische von Toluylen-2,4- und -2,6-diisocyanat genannt.

Im allgemeinen wird etwa 1 Mol Fettalkohol als Komponente ($b_1$″) pro funktionelle Gruppe der verwendeten Komponente ($a_1$″) eingesetzt. So werden in der Regel etwa 2 Mol Fettalkohol pro Mol Komponente ($a_1$″) eingesetzt, sofern die Komponente ($a_1$″) Malonsäure oder Acetondicarbonsäure oder deren Halogenide oder Ester ist, und etwa 3 Mol Fettalkohol pro Mol Komponente (a″) eingesetzt, sofern die Komponente ($a_1$″) Methan-tricarbonsäuremethyl- oder äthylester ist.

Falls die Komponenten ($a_2$″) und ($b_2$″) eingesetzt werden, so werden sie miteinander im allgemeinen in etwa äquimolaren Verhältnissen umgesetzt. Das erhaltene Cyanessigsäurefettamid stellt ein Zwischenprodukt dar, das mit der Komponente (c″) weiter umgesetzt wird, wobei etwa 0,5 Mol Alkylen-, Cycloalkylen- oder Arylendiisocyanat der angegebenen Art als Komponente (c″) pro Mol Zwischenprodukt aus etwa äquimolaren Mengen der Komponenten ($a_2$″) und ($b_2$″) eingesetzt werden.

Bevorzugt eingesetzte Leimungsmittel (A) der angegebenen Art aus den Komponenten ($a_1$″) und ($b_1$″) oder aus den Komponenten ($a_2$″), ($b_2$″) und (c″) weisen Molekulargewichte von etwa 400 bis etwa 3000, vorzugsweise von etwa 600 bis etwa 1500 auf.

Die Umsetzungen der vorstehend angegebenen Komponenten ($a_1$″), ($a_2$″), ($b_1$″), ($b_2$″) und (c″) werden im allgemeinen nach an sich bekannten Methoden durchgeführt. So werden z.B. die Umsetzungen der Komponenten ($a_1$″) mit ($b_1$″) oder ($a_2$″) mit ($b_1$″) gegebenenfalls in Schmelze bei etwa 30 bis etwa 250 °C, vorzugsweise etwa 40 bis 140 °C durchgeführt. Bei Anwendung von hohen Temperaturen von etwa 200 bis 250 °C können nötigenfalls die erhaltenen Produkte mit Aktivkohle gereinigt werden. Vor allem bei Einsatz von Säurehalogeniden als Komponente ($a_1$″) und im allgemeinen bei Umsetzen der Zwischenprodukte aus den Komponenten ($a_2$″) und ($b_2$″) mit der Komponente (c″) ist es jedoch vorteilhaft, die Umsetzungen in Gegenwart mindestens eines Lösungsmittels, das gegenüber jedem der Ausgangs-, Zwischen- und Endprodukte inert sein muss, vorzunehmen. Bei Einsatz solcher Lösungsmittel können die Umsetzungen auch bei tieferen Temperaturen, z.B. 30 bis 120 °C, vorzugsweise 30 bis 50 °C durchgeführt werden. Als Beispiele möglicher Lösungsmittel seien Aceton, Dioxan oder gegebenenfalls halogenierte Kohlenwasserstoffe wie etwa Dichloräthan, Tetrachlorkohlenstoff, Benzol, Toluol, Chlorbenzol, o-, m- und p-Xylol, ein technisches Xylolgemisch oder auch Gemische der erwähnten Kohlenwasserstoffe erwähnt, die sich besonders gut dazu eignen, als Reaktionsmedium zum Umsetzen von Säurehalogeniden als Komponente ($a_1$″) mit Fettalkoholen als Komponente ($b_1$″) eingesetzt zu werden. Bei Umsetzen von Zwischenprodukten aus ($a_2$″) und ($b_2$″) mit der Komponente (c″) eignet sich als weiteres Lösungsmittel vor allem Dimethylformamid oder Dimethylsulfoxyd besonders gut. Beim Umsetzen von freien Säuren als Komponenten ($a_1''$) oder ($a_2''$) mit Fettalkoholen oder Fettaminen als Komponenten ($b_1''$) oder ($b_2''$) kann der Einsatz eines Katalysators, z. B. Salzsäure, Schwefelsäure, Phosphorsäure oder organischen Sulfonsäuren, vorzugsweise p-Toluolsulfonsäure, um nötigenfalls die Veresterungs- oder Umesterungsreaktionen zu beschleunigen, vorteilhaft sein. Sofern ungesättigte Komponenten ($b_1''$) und ($b_2''$) eingesetzt werden, ist es zudem vorteilhaft, die Umsetzung in inerter Stickstoffatmosphäre und/oder, vor allem bei hohen Temperaturen z. B. über 90 °C, in Gegenwart eines Polymerisationsinhibitors, z. B. Methylenblau, Benzthiazin oder vorzugsweise Hydrochinon, durchzuführen.

In einer anderweitigen, ebenfalls bevorzugten Ausführungsart enthalten die erfindungsgemäss verwendeten Leimungsmittel (A) neben 1 bis 6, vorzugsweise 1 bis 4, insbesondere 1 oder 2 $-COO^{\ominus}$ oder –COOH-Gruppen als anionische oder acide Gruppen und 2 bis 10, vorzugsweise 2 bis 6 hydrophoben Substituenten der angegebenen Art zweiwertige Verbindungsglieder, über welche mindestens zwei der nächst benachbarten hydrophoben Substituenten verknüpft sind, die vor allem 4 bis 15 Kohlenstoffatome und mindestens je 2 Heteroatome, vorzugsweise ein Stickstoff- und ein Sauerstoffatom oder insbesondere 2 Stickstoffatome oder 2 Sauerstoffatome aufweisen. Verbindungsglieder mit jeweils 4 bis 10 Kohlenstoff- und zwei Sauerstoffatomen sind besonders bevorzugt. Je nach Anzahl der hydrophoben Substituenten enthalten solche Leimungsmittel 1 bis 5, vorzugsweise 1 bis 3 Verbindungsglieder der angegebenen Art.

Bevorzugte Verbindungsglieder entsprechen im allgemeinen einer der Formeln

(20) 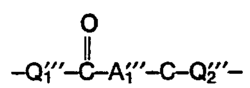 oder

(21) 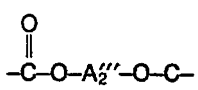 ,

worin $A_1'''$ und $A_2'''$ je einen zweiwertigen, aliphatischen oder aromatischen Rest, $Q_1'''$ und $Q_2'''$ voneinander verschieden oder vorzugsweise gleich sind und –O–, –NH– oder –N< bedeuten.

In Formeln (20) und (21) bilden die Reste $A_1'''$ und $A_2'''$ ein Teil eines aliphatischen oder aromatischen Brückengliedes, welches 1 bis 6, vorzugsweise 1 bis 4, insbesondere 1 oder 2 anionische oder acide Gruppen und gegebenenfalls 1 Stickstoffatom aufweist.

Als spezifische Beispiele bevorzugter Brükkenglieder seien diejenigen einer der Formeln

(22) 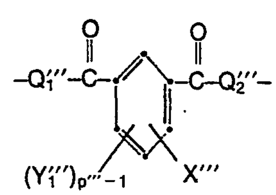 ,

(23) 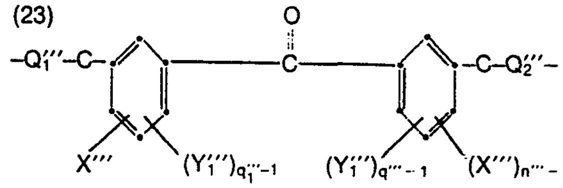 ,

(24) 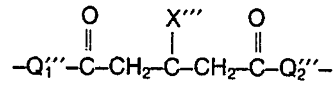 ,

(25) 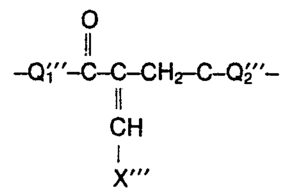 ,

(26) 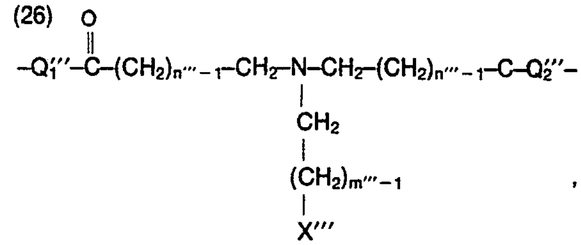 ,

(27) 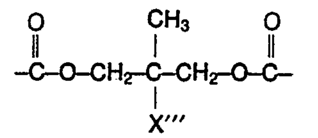 ,

(28) 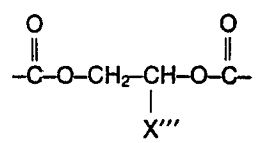 ,

(29) 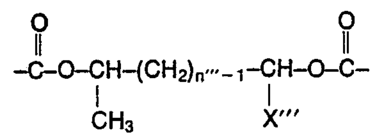 oder

(30) 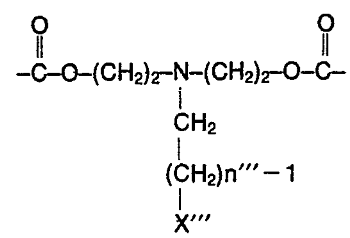

erwähnt, worin $Q_1'''$ und $Q_2'''$ je –O–, –NH– oder –N<, X''' –COOH, $-COO^{\ominus}$, $Y_1'''$ –COOH, $-COO^{\ominus}$ oder

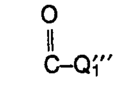 ,

Z''' Wasserstoff, Methyl oder vorzugsweise Hydroxyl, n''' und m''' je 1 oder 2, p''' eine ganze Zahl von 1 bis 4 und q''' und $q_1'''$ je 1, 2 oder 3 bedeuten.

Von besonderer Bedeutung als Leimungsmittel (A) sind vor allem solche, die durch chemische Umsetzung von mindestens

(a''') einer aliphatischen oder aromatischen Carbonsäure mit mindestens 3 Carboxylgruppen, einer Polyalkylenpolyamino-Polyessigsäure mit 4 bis 6 Carboxylgruppen oder einer aliphatischen Monocarbonsäure oder Aminocarbonsäure mit 2 Hydroxylgruppen mit

(b''') einem Fettalkohol und/oder einem Fettamin oder einer Fettsäure erhältlich sind, wobei Fettalkohole und/oder Fettamine als Komponente (b''') bei Verwendung von den Carbonsäuren mit mindestens 3, bzw. 4 bis 6 Carboxylgruppen als Komponente (a''') und Fettsäuren als Komponente (b''') bei Verwendung von Monocarbonsäuren als Komponente (a''') eingesetzt werden.

In der Regel stellen die so erhältlichen Leimungsmittel aliphatische oder aromatische Fettsäureester oder Fettsäureamide dar. Bevorzugte Ester oder Amide dieser Art weisen Brückenglieder einer der Formeln (22) bis (26) auf, worin $Q_1'''$ und $Q_2'''$ gleich sind. Weitere Leimungsmittel liegen jedoch auch als Amidester vor, die sowohl aus Fettalkoholen als auch aus Fettaminen und aus aliphatischen oder aromatischen Säuren mit mindestens 3 Carboxylgruppen, bzw. 4 bis 6 Carboxylgruppen, erhältlich sind. Bevorzugte Amidester dieser Art weisen Brückenglieder einer der Formeln (22) bis (26) auf, worin $Q_1'''$ und $Q_2'''$ voneinander verschieden sind. Sofern Monocarbonsäuren mit 2 Hydroxylgruppen als Komponente (a''') eingesetzt werden, kommen nur Fettsäuren als Komponente (b''') in Frage, wobei Fettsäureester erhältlich sind, die in ihrer bevorzugten Ausführungsform Brückenglieder einer der Formeln (27) bis (30) aufweisen.

Bevorzugt eingesetzte Leimungsmittel (A) der angegebenen Art aus den Komponenten (a''') und (b''') weisen Molekulargewichte von 400 bis etwa 3000, vorzugsweise von etwa 600 bis etwa 1500 auf. Leimungsmittel mit einem Brückenglied einer der Formeln (22) oder (24) sind besonders bevorzugt.

Insbesondere sind Leimungsmittel, die im Vordergrund des Interesses stehen, durch Umsetzung von mindestens

(a''') einer aromatischen Säure, die 9 bis 20 Kohlenstoffatome und 3 bis 6 Carboxylgruppen aufweist, einer aliphatischen Säure, die 3 Carboxylgruppen und gegebenenfalls ein Stickstoffatom aufweist, einer Polyalkylenpolyamino-Polyessigsäure die 4 bis 6 Carboxylgruppen und 2 bis 4 Stickstoffatome aufweist, einer aliphatischen Dihydroxymonocarbonsäure oder Bis(hydroxylalkyl)-amino-monocarbonsäure, die 3 bis 6 Kohlenstoffatome aufweist, oder deren Halogeniden oder Anhydriden, mit

(b''') einem Fettalkohol und/oder einem primären oder sekundären Fettamin oder sofern eine Monocarbonsäure der angegebenen Art als Komponente (a''') eingesetzt wird, aus einer Fettsäure, wobei die Fettreste der Fettalkohole, Fettamine und Fettsäuren ungesättigt oder vorzugsweise gesättigt sind und 6 bis 22, vorzugsweise 8 bis 22, insbesondere 16 bis 20 Kohlenstoffatome aufweisen, erhältlich.

Damit wie eingangs erwähnt, die Leimungsmittel mindestens eine $-COO^{\ominus}$ oder $-COOH$ Gruppe als anionische oder acide Substituenten aufweisen und das Verbindungsglied, über welches die hydrophoben Substituenten miteinander direkt verknüpft sind, mindestens 2 Heteroatome enthält, sind die Leimungsmittel in ihrer bevorzugten Ausführungsart aus 1 Mol der Komponente (a''') und 2 bis (h'''–l) Mol der Komponente (b''') erhältlich, wobei h''' die Anzahl Carboxylgruppen der Komponente (a''') bedeutet, sofern als Komponente (a''') Carbonsäuren mit mindestens 3, bzw. 4 bis 6 Carboxylgruppen und als Komponente (b''') Fettalkohole und/oder Fettamine eingesetzt werden. Beim Einsatz von 1 Mol Monocarbonsäure mit 2 Hydroxylgruppen als Komponente (a''') werden hingegen in der Regel 2 Mol Fettsäure eingesetzt.

Infolge der Gegenwart mindestens einer $-COO^{\ominus}$ oder $-COOH$ Gruppe in den Leimungsmitteln, weisen letztere eine Säurezahl (mg KOH/g Substanz) von etwa 15 bis etwa 150, vorzugsweise von etwa 40 bis 100 auf.

Als Carbonsäuren mit mindestens 3 Carboxylgruppen für die Komponente (a'''), aus welcher das Leimungsmittel (A) erhältlich ist, kommen vor allem aromatische einkernige Polycarbonsäuren, z. B.
Hemimellith-, Trimellith-, Trimesin-, Prehnit-,
Mellophan-, Pyromellith- oder Mellithsäure,
Benzolpentacarbonsäure,
aromatische zweikernige Polycarbonsäuren,
z. B.
Benzophenontri- bis -hexacarbonsäure
oder Anhydride der genannten aromatischen Carbonsäuren,
aliphatische Tricarbonsäuren, z. B.
Tricarballyl-, Aconit- oder Citronensäure,
aliphatische Tricarbonsäuren,
die ein Stickstoffatom aufweisen, z. B.
Bis(2-carboxyäthyl)-carboxymethyl-amin,
Nitrilotripropionsäure und Nitrilotriessigsäure,
und Polyalkylenamino-polyessigsäuren, z. B.
Triäthylen-tetramin-hexaessigsäure,
Diäthylentriaminpentaessigsäure,
Dipropylentriamin-pentaessigsäure,
Äthylendiamin-tetraessigsäure,
N,N'-Bis(2-carboxyäthyl)-N,N'-bis(carboxymethyl)-äthylendiamin,
Tris[2-bis(carboxymethyl-amino)-ethyl]-amin,
Bis[2-bis(carboxymethylamino)-ethyl]-methylamin oder
N,N'-Bis[2-bis(carboxymethyl-amino)-äthyl]-N,N'-dimethyl-äthylendiamin
in Betracht. Wegen ihrer leichten Zugänglichkeit sind hierbei
Trimellithsäureanhydrid,
Pyromellithsäuredianhydrid,
Hemimellithsäure,
Benzophenontetracarbonsäuredianhydrid,
Tricarballylsäure, trans-Aconitsäure,
Citronensäure oder
Nitrilotriessigsäure
besonders bevorzugt. Als aliphatische Monocar-

bonsäuren für die Komponente (a''') kommen z. B.
Glycerinsäure, 1,2-Dihydroxybuttersäure,
1,3- und 2,3-Dihydroxyvaleriansäure,
2,2-Bis(hydroxymethyl)-propionsäure,
N,N-Bis(hydroxyäthyl)-β-alanin und
N,N-Bis(hydroxyäthyl)-glycin
in Frage, wobei 2,2-Bis(hydroxymethyl)-propionsäure und
N,N-Bis(hydroxyäthyl)-glycin
bevorzugt sind. Im Vordergrund des Interesses stehen Trimellithsäureanhydrid, Pyromellithsäuredianhydrid und Citronensäure.

Sofern Polyalkylenpolyamino-polyessigsäuren als Komponente (a''') eingesetzt werden, sind daraus oligomere Leimungsmittel erhältlich. Beim Einsatz der übrigen Säuren als Komponente (a''') sind hingegen monomere Leimungsmittel erhältlich, welche gegenüber den oligomeren Leimungsmitteln auch bevorzugt sind.

Leimungsmittel (A) mit einem Brückenglied der Formel (22) sind z.B. aus den genannten aromatischen einkernigen Polycarbonsäuren, mit einem Brückenglied der Formel (23) aus einer Benzophenontri-bis-hexacarbonsäure, mit einem Brückenglied der Formel (24) aus der Tricarballyl- oder Citronensäure, mit einem Brückenglied der Formel (25) aus der Aconitsäure mit einem Brückenglied der Formel (26) aus aliphatischen Tricarbonsäuren, die ein Stickstoffatom aufweisen, mit einem Brückenglied der Formel (27) aus der 2,2-Bis-(hydroxymethyl)-propionsäure, mit einem Brückenglied der Formel (28) aus der Glycerinsäure, mit einem Brückenglied der Formel (29) aus der 1,3-Dihydroxyvaleriansäure oder der 1,2-Dihydroxybuttersäure und mit einem Brückenglied der Formel (30) aus N,N-Bis(hydroxyäthyl)-glycerin als Komponente (a''') erhältlich.

Als Komponente (b'''), aus welchen das Leimungsmittel (A) erhältlich ist, kommen vor allem gegebenenfalls ungesättigte, aliphatische Fettsäuren und Alkohole mit 6 bis 22, vorzugsweise 8 bis 22, insbesondere 16 bis 20 Kohlenstoffatomen oder Mono- oder Dialkylamine oder Mono- oder Dialkenylamine mit jeweils 6 bis 22, vorzugsweise 8 bis 22, insbesondere 16 bis 20 Kohlenstoffatomen im Alkyl- oder Alkenylrest in Betracht. Bei den ungesättigten oder gesättigten $C_6$–$C_{22}$-, vorzugsweise $C_8$–$C_{22}$-, insbesondere $C_{16}$–$C_{20}$-Fettsäuren als Komponente (b''') handelt es sich z.B. um Capron-, vorzugsweise Capryl-, Caprin-, Laurin-, Myristin- und Arachinsäure, insbesondere Palmitin-, Stearin- und Behensäure oder Myristolein-, Palmitolein-, Elaeostearin-, Clupanodonsäure, insbesonder Öl-, Elaidin-, Eruka-, Linol- und Linolensäuren. Hierbei kommt der Palmitin-, Stearin-, Öl- und Behensäure eine besondere Bedeutung zu, wobei Palmitin- und vor allem Stearinsäure im Vordergrund des Interesses stehen. Auch technische, gut zugängliche Gemische der soeben erwähnten Säuren kommen in Betracht. Die ungesättigten oder gesättigten Fettalkohole und die ungesättigten oder vorzugsweise gesättigten Mono- oder Dialkylamine oder Mono- oder Dialkenylamine leiten sich strukturell von den soeben erwähnten Fettsäuren ab. Als spezifische Vertreter von $C_{16}$–$C_{20}$ Fettalkoholen und von Mono- oder Dialkylaminen mit $C_{16}$–$C_{20}$ Alkylresten seien ihrer guten Zugänglichkeit wegen Hexadecanol, Octadecanol, Oleylalkohol, Octadecylamin und Dioctadecylamin erwähnt. Auch technische Gemische der Fettalkohole bzw. der Fettamine der angegebenen Art kommen in Betracht.

Die Umsetzungen der Komponenten (a''') und (b''') werden im allgemeinen in Schmelze bei etwa 120 bis etwa 250 °C, vorzugsweise bis 200 °C durchgeführt. Bei Anwendung von hohen Temperaturen von etwa 200 bis 250 °C können nötigenfalls die erhaltenen Produkte mit Aktivkohle gereinigt werden. Vor allem bei Einsatz von Säureanhydriden ist es jedoch auch möglich, die Umsetzungen in Gegenwart mindestens eines Lösungsmittels, das gegenüber jedem der Ausgangs- und Endprodukte inert sein muss, vorzunehmen. Bei Einsatz solcher Lösungsmittel können die Umsetzungen auch bei tieferen Temperaturen, z.B. 30 bis 120 °C, durchgeführt werden. Als Beispiele möglicher Lösungsmittel seinen Aceton, Dioxan oder gegebenenfalls halogenierte Kohlenwasserstoffe wie etwa Dichloräthan, Tetrachlorkohlenstoff, Benzol, Toluol, Chlorbenzol, o-, m- und p-Xylol, ein technisches Xylolgemisch oder auch Gemische der erwähnten Kohlenwasserstoffe erwähnt. Beim Umsetzen von beispielsweise Diolen mit Fettsäuren kann der Einsatz eines Katalysators, z.B. Salzsäure, Schwefelsäure, Phosphorsäure oder organischen Sulfonsäuren, vorzugsweise p-Toluolsulfonsäure, um nötigenfalls die Veresterungsreaktionen zu beschleunigen, vorteilhaft sein. Falls Polycarbonsäuren mit primären Fettaminen umgesetzt werden, empfiehlt es sich, zur Verhinderung der Bildung von Nebenprodukten eine etwa äquimolare Menge (bezogen auf das primäre Fettamin) einer schwachen, stickstoffhaltigen Base, z.B. Pyridin, Triäthylamin, Isochinolin oder vorzugsweise Chinolin mitzuverwenden. Sofern ungesättigte Fettsäuren, Alkohole oder Fettamine eingesetzt werden, ist es zudem vorteilhaft, die Umsetzung in inerter Stickstoffatmosphäre und/oder in Gegenwart eines Polymerisationsinhibitors, z.B. Methylenblau, Benzthiazin oder vorzugsweise Hydrochinon durchzuführen.

In der Regel werden die Umsetzungen einstufig durchgeführt. Es ist jedoch auch möglich, beispielsweise die Polycarbonsäure mit einem sekundären Fettamin in einer ersten Stufe und anschliessend mit einem primären Fettamin in einer zweiten Stufe umzusetzen.

Vor ihrem Einsatz als Komponente (A) in erfindungsgemässen Papierleimungsverfahren brauchen die Leimungsmittel nach erfolgter Herstellung aus den Komponenten (a), (b) und (c), aus den Komponenten (a'), (b') und (c'), aus den Komponenten ($a_1$'') und ($b_1$''), aus den Komponenten ($a_2$''), ($b_2$'') und (c'') oder aus den Komponenten (a''') und (b''') im allgemeinen nicht gereinigt oder umkristallisiert zu werden und können somit in der Regel direkt verwendet werden. Dies gilt auch für die Zwischenprodukte aus den Komponenten (a) und (b), (a') und (b') oder ($a_2$'') und ($b_2$''), die

somit vor ihrer Weiterverarbeitung mit der Komponente (c), (c') oder (c'') zu den Leimungsmitteln (A) in der Regel ebenfalls nicht gereinigt oder umkristallisiert werden.

Vor allem bei separater Zugabe (in beliebiger Reihenfolge) des Leimungsmittels (A) und des Retentionsmittels (B) zur Faserstoffsuspensiom beim erfindungsgemässen Verfahren zum Leimen von Papier oder Karton ist es zweckmässig, das Leimungsmittel mindestens teilweise in Salzform einzusetzen. Solche Salze können bei Bedarf dadurch erhalten werden, dass man nach beendeter Umsetzung der Komponenten (a), (b) und (c), (a'), (b') und (c'), ($a_1$'') und ($b_1$''), ($a_2$''), ($b_2$'') und (c'') oder (a''') und (b''') die erhaltenen Umsetzungsprodukte durch Zugabe u.a. eines Alkylamins, Alkanolamins, mit insgesamt höchstens 6 Kohlenstoffatomen, z.B. Trimethylamin, Monoäthylamin, Diäthanolamin, vor allem durch Zugabe von Ammoniak oder eines Alkalimetallhydroxydes, beispielsweise Kalium- oder vor allen Natriumhydroxyd, in der Regel in wässrigem Medium bei Raumtemperatur (etwa 15 bis etwa 25 °C) in die entsprechenden Salze gegebenenfalls mindestens teilweise überführt. Zweckmässigerweise wird ein Alkalimetallhydroxyd, z.B. Kalium- oder vor allem Natriumhydroxyd oder insbesondere Ammoniak in der Regel in Form ihrer verdünnten, etwa 1 bis etwa 10 gewichtsprozentigen, wässrigen Lösungen verwendet. Zweckmässig wird in der Regel höchstens 2 Mol, vor allem höchstens 1 Mol, vorzugsweise 0,1 bis 0,9, insbesondere 0,2 bis 0,7 Mol Ammoniak oder Alkalihydroxyd pro vorhandene Ladung des anionischen Leimungsmittels eingesetzt. Die als Salze vorliegenden Leimungsmittel weisen somit z.B. acide $-CH_2-$, $-\overset{|}{C}H$, $-SO_3H$ oder $-COOH$ Gruppen auf, die mindestens teilweise in die Gruppen $-\overset{|}{C}H^{\ominus}M^{\oplus}$, $-\underset{|}{\overset{|}{C}}|^{\ominus}M^{\oplus}$, $-SO_3{}^{\ominus}M^{\oplus}$ oder $-COO^{\ominus}M^{\oplus}$ überführt werden, worin $M^{\oplus}$ die entsprechenden Amin-, Ammonium- oder Alkalimetall-Kationen bedeutet.

Teilweise als Salz vorliegende Verbindungen, die als Leimungsmittel (A) in Frage kommen, sind z.B. solche, die durch Umsetzung von (a) 1 Mol Triäthanolamin oder Glycerin mit (b) 2 Mol Stearinsäure, hierauf mit (c) 1 bis 1,5 Mol Chlorsulfonsäure oder Phosphorpentoxyd und zuletzt mit 0,1 bis 0,9 Äquivalenten einer wässrigen Kalium- oder Natriumhydroxydlösung, bezogen auf die aciden Protonen der sauren Substituenten des Leimungsmittels, d.h. im vorliegenden Fall des Gemisches saurer Ester, die aus (a), (b) und (c) erhältlich sind.

Im erfindungsgemässen Papierleimungsverfahren wird neben dem vorstehend beschriebenen, monomeren bis oligomeren, anionischen Leimungsmittel (A) stets ein polymeres kationisches Retentionsmittel (B) eingesetzt, welches in der Regel ein Molekulargewicht von mindestens etwa 1000, vorzugsweise etwa 2000 bis etwa 2 000 000 aufweist. Retentionsmittel mit Molekulargewichten im Bereich von 10 000 bis 100 000 sind besonders bevorzugt. Grundsätzlich kann jedes handelsübliche Retentionsmittel im erfindungsgemässen Verfahren eingesetzt werden. Als Beispiele herkömmlicher Retentionsmittel (B), die sich besonders gut dazu eignen, zusammen mit dem Leimungsmittel (A) im erfindungsgemässen Papierleimungsverfahren eingesetzt zu werden, seien Polyalkylenimine, Epihalogenhydrin-Addukte von Umsetzungsprodukten aus Polyalkylenpolyaminen und aus aliphatischen Dicarbonsäuren oder von Umsetzungsprodukten aus Polyalkylenpolyaminen, aus Dicyandiamid und gegebenenfalls aus unveresterten oder mit Alkanolen veresterten organischen Dicarbonsäuren, Umsetzungsprodukte aus Dicyandiamid, aus Formaldehyd, aus Ammoniumsalzen starker organischer Säuren und aus Alkylendiaminen oder Polyalkylenpolyaminen, kationisch modifizierte Stärken oder Kohlenhydrate aus Johannisbrot- oder Guarkernmehl, Copolymerisate auf Basis von Polyamid-Aminen und Umsetzungsprodukte aus Epihalogenhydrinen und aus polymerisierten Diallylaminen erwähnt.

Bevorzugte Epichlorhydrin-Addukte von Umsetzungsprodukten aus Polyalkylenpolyaminen und aliphatischen Dicarbonsäuren sind z.B. in GB-A-865 727, Epichlorhydrin-Addukte aus Umsetzungsprodukten aus Diäthylentriamin und Dicyandiamid z.B. in DE-A-2 710 061 und in GB-A-1 125 486, Epichlorhydrin-Addukte von Umsetzungsprodukten aus Diäthylentriamin, Dicyandimid und unveresterten oder vorzugsweise mit Niederalkanolen veresterten Dicarbonsäuren, insbesondere Dimethyladipat, z.B. in GB-A-1 125 486 und Umsetzungsprodukte aus Dicyandiamid, Formaldehyd, Ammoniumsalzen, starker anorganischer Säuren und aus Äthylendiamin oder Triäthylentetramin z.B. in der US-A-3 491 064 beschrieben. Bevorzugte kationisch modifizierte Stärken oder Kohlehydrate aus Johannisbrot- oder Guarkernmehl sind Alkylenoxyd-Addukte dieser Stärken oder Kohlenhydrate, wobei das eingesetzte Alkylenoxyd 2 oder 3 Kohlenstoffatome im Alkylenrest und quaternäre Ammoniumgruppen aufweist. Copolymerisate auf Basis von Polyamid-Aminen weisen Molekulargewichte von $10^3$ bis $10^5$, vorzugsweise $10^3$ bis $10^4$ auf und sind z.B. aus aliphatischen, gesättigten Dicarbonsäuren mit 2 bis 10, vorzugsweise 3 bis 6 Kohlenstoffatomen, insbesondere Adipinsäure, und Polyalkylenpolyaminen, z.B. Polypropylen- und Polyäthylenpolyamin, insbesondere Dimethylamino-hydroxypropyldiäthylentriamin, erhältlich. Sie sind z.B. in CTFA Cosmetic Ingredient Dictionary, 3. Auflage 1982, der Cosmetic, Toiletry and Fragrance Association beschrieben. Umsetzungsprodukte aus Epihalogenhydrinen und polymerisierten Diallylaminen weisen bevorzugt Molekulargewichte von 1000 bis 2000 auf und sind z.B. in US-A-3 700 623 und US-A-4 279 794 beschrieben.

Als Retentionsmittel (B), die zur Verwendung zusammen mit den Leimungsmitteln (A) im erfindungsgemässen Papierleimungsverfahren im Vordergrund des Interesses stehen, sei eine mit einem quaternären Ammoniumgruppen enthaltenden 1,2-Propylenoxyd modifizierte Mais- oder

Kartoffelstärke, deren 25%ige Anschlämmung in destilliertem Wasser bei 20 °C einen pH-Wert von 4,2 bis 4,6 aufweist, ein Polyäthylenimin, das ein Molekulargewicht von 10 000 bis 100 000 aufweist, ein Epichlorhydrin-Addukt eines Umsetzungsproduktes aus Triäthylentetramin und Dicyandiamid, ein Epichlorhydrin-Addukt eines Umsetzungsproduktes aus Diäthylentriamin, Dicyandiamid und Dimethyladipat, ein Umsetzungsprodukt aus Dicyandiamid, Formaldehyd, Ammoniumchlorid und Äthylendiamin, ein Epichlorhydrin-Addukt eines Poly-N-Methyldiallylamins und ein Copolymerisat aus Adipinsäure und Dimethylamino-hydroxypropyl-diäthylentriamin genannt.

Verfahrensgemäss werden in der Regel 0,02 bis 3, vorzugsweise 0,1 bis 3, insbesondere 0,2 bis 0,8 Gewichtsprozent des Leimungsmittels (A) und 0,02 bis 3, vorzugsweise 0,1 bis 3, insbesondere 0,2 bis 0,4 Gewichtsprozent des Retentionsmittels (B), bezogen jeweils auf Trockensubstanz an (A) und an (B) und auf den Feststoffgehalt der Faserstoffsuspension, eingesetzt. 0,02 bis weniger als 0,1 Gewichtsprozent des Leimungsmittels (A) und des Retentionsmittels (B) reichen nur für das sogenannte «size press control», das mit konventionellen Leimungstests nicht erfassbar ist (vgl. z.B. Artikel «Control and understanding of size press pickup» von D.R. Dill in der Zeitschrift TAPPI (Proceedings of the Technical Association of the Pulp and Paper Industry), Band 57, Nr. 1 vom Januar 1974, Seiten 97 bis 100).

Die Faserstoffsuspension, zu welcher die Leimungsmittel (A) und die Retentionsmittel (B) gegeben werden, weist in der Regel einen Feststoffgehalt von 0,1 bis 5, vorzugsweise 0,3 bis 3, insbesondere 0,3 bis 1 Gewichtsprozent und einen Schopper-Riegler-Mahlgrad von etwa 10° bis etwa 90°, vor allem 20° bis 60°, vorzugsweise 20° bis 45°, insbesondere 25° bis 35° auf. Sie enthält in der Regel Zellstoff, insbesondere solchen aus Nadelholz, z.B. Kiefernholz, oder aus Hartholz, d.h. Laubholz, z.B. Buchenholz, der nach herkömmlichen Verfahren, z.B. dem Sulfit- oder vor allem dem Sulfatverfahren hergestellt wird. Zudem enthält die Faserstoffsuspension gegebenenfalls Holzschliff. Auch alaunhaltiges Altpapier kann in der Faserstoffsuspension enthalten sein. Auch Zellstoffsuspensionen, die nach dem sogenannten CMP- oder CTMP-Verfahren (Chemimechanical and chemithermomechanical pulping processes, vgl. z.B. Artikel «Developments in refiner mechanical pulping» von S.A. Collicutt und Mitarbeitern in TAPPI, Band 64, Nr. 6, vom Juni 1981, Seiten 57 bis 61) hergestellt werden, kommen in Betracht.

Die Faserstoffsuspension kann zudem organische oder mineralische Füllmittel enthalten. Als organische Füllmittel kommen u.a. synthetische Pigmente, z.B. Polykondensationsprodukte aus Harnstoff oder Melamin und Formaldehyd mit grossen spezifischen Oberflächen, die in hochdisperser Form vorliegen und u.a. in GB-A-1 043 437 und GB-A-1 318 244 beschrieben sind, und als mineralische Füllmittel kommen u.a. Montmorillonit, Titandioxyd, Calciumsulfat und vor allem Talk, Kaolin und/oder Kreide (Calciumcarbonat) in Betracht. In der Regel enthält die Faserstoffsuspension 0 bis 40, vorzugsweise 5 bis 25, insbesondere 15 bis 20 Gewichtsprozent, bezogen auf den Feststoffgehalt der Faserstoffsuspension, an Trockensubstanz der Füllmittel der angegebenen Art.

Der pH-Wert der Faserstoffsuspension kann in einem weiten Bereich liegen, wobei z.B. Werte von 3,5 bis etwa 10 vorliegen können.

Bei Zusatz von z.B. Calciumcarbonat werden alkalische Faserstoffsuspensionen mit einem pH-Wert von etwa 7 bis etwa 9, vorzugsweise 7,5 bis 8,5 erhalten. Saure Faserstoffsuspensionen mit einem pH-Wert von 3,5 bis 7, vorzugsweise 5 bis 7, insbesondere 5 bis 6, können in Abwesenheit von Calciumcarbonat durch Zugabe von Säuren, z.B. Schwefel- oder Ameisensäure oder vor allem von z.B. latent sauren Sulfaten, wie Aluminiumsulfat (Alaun), erhalten werden.

Faserstoffsuspensionen, die kein Füllmittel enthalten können in einem breiten pH-Bereich von z.B. 3,5 bis 10 vorliegen. Bevorzugt sind Faserstoffsuspensionen, die gegebenenfalls durch Zusatz von Kreide einen pH-Wert von etwa 7 bis 9 aufweisen und insofern vorteilhaft sind, dass mögliche Korrosionserscheinungen an den empfindlichen Papiermaschinen ausgeschlossen werden.

Die Faserstoffsuspension kann auch Additive enthalten, wie z.B. Stärke oder ihre Abbauprodukte, welche die Faser/Faser- oder Faser/Füllmittel-Bindung erhöhen.

Auch hochmolekulare Polymere der Acrylsäurereihe, z.B. Polyacrylamide, mit Molekulargewichten über 1 000 000 können zu den Faserstoffsuspensionen als Hilfsmittel zum Zurückhalten feinster Zellstoffaserteilchen gegeben werden, wobei minimale Einsatzmengen von etwa 0,005 bis 0,02 Gewichtsprozent, bezogen auf Trockensubstanz des Polymers und den Feststoffgehalt der Faserstoffsuspensionen, genügend sind.

Die Faserstoffsuspension wird im erfindungsgemässen Verfahren auf an sich bekannte Weise auf Blattbildnern oder vorzugsweise kontinuierlich auf Papiermaschinen üblicher Bauart zu Papier oder Karton weiterverarbeitet. Nach einer Trocknung bei etwa 100 bis 140 °C während etwa 0,5 bis 10 Minuten können z.B. Papiere eines variablen Flächengewichtes von z.B. 50 bis 200 $g/m^2$ erhalten werden.

Wie eingangs erwähnt, enthält die wässrige Zusammensetzung zur Durchführung des erfindungsgemässen Papierleimungsverfahrens das Leimungsmittel (A) neben fakultativen üblichen Zusätzen, sofern das Leimungsmittel und das Retentionsmittel (B) separat zur Faserstoffsuspension gegeben werden. In diesem Fall enthält die Zubereitung das Leimungsmittel in der Regel teilweise in Form seiner Salze (erhalten unter Mitverwendung von z.B. Ammoniak, eines Alkyl- oder Alkanolamins oder eines Alkalimetallhydroxydes der angegebenen Art in den vorstehend angegebenen Verhältnissen). Im allgemeinen enthalten solche Zusammensetzungen 5 bis 30, vorzugsweise 5 bis 20 Gewichtsprozent an Trockensubstanz des teilweise in Salzform vorliegenden Leimungsmittels bezogen auf das Gewicht der wässrigen Zusammensetzung.

Hingegen enthält die wässrige Zusammensetzung neben den fakultativen, üblichen Zusätzen, sofern das Leimungsmittel (A) und das Retentionsmittel (B) gleichzeitig zur Faserstoffsuspension gegeben werden, (A) 2 bis 40, vorzugsweise 5 bis 30, insbesondere 5 bis 10 Gewichtsprozent Leimungsmittel (berechnet als Trockensubstanz), bezogen auf das Gewicht der wässrigen Zusammensetzung, wobei das Leimungsmittel gegebenenfalls in Salzform vorliegt, und (B) 0,1 bis 20, vorzugsweise 0,5 bis 10, insbesondere 3 bis 8 Gewichtsprozent Retentionsmittel (berechnet als Trockensubstanz), bezogen auf die wässrige Zusammensetzung.

Die wässrigen Zusammensetzungen der angegebenen Art enthalten gegebenenfalls als übliche Zusätze oberflächenaktive Verbindungen, z.B. Dispergatoren oder ferner Emulgatoren und/oder wasserlösliche, organische Lösungsmittel. Als Dispergatoren und Emulgatoren kommen z.B. herkömmliche Ligninsulfonate, Äthylenoxydaddukte von Alkylphenolen, Fettaminen, Fettalkoholen oder Fettsäuren, Fettsäureester mehrwertiger Alkohole, substituierte Benzimidazole oder Kondensationsprodukte aus aromatischen Sulfonsäuren und Formaldehyd in Betracht. Weitere oberflächenaktive Verbindungen sind vorzugsweise die anionischen Tenside, insbesondere Sulfat-Tenside, z.B. Diäthanolaminlaurylsulfat oder äthoxylierte Laurylsulfate. Mögliche wasserlösliche, organische Lösungsmittel sind aliphatische Äther mit 1 bis 10 Kohlenstoffatomen, z.B. Dioxan, Methylenglykol-n-butyläther oder Diäthylenglykolmonobutyläther oder Alkohole mit 1 bis 4 Kohlenstoffatomen, z.B. Isopropanol, Äthanol oder Methanol.

Die Zusammensetzungen werden auf übliche Weise hergestellt, indem man das Leimungsmittel (A) zusammen mit dem Retentionsmittel (B) oder das Leimungsmittel (A) in der Regel teilweise in Form seines Salzes für sich allein entweder in geschmolzenem Zustand oder vorzugsweise in festem Zustand, insbesondere in pulverisierter Form, in der Regel in Gegenwart von Glasperlen und nötigenfalls von Emulgatoren (bei Leimungsmittel in geschmolzenem Zustand) oder Dispergatoren (bei Leimungsmittel in Pulverform) bei höchstens 90 °C, vorzugsweise etwa 50 bis etwa 85 °C bei Emulsionen, insbesondere bei etwa 15 bis etwa 25 °C bei Dispersionen, verrührt, wobei lagerstabile, homogene, weiter verdünnbare Emulsionen oder vorzugsweise Dispersionen erhalten werden. Da die Leimungsmittel zusammen mit den Retentionsmitteln oder die teilweise als Salze vorliegenden Leimungsmittel in der Regel selbst-dispergierend oder selbst-emulgierend sind, ist der Einsatz von Dispergatoren oder Emulgatoren im allgemeinen nicht unbedingt erforderlich. Dies gilt auch für den fakultativen Zusatz von Lösungsmitteln und/oder Tensiden, die nur bei ungenügender Lagerstabilität der Dispersionen oder Emulsionen eingesetzt werden.

Als Vorteil des erfindungsgemässen Verfahrens sei erwähnt, dass Faserstoffsuspensionen der verschiedensten Art mit relativ kleinen Mengen an Leimungs- und Retentionsmittel auf einfache Art und Weise zu Papier verarbeitet werden können, welches gute Leimungseigenschaften (Tintenschwimmdauer und vor allem Wasseraufnahme nach Cobb) aufweist. Das verfahrensgemäss geleimte Papier weist gute mechanische Eigenschaften, d.h. gute Festigkeiten, insbesondere eine gute Reissfestigkeit auf. Eine gute Reproduzierbarkeit des Verfahrens ist gewährleistet. Insbesondere können holzschliffhaltige oder altpapierhaltige Faserstoffsuspensionen verarbeitet werden. Auch die Kompatibilität des erfindungsgemäss verwendeten Leimungsmittels mit den verschiedensten Füllmitteln, und auch deren Zusätze, wie z.B. Kaolin oder Alaunen im sauren Bereich der Faserstoffsuspensionen, ist vorteilhaft. Die Leimungsmittel weisen ebenfalls eine vorteilhafte Kompatibilität mit optischen Aufhellern auf. Zudem wird der Weissgrad des geleimten Papiers durch die Leimung nicht wesentlich beeinflusst und kann sogar u.U. verbessert werden. Vor allem ist die in der Regel überraschend hohe Lagerstabilität der Leimungsmitteldispersionen der angegebenen Art von grossem Vorteil.

Die in den nachfolgenden Herstellungsvorschriften und Ausführungsbeispielen angegebenen Teile und Prozente beziehen sich auf das Gewicht.

Herstellungsbeispiele von Zwischenprodukten

Beispiel 1:

149 Teile Triäthanolamin (1 Mol), 568 Teile Stearinsäure (2 Mol) und 3,5-Teile p-Toluolsulfonsäure als Katalysator werden in 510 Teilen p-Xylol gelöst. Diese Lösung wird auf die Rückflusstemperatur von ca. 140 °C aufgeheizt und so lange bei dieser Temperatur gehalten, bis die durch die Veresterungsreaktion gebildete theoretische Menge Wasser (2 Mol) mittels eines Wasserabscheiders azeotrop entfernt wird. Dann wird das Xylol abdestilliert. Als Rückstand erhält man 680 Teile eines wachsartigen Estergemisches, das neben homologen Monoestern und Triestern als Hauptbestandteil den Diester der Formel

(31) 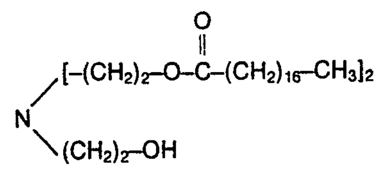

enthält. Schmelzpunkt: 40–44 °C.

Beispiel 2:

22 Teile Brenzcatechin (0,2 Mol) werden in 200 Teilen Dimethylsulfoxyd gelöst und anschliessend mit 44,8 Teilen einer 50%igen, wässrigen Kaliumhydroxydlösung (0,4 Mol) versetzt. Das Reaktionsgemisch wird 30 Minuten bei 20 °C unter Rühren gehalten. Dann werden zum Reaktionsgemisch 133,2 Teile Octadecylbromid (0,4 Mol) gegeben. Das Reaktionsgemisch wird auf 50 °C aufgeheizt

und 6 Stunden bei dieser Temperatur gehalten, hierauf mit 1000 Teilen Wasser verdünnt und mit einer wässrigen Essigsäurelösung auf den pH-Wert von 4 bis 5 gestellt, wobei das Reaktionsprodukt ausfällt. Das Produkt wird abfiltriert, mit Wasser nachgewaschen und aus Aceton umkristallisiert. Man erhält 98,6 Teile des Diäthers der Formel

(32) 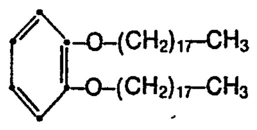

Schmelzpunkt: 58–61 °C.

Beispiel 3:

Man verfährt wie in Beispiel 2 angegeben, setzt jedoch 22 Teile Resorcin (anstelle von Brenzcatechin) ein.

Man erhält 97 Teile des Diäthers der Formel

(33) 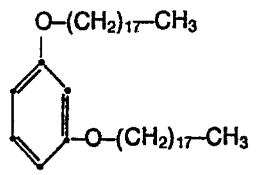

Schmelzpunkt: 71–73 °C.

Beispiel 4:

1136 Teile Stearinsäure (4,0 Mol) werden bei 100 °C geschmolzen. Zu dieser Schmelze werden 227 Teile Diäthylentriamin (2,2 Mol) innerhalb von 1 Stunde gegeben, wobei sich die Temperatur des Reaktionsgemisches von selbst auf 110 °C erhöht. Das Reaktionsgemisch wird nun innerhalb von 2 Stunden auf 160 °C aufgeheizt und 2 Stunden bei dieser Temperatur gehalten, wobei unter schwachem Stickstoffstrom die durch die Reaktion freigesetzte, theoretische Menge Wasser (4 Mol) mit dem überschüssigen Diäthylentriamin aus dem Reaktionsgemisch abdestilliert wird. Das Reaktionsgemisch wird dann auf Raumtemperatur (15 bis 25 °C) gekühlt. Man erhält 1232 Teile eines als gelbliches Wachs vorliegenden Amidgemisches, welches als Hauptbestandteil das Diamid der Formel

(34) 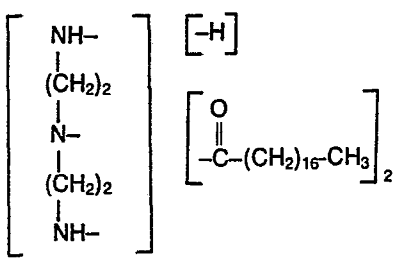

enthält. Schmelzpunkt: 100–105 °C.

Beispiel 5:

Man verfährt wie in Beispiel 4 angegeben, setzt jedoch 287 Teile Dipropylentriamin (2,2 Mol) (anstelle von 277 Teilen Diäthylentriamin) ein. Man erhält 1288 Teile eines als weissliches Wachs vorliegenden Amidgemisches, welches als Hauptbestandteil das Diamid der Formel

(35) 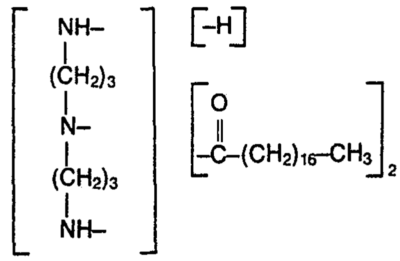

enthält. Schmelzpunkt: 101–104 °C.

Beispiel 6:

Man verfährt wie in Beispiel 4 angegeben, setzt jedoch 1128 Teile Ölsäure (4,0 Mol) (anstelle von 1136 Teilen Stearinsäure) und 1 Teil Hydrochinon als Polymerisationsinhibitor ein, wobei das Reaktionsgemisch während 3 Stunden (statt 2 Stunden) bei 160 °C gehalten wird. Man erhält 1225 Teile eines als bernsteinfarbenes Öl vorliegenden Amidgemisches, welches als Hauptbestandteil das Diamid der Formel

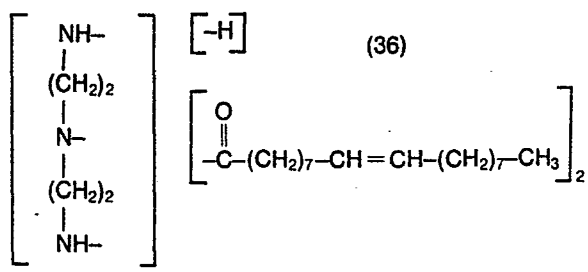
(36)

enthält.

Beispiel 7:

Man verfährt wie in Beispiel 1 angegeben, setzt jedoch 284 Teile Stearinsäure (1 Mol) und 256 Teile Palmitinsäure (1 Mol) (anstelle von 568 Teilen Stearinsäure) ein. Man erhält 650 Teile eines wachsartigen Estergemisches, welches neben homologen Mono- und Triestern als Hauptbestandteil das Diestergemisch der Formel

(37) 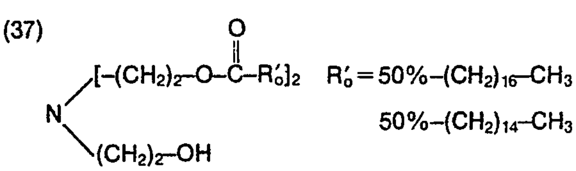

enthält. Schmelzpunkt: 38–42 °C.

Beispiel 8:

Man verfährt wie in Beispiel 1 angegeben, setzt jedoch 136 Teile Pentaerythrit (1 Mol), 852 Teile Stearinsäure (3 Mol), 5 Teile p-Toluolsulfonsäure und 1000 Teile p-Xylol ein, wobei 3 Mol Wasser azeotrop entfernt werden. Man erhält 930 Teile eines wachsartigen Estergemisches, das neben homologen Mono-, Di- und Tetraestern als Hauptbestandteil den Triester der Formel

(38) 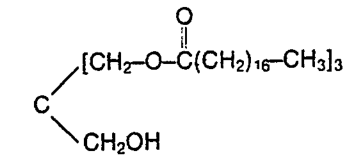

enthält. Schmelzpunkt: 54–58 °C.

Beispiel 9:

Man verfährt wie in Beispiel 1 angegeben, setzt jedoch 121 Teile Tris(hydroxymethyl)-aminomethan (1 Mol), 568 Teile Stearinsäure (2 Mol), 3,4 Teile p-Toluolsulfonsäure und 600 Teile p-Xylol ein, wobei die durch die Veresterungsreaktion unter Ringschluss gebildete, theoretische Menge von 3 Mol Wasser azeotrop entfernt wird. Man erhält 630 Teile eines wachsartigen Gemisches, das neben homologen, ringförmigen Mono- und Triestern als Hauptbestandteil den Ester der Formel

(39) 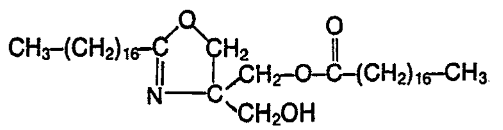

enthält. Schmelzpunkt: 75–78 °C.

Beispiel 10:

Man verfährt wie in Beispiel 1 angegeben, setzt jedoch 273 Teile Laurylamindiäthoxylat (1 Mol) (hergestellt aus 1 Mol Laurylamin und 2 Mol Äthylenoxyd), 340 Teile Behensäure (1 Mol), 2,3 Teile p-Toluolsulfonsäure und 625 Teile p-Xylol ein, wobei 1 Mol Wasser azeotrop entfernt wird. Man erhält 593 Teile eines hellgelben, wachsartigen Estergemisches, das neben dem homologen Diester als Hauptbestandteil den Monoester der Formel

(40) 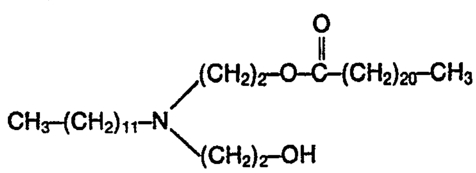

enthält. Schmelzpunkt: 35–37 °C.

Beispiel 11:

Man verfährt wie in Beispiel 1 angegeben, setzt jedoch 292 Teile N,N,N′,N′-Tetrakis(2-hydroxypropyl)-äthylendiamin (1 Mol), 852 Teile Stearinsäure (3 Mol), 6 Teile p-Toluolsulfonsäure und 933 Teile p-Xylol ein, wobei 3 Mol Wasser azeotrop entfernt werden. Man erhält 1085 Teile eines wachsartigen Estergemisches, das neben homologen Mono-, Di- und Tetraestern als Hauptbestandteil den Triester der Formel

(41) 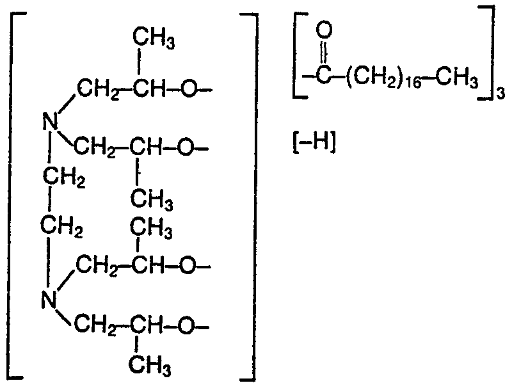

enthält. Schmelzpunkt: 35–40 °C.

Beispiel 12:

Man verfährt wie in Beispiel 1 angegeben, setzt jedoch 42,4 Teile 1,2,4-Butantriol (0,4 Mol), 227,2 Teile Stearinsäure (0,8 Mol), 1,3 Teile p-Toluolsulfonsäure und 270 Teile p-Xylol ein, wobei 0,8 Mol Wasser azeotrop entfernt wird. Man erhält 240 Teile eines wachsartigen Estergemisches, das als Hauptbestandteil den Diester der Formel

(42) 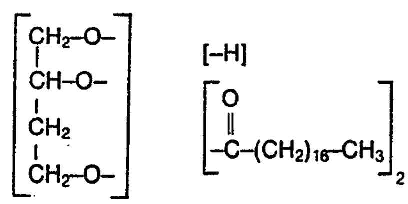

enthält. Schmelzpunkt: 46–48 °C.

Beispiel 13:

Man verfährt wie in Beispiel 1 angegeben, setzt jedoch 65,25 Teile Tris(hydroxyäthyl)-isocyanurat (0,25 Mol), 142 Teile Stearinsäure (0,5 Mol), 1,2 Teile p-Toluolsulfonsäure und 170 Teile p-Xylol ein, wobei 0,5 Mol Wasser azeotrop entfernt werden und kristalliert das erhaltene Rohprodukt aus Aceton um. Man erhält als weisses Pulver 150 Teile eines Estergemisches, das als Hauptbestandteil den Diester der Formel

(43) 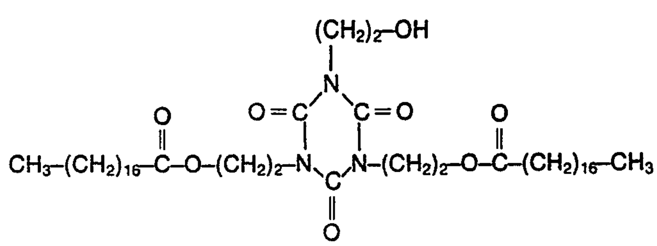

enthält. Schmelzpunkt: 67–69 °C.

Beispiel 14:

201,8 Teile Stearylamin (0,75 Mol) werden bei 50 °C in 700 Teilen Isopropanol gelöst. In diese Lösung werden 74,3 Teile Isocyanursäure-triglycid (0,25 Mol) (hergestellt aus 1 Mol Isocyanursäure und 3 Mol Epichlorhydrin) eingetragen. Das Reaktionsgemisch wird auf 80 °C aufgeheizt und 5 Stunden bei dieser Temperatur unter Rühren gehalten, wobei eine schwach trübe Lösung entsteht. Bei 80 °C wird diese Lösung filtriert. Beim Abkühlen des Filtrats fällt das Reaktionsprodukt aus. Das Produkt wird abfiltriert, mit 200 Teilen Isopropanol portionsweise gewaschen und bei 35 °C unter vermindertem Druck getrocknet. Man erhält 223 Teile einer Verbindung der Formel

(44) 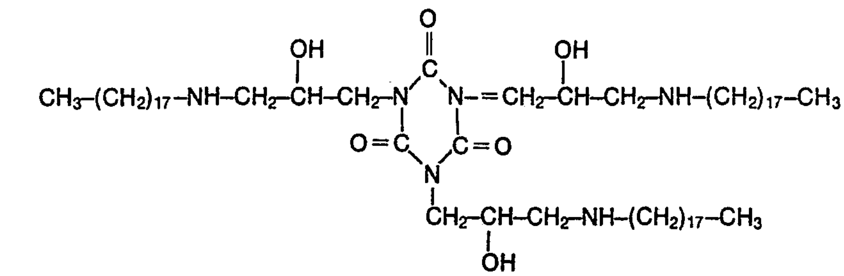

Schmelzpunkt: 78–83 °C.

Beispiel 15:

167 Teile Isocyanursäure-triglycid (0,5 Mol), 427 Teile Stearinsäure (1,5 Mol) und 1,5 Teile Stearinsäurenatriumsalz als Katalysator werden auf 135 °C aufgeheizt. Bei dieser Temperatur wird das Heizbad entfernt. Die Temperatur des Reaktionsgemisches steigt alsdann von selbst auf 160 °C. Nach Abklingen der exothermen Reaktion wird das Reaktionsgemisch bei 150 °C während 3 Stunden unter Rühren gehalten. Dann lässt man die Schmelze abkühlen und erstarren. Man erhält 594 Teile der Verbindung der Formel

(45) 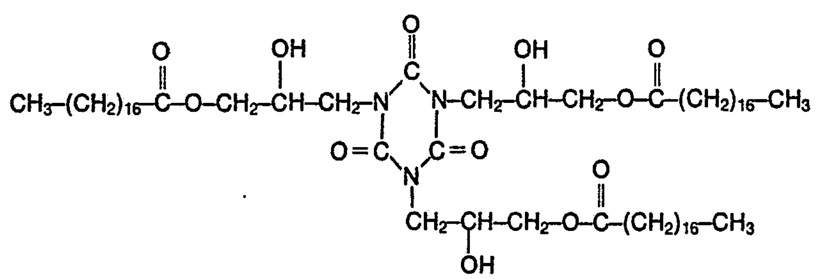 ,

die als hellbeige gefärbtes Rohprodukt vorliegt. Eine Probe des Rohproduktes wird aus Methanol umkristallisiert. Das umkristallisierte Produkt ist farblos. Schmelzpunkt: 58–62 °C.

Beispiel 16:

139 Teile Stearylamin (0,5 Mol) werden bei 75 °C in 400 Teilen Isopropanol gelöst. In diese Lösung werden 50,5 Teile (0,25 Mol) Butandioldiglycid (hergestellt aus 1 Mol 1,4-Butandiol und 2 Mol Epichlorhydrin) zugegeben. Die Reaktionslösung wird auf 80 °C aufgeheizt und 4 Stunden bei 80 °C unter Rühren gehalten. Aus der klaren Lösung wird das Lösungsmittel unter vermindertem Druck abdestilliert.

Der Destillationsrückstand wird aus Methanol umkristallisiert. Man erhält 163 Teile der Verbindung der Formel

(46) 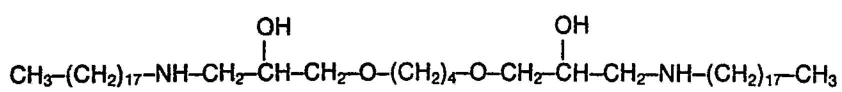

Schmelzpunkt: 85–88 °C.

Beispiel 17:

76,02 Teile der gemäss Beispiel 16 erhaltenen Verbindung der Formel (46) (0,1 Mol) werden in 200 Teilen o-Xylol bei 60 °C gelöst. In diese Lösung werden 56,9 Teile Stearinsäure (0,2 Mol) eingetragen. Diese Lösung wird auf die Rückflusstemperatur von ca. 145 °C aufgeheizt und während ca. 8 Stunden bei dieser Temperatur gehalten, bis die durch die Veresterungsreaktion gebildete theoretische Menge Wasser (0,2 Mol) mittels eines Wasserabscheiders azeotrop entfernt wird. Aus der klaren Lösung wird das Lösungsmittel unter vermindertem Druck abdestilliert. Man erhält als Destillationsrückstand 128 Teile der Verbindung der Formel

(47) 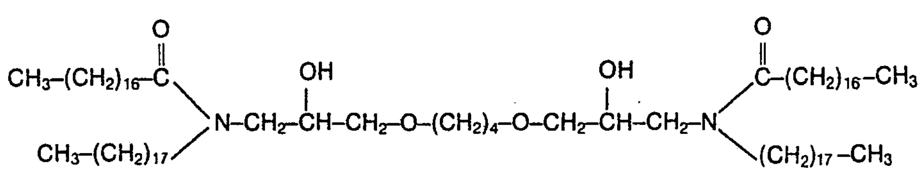

Schmelzpunkt: 82–84 °C.

Beispiel 18:

Zu einer Lösung von 10,4 Teilen Diäthylentriamin (0,1 Mol) in 80 Teilen Toluol wird eine Lösung von 59 Teilen Octadecylisocyanat (0,2 Mol) in 240 Teilen Toluol bei Raumtemperatur (15 bis 25 °C) innerhalb von 20 Minuten gegeben. Die Temperatur des Reaktionsgemisches steigt von selbst auf 50 °C. Hierauf wird das Reaktionsgemisch auf die Rückflusstemperatur von ca. 111 °C aufgeheizt, während 15 Minuten bei dieser Temperatur gehalten, dann auf 60 °C abgekühlt und in zwei gleiche Teile halbiert. Aus der Hälfte des Reaktionsgemisches wird das Toluol bei 60 °C unter vermindertem Druck abdestilliert. Man erhält als Destillationsrückstand 34 Teile eines als beige gefärbtes Pulver vorliegenden Amidgemisches, welches als Hauptbestandteil das Umsetzungsprodukt der Formel

(48) 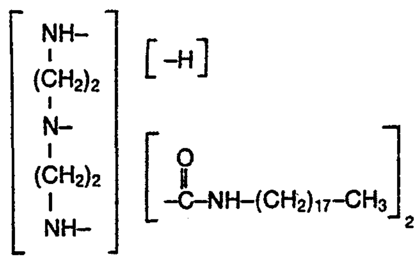

enthält. Schmelzpunkt: 138–141 °C.

Beispiel 19:

106,7 Teile eines technischen, im Handel erhältlichen Gemisches, das als Hauptbestandteil $N_1$-Stearyl-diäthylentriamin (0,3 Mol) enthält, werden bei Raumtemperatur (15–25 °C) in 300 Teilen Toluol gelöst. Zu dieser Lösung wird eine Lösung von 88,8 Teilen Octadecylisocyanat (0,3 Mol) in 200 Teilen Toluol gegeben, wobei das Reaktionsgemisch auf 25 °C gekühlt werden muss und während 6 Stunden bei dieser Temperatur gehalten wird. Hierauf wird das Reaktionsgemisch auf 50 °C aufgeheizt und 2 Stunden bei dieser Temperatur gehalten. Das Toluol wird dann aus dem Reaktionsgemisch unter vermindertem Druck bei 50 °C abdestilliert und der Reaktionsrückstand aus Methanol umkristallisiert. Man erhält 163 Teile eines als farbloses Pulver vorliegenden Produktgemisches, das als Hauptbestandteil das Umsetzungsprodukt der Formel

(49) 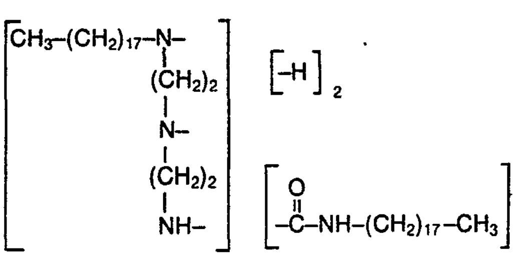

enthält. Schmelzpunkt: 86–92 °C.

Beispiel 20:

177,9 Teile des im Beispiel 19 eingesetzten Gemisches, das als Hauptbestandteil $N_1$-Stearyl-diäthylentetramin (0,5 Mol) enthält, werden zusammen mit 199,3 Teilen Stearinsäuremethylester (0,5 Mol) bei 60 °C geschmolzen. Diese Schmelze wird auf 190 °C aufgeheizt und solange bei dieser Temperatur gehalten, bis die durch die Reaktion freigesetzte theoretische Menge Methanol (0,5 Mol) aus dem Reaktionsgemisch abdestilliert wird. Das erhaltene Rohprodukt wird aus Essigsäureäthylester umkristallisiert. Man erhält 296 Teile eines als hellgelbes Wachs vorliegenden Produktgemisches, das als Hauptbestandteil das Umsetzungsprodukt der Formel

(50) 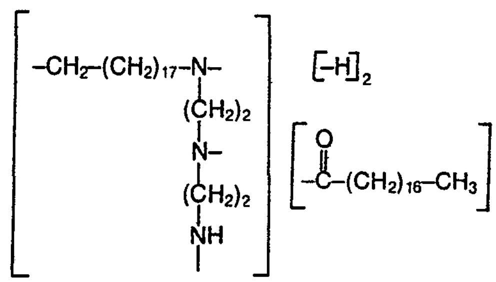

enthält. Schmelzpunkt: 66–70 °C.

Beispiel 21:

53 Teile Octadecylamin (0,2 Mol) werden bei 35 °C geschmolzen. Zu dieser Schmelze werden 22,6 Teile Cyanessigsäureäthylester (0,2 Mol) bei 40 °C gegeben. Das Reaktionsgemisch wird anschliessend während 2 Stunden bei 40 °C gehalten und dann auf Raumtemperatur (15–25 °C) abgekühlt, wobei das Reaktionsprodukt ausfällt. Das Rohprodukt wird abfiltriert, mit Äthanol nachgewaschen und aus Toluol umkristallisiert. Man erhält 65 Teile eines als weisses Pulver vorliegenden Umsetzungsproduktes der Formel

(51) $NC-CH_2-CO-NH-(CH_2)_{17}-CH_3$

Schmelzpunkt: 79–80 °C.

Herstellungsbeispiele von Leimungsmitteln

Beispiel 22:

624 Teile eines technischen Gemisches, das neben homologen Mono- und Triestern als Hauptbestandteil 1,3- und 1,2-Glycerindistearat (1 Mol) enthält, werden in 2000 Teilen Tetrachlorkohlenstoff gelöst. Andererseits werden 142 Teile Phosphorpentoxyd (1 Mol) in 500 Teilen Tetrachlorkohlenstoff dispergiert. Nun wird die Lösung des Glycerindistearates innerhalb von 20 Minuten zur Phosphorpentoxyddispersion gegeben. Hierauf wird das Reaktionsgemisch auf die Rückflusstemperatur von ca. 76 °C aufgeheizt und bei dieser Temperatur während 12 Stunden gehalten. Die Reaktionslösung wird anschliessend von Verunreinigungen geklärt. Nach dem Entfernen des Lösungsmittels wird das Rohprodukt aus Methyläthylketon umkristallisiert. Man erhält 650 Teile eines Estergemisches als beigefarbenes Pulver, welches als Hauptbestandteil den sauren Diester der Formel

(52) 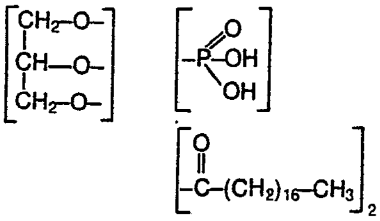

enthält. Schmelzpunkt: 57–59 °C. Säurezahl: 112.

Beispiel 23:

320 Teile des gemäss Beispiel 4 erhaltenen Amidgemisches (0,5 Mol) werden bei 20 °C in 1500 Teilen Chloroform suspendiert. Zu dieser Suspension wird eine Lösung von 74 Teilen Phthalsäureanhydrid (0,5 Mol) in 800 Teilen Aceton innerhalb von 35 Minuten bei 20 °C gegeben. Die Temperatur des Reaktionsgemisches erhöht sich von selbst auf 25 °C und wird 1 Stunde bei dieser Temperatur gehalten. Anschliessend wird das Reaktionsgemisch auf die Rückflusstemperatur von ca. 57 °C aufgeheizt und während einer Stunde bei dieser Temperatur gehalten, wobei eine klare Lösung entsteht. Das Lösungsmittel wird unter vermindertem Druck aus dem Reaktionsgemisch abdestilliert. Man erhält 390 Teile eines als gelbliches Pulver vorliegenden Rohproduktes das aus Aceton umkristallisiert werden kann und als Hauptbestandteil das Umsetzungsprodukt der Formel

(53) 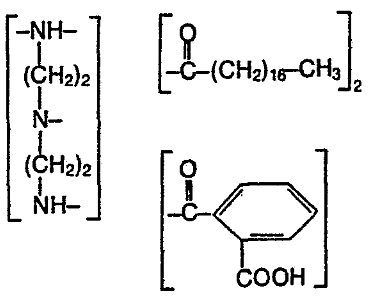

enthält.
Schmelzpunkt (umkristallisiertes Produkt): 77–78 °C
Säurezahl (umkristallisiertes Produkt): 73

Beispiel 24:

Man verfährt wie in Beispiel 23 angegeben, setzt jedoch 50 Teile Bernsteinsäureanhydrid (0,5 Mol) (anstelle von 74 Teilen Phthalsäureanhydrid) ein. Man erhält 366 Teile eines als ockerfarbenes Pulver vorliegenden Rohproduktes, das aus Äthanol umkristallisiert werden kann und als Hauptbestandteil das Umsetzungsprodukt der Formel

(54) 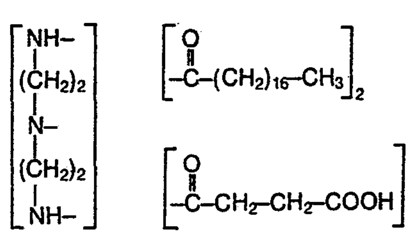

erhält.

Schmelzpunkt (umkristallisiertes Produkt): 108–112 °C
Säurezahl (umkristallisiertes Produkt): 68

Beispiel 25:

Man verfährt wie in Beispiel 23 angegeben, setzt jedoch 77 Teile Hexahydrophthalsäureanhydrid (0,5 Mol) ein. Man erhält 391 Teile eines als ockerfarbenes Pulver vorliegenden Rohproduktes, das aus Aceton umkristallisiert werden kann und als Hauptbestandteil das Umsetzungsprodukt der Formel

(55) 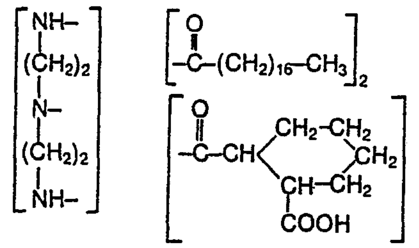

enthält.
Schmelzpunkt (umkristallisiertes Produkt): 66–69 °C
Säurezahl (umkristallisiertes Produkt): 66

Beispiel 26:

Man verfährt wie in Beispiel 23 angegeben, setzt jedoch 49 Teile Maleinsäureanhydrid (0,5 Mol) ein. Man erhält 350 Teile eines als gelbliches Pulver vorliegendes Rohprodukts, das aus Aceton umkristallisiert werden kann und als Hauptbestandteil das Umsetzungsprodukt der Formel

(56) 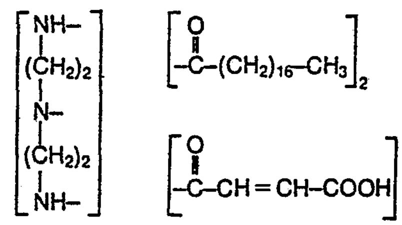

enthält.
Schmelzpunkt (umkristallisiertes Produkt): 82–87 °C
Säurezahl (umkristallisiertes Produkt): 88

Beispiel 27:

Man verfährt wie in Beispiel 23 angegeben, setzt jedoch 56 Teile Citraconsäureanhydrid (0,5 Mol) ein. Man erhält 360 Teile eines als ockerfarbenes Pulver vorliegenden Rohproduktes, das aus Aceton umkristallisiert werden kann und als Hauptbestandteil das Umsetzungsprodukt der Formel

(57) 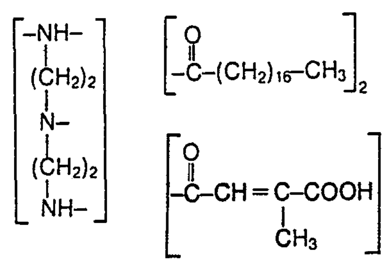

enthält.

Schmelzpunkt (umkristallisiertes Produkt): 71–75 °C
Säurezahl (umkristallisiertes Produkt): 82

Beispiel 28:

Man verfährt wie in Beispiel 23 angegeben, setzt jedoch 232 Teile des gemäss Beispiel 5 erhaltenen Amidgemisches (0,5 Mol) und 49 Teile Maleinsäureanhydrid (0,5 Mol) (anstelle von 320 Teilen des gemäss Beispiel 4 erhaltenen Amidgemisches und von 74 Teilen Phthalsäureanhydrid) ein. Man erhält 370 Teile eines als weissliches Pulver vorliegenden Rohproduktes, das aus Aceton umkristallisiert werden kann und als Hauptbestandteil das Umsetzungsprodukt der Formel

(58) 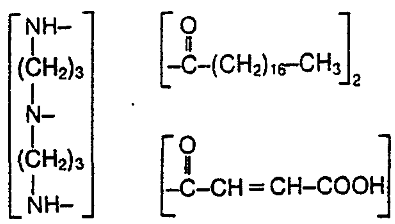

enthält.
Schmelzpunkt (umkristallisiertes Produkt): 89–100 °C
Säurezahl (umkristallisiertes Produkt): 72

Beispiel 29:

Man verfährt wie in Beispiel 23 angegeben, setzt jedoch 313 Teile des gemäss Beispiel 6 erhaltenen Amidgemisches (0,5 Mol) und 49 Teile Maleinsäureanhydrid (0,5 Mol) ein. Man erhält 350 Teile eines braungefärbten Öls, das als Hauptbestandteil das Umsetzungsprodukt der Formel

(59) 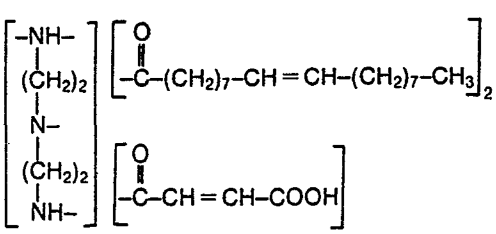

enthält. Säurezahl: 57

Beispiel 30:

320 Teile des gemäss Beispiel 4 erhaltenen Amidgemisches (0,5 Mol) werden geschmolzen und auf 110 °C aufgeheizt. Zu dieser Schmelze werden 96 Teile Trimellithsäureanhydrid (0,5 Mol) in frisch gepulverter Form gegeben. Das als Suspension vorliegende Reaktionsgemisch wird auf 160 °C aufgeheizt. Bei 120 bis 125 °C tritt eine exotherme Reaktion ein, wobei sich das suspendierte Trimellithsäureanhydrid löst. Das Reaktionsgemisch wird anschliessend während 30 Minuten bei 160 °C gehalten und dann auf Raumtemperatur (15–25 °C) abgekühlt. Man erhält 410 Teile eines hellbraun gefärbten Wachses, das als Hauptbestandteil das Umsetzungsprodukt der Formel

(60) 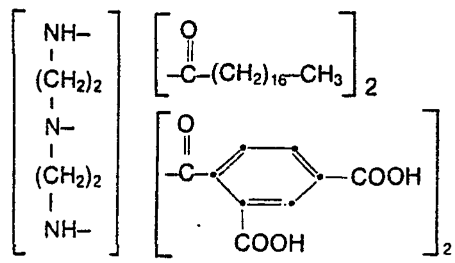

enthält. Schmelzpunkt: 67–70 °C, Säurezahl: 115.

Beispiel 31:

Man verfährt wie in Beispiel 30 angegeben, setzt jedoch 48 Teile (anstelle von 96 Teilen) Trimellithsäureanhydrid (0,25 Mol) ein. Man erhält 350 Teile eines hellbraun gefärbten Wachses, das als Hauptbestandteil das Umsetzungsprodukt der Formel

(61) 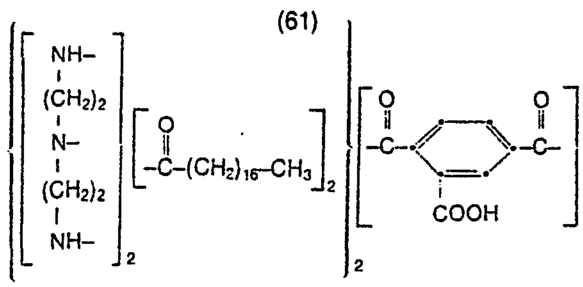

enthält. Schmelzpunkt: 68–71 °C, Säurezahl: 57.

Beispiel 32:

635 Teile des gemäss Beispiel 4 erhaltenen Amidgemisches (1 Mol) werden in 150 Teilen Chloroform suspendiert. Zu dieser Suspension werden 116 Teile Natriumchloracetat (1 Mol) und 102 Teile Triäthylamin (1 Mol) als Salzsäureempfänger gegeben. Das Reaktionsgemisch wird 1 Stunde bei Raumtemperatur (15–25 °C) unter Rühren gehalten, dann auf die Rückflusstemperatur von ca. 62 °C aufgeheizt und während 1 Stunde bei dieser Temperatur gehalten. Anschliessend wird das Lösungsmittel aus dem Reaktionsgemisch abdestilliert. Zur Aufarbeitung wird der Destillationsrückstand in 500 Teilen Wasser gelöst, während 15 Minuten unter Rühren gehalten und abfiltriert. Man erhält 670 Teile eines als schwach gelbgefärbtes Pulver vorliegenden Rohproduktes, das aus Dioxan umkristallisiert werden kann und als Hauptbestandteil das Umsetzungsprodukt der Formel

(62) 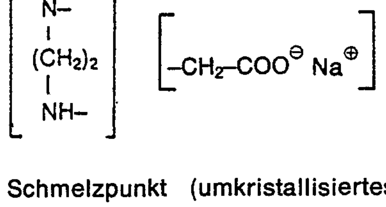

enthält. Schmelzpunkt (umkristallisiertes Produkt): 103–107 °C.

Beispiel 33:

52,3 Teile eines technischen Gemisches aus 2/3 Hexadecanol und 1/3 Octadecanol (0,2 Mol) werden in 250 Teilen Toluol bei 35 °C gelöst. Zu dieser Lösung werden 13,9 Teile Malonsäuredichlorid (0,1 Mol) innerhalb von 30 Minuten gegeben, wobei die Temperatur des Reaktionsgemisches sich von selbst auf 40 °C erhöht und Salzsäuregas freigesetzt wird. Anschliessend wird das Reaktionsprodukt auf 40 °C aufgeheizt, während 24 Stunden bei dieser Temperatur gehalten und dann auf 10 °C abgekühlt, wobei das Reaktionsprodukt ausfällt. Das abfiltrierte Rohprodukt wird aus Aceton umkristallisiert. Man erhält 49 Teile des als farblose Kristalle vorliegenden Umsetzungsproduktes der Formel

(63) 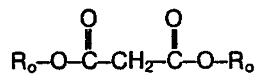

$R_o = 67\%-(CH_2)_{15}-CH_3$

$33\%-(CH_2)_{17}-CH_3$

Beispiel 34:

19 Teile Acetondicarbonsäure-diäthylester (0,1 Mol) und 54,1 Teile Octadecanol (0,2 Mol) werden auf 100 °C aufgeheizt und 24 Stunden bei dieser Temperatur gehalten, wobei die durch die Umesterungsreaktion freigesetzte, theoretische Menge Äthanol (0,2 Mol) aus dem Reaktionsgemisch abdestilliert wird. Das Reaktionsgemisch wird dann auf 20 °C abgekühlt und das Rohprodukt aus Aceton umkristallisiert. Man erhält 39 Teile eines als farbloses Pulver vorliegenden Umsetzungsproduktes der Formel

(64) 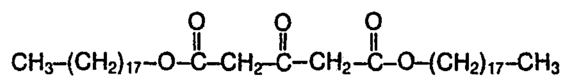

Schmelzpunkt: 58–60 °C.

Beispiel 35:

192 Teile Trimellithsäureanhydrid (1 Mol) und 540 Teile Octadecanol (2 Mol) werden auf 160 °C aufgeheizt und während 60 Minuten bei dieser Temperatur gehalten. Nach Abkühlen des Reaktionsgemisches auf 15 bis 25 °C erhält man 690 Teile eines farblosen, wachsartigen Estergemisches, das als Hauptbestandteil den Diester der Formel

(65) 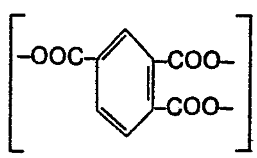 [–H] $[-(CH_2)_{17}-CH_3]_2$

enthält. Schmelzpunkt: 52–60 °C, Säurezahl: 90

Beispiel 36:

Man verfährt wie in Beispiel 35 angegeben, setzt jedoch 218 Teile Pyromellithsäuredianhydrid (1 Mol) (an Stelle von 192 Teilen Trimellithsäureanhydrid) ein. Man erhält 748 Teile eines ebenfalls farblosen, wachsartigen Estergemisches, das als Hauptbestandteil den Diester der Formel

(66) 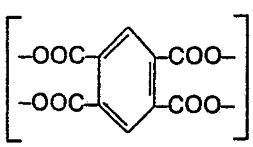 $[-H]_2$ $[-(CH_2)_{17}-CH_3]_2$

enthält. Schmelzpunkt: 81–90 °C, Säurezahl: 135

Beispiel 37:

Man verfährt wie in Beispiel 35 angegeben, setzt jedoch 218 Teile Pyromellithsäuredianhydrid (1 Mol) und 484 Teile Hexadecanol (2 Mol) ein. Man erhält 683 Teile eines ebenfalls farblosen, wachsartigen Estergemisches, das als Hauptbestandteil den Diester der Formel

(67) 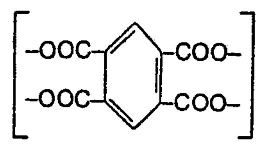 $[-H]_2$ $[-(CH_2)_{15}-CH_3]_2$

enthält. Schmelzpunkt: 35–46 °C, Säurezahl: 132

Beispiel 38:

Man verfährt wie in Beispiel 35 angegeben, setzt jedoch 218 Teile Pyromellithsäuredianhydrid (1 Mol) und 810 Teile (an Stelle von 540 Teilen) Octadecanol (3 Mol) ein. Man erhält 1011 Teile eines ebenfalls farblosen, wachsartigen Estergemisches, das als Hauptbestandteil den Triester der Formel

(68) 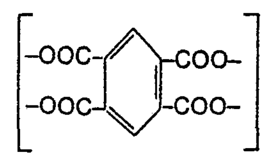 [–H] $[-(CH_2)_{17}-CH_3]_3$

enthält. Schmelzpunkt: 74–82 °C, Säurezahl: 51

Beispiel 39:

218 Teile Pyromellithsäuredianhydrid (1 Mol) und 536 Teile Oleylalkohol (2 Mol) werden in Gegenwart von 2 Teilen Hydrochinon als Polymerisationsinhibitor in inerter Stickstoffatmosphäre auf 160 °C aufgeheizt und während einer Stunde bei dieser Temperatur gehalten. Nach Abkühlen des Reaktionsgemisches auf 15 bis 25 °C erhält man 743 Teile eines hellbraunen, flüssigen, cremigen Estergemisches, das als Hauptbestandteil den Diester der Formel

(69) 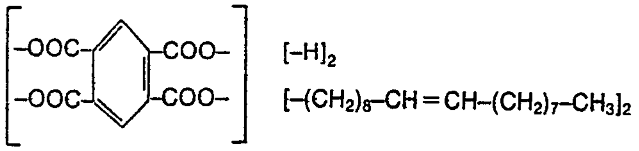

enthält. Säurezahl: 145

Beispiel 40:

109 Teile Pyromellithsäuredianhydrid (0,5 Mol) und 521 Teile Dioctadecylamin (1 Mol) werden auf 160 °C aufgeheizt und während 5 Stunden bei dieser Temperatur gehalten. Nach Abkühlen des Reaktionsgemisches auf 15 bis 25 °C erhält man 627 Teile eines ockerfarbenen, wachsartigen Amidgemisches, welches als Hauptbestandteil das Diamid der Formel

(70) 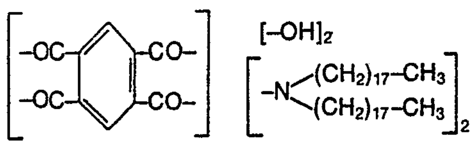

enthält. Schmelzpunkt: 45–55 °C, Säurezahl: 94

Beispiel 41:

Zu einer Lösung von 53,8 Teilen Octadecylamin (0,2 Mol) und 25,8 Teilen Chinolin (0,2 Mol) in 300 Teilen Aceton wird innerhalb von 30 Minuten eine Lösung von 21,8 Teilen Pyromellithsäuredianhydrid (0,1 Mol) in 300 Teilen Aceton gegeben, wobei die Temperatur des Reaktionsgemisches von selbst auf 40 bis 50 °C steigt, und ein weisser Niederschlag aus dem Reaktionsgemisch ausfällt. Das Reaktionsgemisch wird anschliessend während 3 Stunden unter Rühren gehalten, wobei die Temperatur von anfänglich 40 bis 50 °C nach 3 Stunden auf 15 bis 35 °C sinkt. Nach dieser Nachreaktionszeit werden dem Reaktionsgemisch 35 Teile einer wässrigen, 38%igen Salzsäurelösung und 100 Teile Wasser zugegeben, worauf das Reaktionsgemisch während 30 Minuten bei 15 bis 25 °C gehalten wird. Der ausgefallene, weisse Niederschlag wird abfiltriert und mit Wasser nachgewaschen, bis das Waschwasser einen pH-Wert von 6,0 aufweist. Man erhält nach dem Trocknen des Produktes bei 50 °C unter vermindertem Druck 71 Teile eines als weisses Pulver vorliegenden Amidgemisches, welches als Hauptbestandteil das Diamid der Formel

(71) 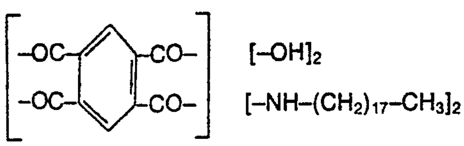

enthält. Schmelzpunkt: 175–180 °C, Säurezahl: 150

Beispiel 42:

Man verfährt wie in Beispiel 40 angegeben, setzt jedoch 161 Teile Benzophenontetracarbonsäuredianhydrid (0,5 Mol) (an Stelle von 109 Teilen Pyromellithsäuredianhydrid) ein. Man erhält 664 Teile eines ebenfalls ockerfarbenen, halbfesten, wachsartigen Amidgemisches, welches als Hauptbestandteil das Diamid der Formel

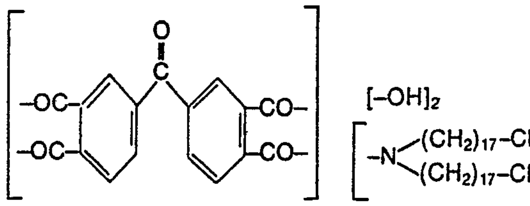 (72)

enthält. Säurezahl: 88

Beispiel 43:

Eine Lösung von 315 Teilen gemäss Beispiel 40 erhaltenen Amidgemisches (0,25 Mol) und 46 Teilen Dodecylamin (0,25 Mol) in 1500 Teilen Toluol wird auf die Rückflusstemperatur von ca. 111 °C aufgeheizt und bei dieser Temperatur während 10 Stunden gehalten, wobei das durch die Reaktion freigesetzte Wasser mit Hilfe eines Wasserabscheiders entfernt wird. Anschliessend wird das Toluol unter vermindertem Druck abdestilliert und das als Rückstand erhaltene Produkt getrocknet. Man erhält 343 Teile eines ockerfarbenen, wachsartigen Amidgemisches, welches als Hauptbestandteil das Triamid der Formel

(73) 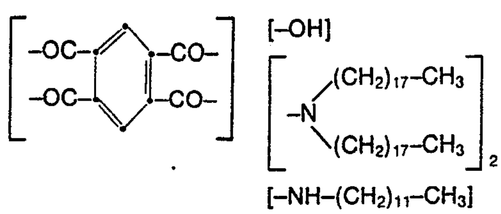

enthält. Schmelzpunkt: 81–85 °C, Säurezahl: 41

Beispiel 44:

Man verfährt wie in Beispiel 40 angegeben, setzt jedoch 105 Teile Hemimellithsäure (0,5 Mol) (an Stelle von 109 Teilen Pyromellithsäuredianhydrid) ein. Man erhält 580 Teile eines hellbraunen, wachsartigen Amidgemisches, das als Hauptbestandteil das Diamid der Formel

(74) 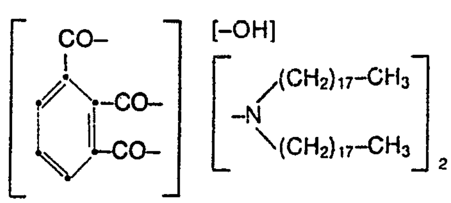

enthält. Schmelzpunkt: 46–50 °C, Säurezahl: 44

Beispiel 45:

Man verfährt wie in Beispiel 35 angegeben, setzt jedoch 210 Teile Citronensäuremonohydrat (1 Mol) (an Stelle von 192 Teilen Trimellithsäureanhydrid) ein, wobei die Reaktionsdauer bei 160 °C 90 Minuten (statt 60 Minuten) beträgt. Man erhält 672 Teile eines farblosen, wachsartigen Estergemisches, das als Hauptbestandteil den Diester der Formel

(75) 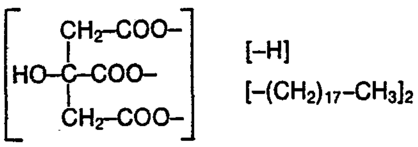

enthält. Schmelzpunkt: 48–50 °C, Säurezahl: 83

Beispiel 46:

Man verfährt wie in Beispiel 35 angegeben, setzt jedoch 210 Teile Citronensäuremonohydrat (1 Mol) (an Stelle von 192 Teilen Trimellithsäureanhydrid) und 507 Teile eines technischen Gemisches aus $^2/_3$ Hexadecanol und $^1/_3$ Octadecanol (2 Mol) (an Stelle von 540 Teilen Octadecanol) ein, wobei die Reaktionsdauer bei 160 °C 90 Minuten (statt 60 Minuten) beträgt. Man erhält 635 Teile eines gelblichen, wachsartigen Estergemisches, das als Hauptbestandteil den Diester der Formel

(76) 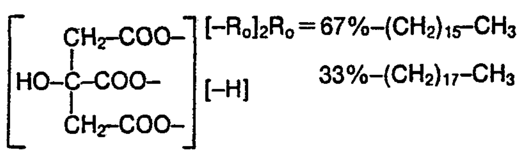

enthält. Schmelzpunkt: 44–46 °C, Säurezahl: 87

Beispiel 47:

Man verfährt wie in Beispiel 35 angegeben, setzt jedoch 210 Teile Citronensäuremonohydrat (1 Mol) (an Stelle von 192 Teilen Trimellithsäureanhydrid) und 1042 Teile Dioctadecylamin (2 Mol) (an Stelle von 540 Teilen Octadecanol) ein, wobei die Reaktionsdauer bei 160 °C 70 Minuten (statt 60 Minuten) beträgt. Man erhält 1150 Teile eines gelblichen, wachsartigen Amidgemisches, das als Hauptbestandteil das Diamid der Formel

(77) 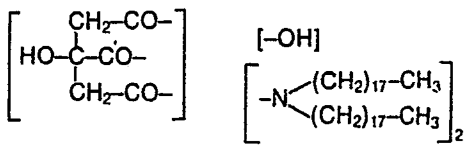

enthält. Schmelzpunkt: 49–56 °C, Säurezahl: 45

Beispiel 48:

284 Teile Stearinsäure (1 Mol), 67 Teile 2,2-Bis-(hydroxymethyl)-propionsäure (0,5 Mol) und 2,5 Teile p-Toluolsulfonsäure als Katalysator werden in 200 Teilen p-Xylol gelöst. Diese Lösung wird auf die Rückflusstemperatur von ca. 140 °C aufgeheizt und so lang bei dieser Temperatur gehalten, bis die durch die Veresterungsreaktion gebildete, theoretische Menge Wasser (1 Mol) mittels eines Wasserabscheiders azeotrop entfernt wird. Anschliessend wird das p-Xylol unter vermindertem Druck abdestilliert und das als Rückstand erhaltene Produkt getrocknet. Man erhält 306 Teile eines gelblichen, wachsartigen Estergemisches, welches als Hauptbestandteil den Diester der Formel

(78) 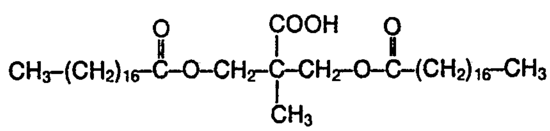

enthält. Schmelzpunkt: 39–42 °C, Säurezahl: 92.

Beispiel 49:

222,3 Teile (0,5 Mol) Sulfobernsteinsäure-bis-2-äthylhexylester-Natriumsalz und 270 Teile (1,0 Mol) Stearylalkohol werden zusammen mit 2,2 Teilen (0,04 Mol) Natriummethylat auf 165–170 °C aufgeheizt. Die erhaltene Schmelze wird 3 Stunden bei dieser Temperatur gehalten und auf 90 °C abgekühlt. Der entstandene 2-Äthyl-hexylalkohol wird unter vermindertem Druck abdestilliert.

Man erhält als Destillationsrückstand 360 Teile Sulfobernsteinsäure-bis-octadecyl-ester-Natriumsalz der Formel

(79) 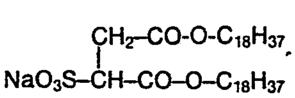

als gelbes wachsartiges Produkt.

Beispiel 50:

681 Teile des gemäss Beispiel 1 hergestellten Estergemisches (1 Mol) werden in 1000 Teilen Dichlormethan gelöst. In diese Lösung werden innerhalb von 45 Minuten 128 Teile Chlorsulfonsäure (1,1 Mol) gegeben, wobei die Temperatur durch Kühlen zwischen 25° und 35 °C gehalten und das entweichende Salzsäuregas mit Stickstoff ausgetrieben wird. Nach beendeter Chlorsulfonsäurezugabe wird das Reaktionsgemisch während einer Stunde bei 35 °C gehalten. Dann wird das Dichlormethan abdestilliert. Das als Rückstand erhaltene Rohprodukt wird aus Methyläthylketon umkristallisiert. Man erhält 620 Teile eines Estergemisches als weisses Pulver, welches als Hauptbestandteil den sauren Ester der Formel

(80) 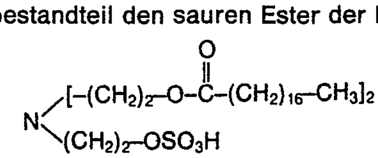

enthält. Schmelzpunkt: 80–83 °C, Säurezahl: 122.

Beispiel 51:

Man verfährt wie in Beispiel 50 angegeben, setzt jedoch anstelle von 681 Teilen des gemäss

Beispiel 1 hergestellten Estergemisches (1 Mol) 653 Teile des gemäss Beispiel 7 hergestellten Estergemisches (1 Mol) ein. Man erhält 613 Teile eines ebenfalls aus Methyläthylketon umkristallisierten, als weisses Pulver vorliegenden Gemisches, das als Hauptbestandteil den sauren Ester der Formel

(81) 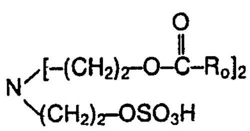 $R_o = 50\%-(CH_2)_{16}-CH_3$ $50\%-(CH_2)_{14}-CH_3$

enthält. Schmelzpunkt: 70–75 °C, Säurezahl: 105.

Beispiel 52:

Man verfährt wie in Beispiel 22 angegeben, setzt jedoch anstelle von 624 Teilen des Estergemisches, das Glycerindistearat als Hauptbestandteil (1 Mol) enthält, 681 Teile des gemäss Beispiel 1 hergestellten Estergemisches (1 Mol) ein und kristallisiert das Rohprodukt aus Methyläthylketon um. Man erhält 600 Teile eines Estergemisches als weisses Pulver, welches als Hauptbestandteil den sauren Ester der Formel

(82) 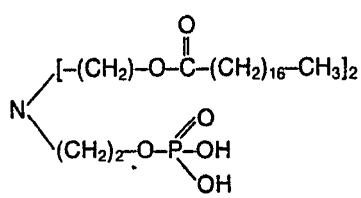

enthält. Schmelzpunkt: 78–83 °C, Säurezahl: 95.

Beispiel 53:

681 Teile des gemäss Beispiel 1 hergestellten Estergemisches (1 Mol), 147 Teile Maleinsäureanhydrid (1,5 Mol), 4 Teile p-Toluolsulfonsäure als Katalysator und 0,33 Teile Hydrochinon als Polymerisationsinhibitor werden in 1000 Teilen Toluol gelöst. Diese Lösung wird auf die Rückflusstemperatur von ca. 110 °C geheizt und während 12 Stunden bei dieser Temperatur gehalten. Die Reaktionslösung wird anschliessend von Verunreinigungen geklärt. Nach dem Abdestillieren des Lösungsmittels wird das Rohprodukt aus Aceton umkristallisiert. Man erhält 662 Teile eines Estergemisches als weisses Pulver, welches als Hauptbestandteil den sauren Ester der Formel

(83) 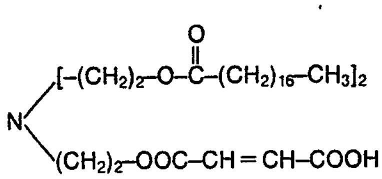

enthält. Schmelzpunkt: 53–55 °C, Säurezahl: 69.

Beispiel 54:

Man verfährt wie in Beispiel 50 angegeben, setzt jedoch 934 Teile des gemäss Beispiel 8 hergestellten Estergemisches (1 Mol), 126,8 Teile Chlorsulfonsäure (1,088 Mol) und 1555 Teile Dichlormethan ein, gibt die Chlorsulfonsäure innerhalb von 30 Minuten zu und hält das Reaktionsgemisch anschliessend während 4 Stunden bei der Rückflusstemperatur von 35 °C. Man erhält 867 Teile eines ebenfalls aus Methyläthylketon umkristallisierten Estergemisches als hellbeiges Pulver, welches als Hauptbestandteil den sauren Ester der Formel

(84) 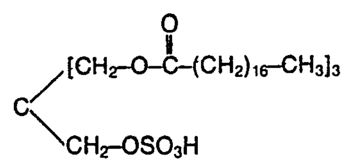

enthält. Schmelzpunkt: 60–64 °C, Säurezahl: 49,5.

Beispiel 55:

Man verfährt wie in Beispiel 50 angegeben, setzt jedoch 635 Teile des gemäss Beispiel 9 hergestellten Estergemisches (1 Mol), 128 Teile Chlorsulfonsäure (1,1 Mol) und 1500 Teile Dichlormethan ein, gibt die Chlorsulfonsäure innerhalb von 30 Minuten zu und kristallisiert das Rohprodukt aus Aceton um. Man erhält 622 Teile eines Estergemisches als weisses Pulver, welches als Hauptbestandteil den sauren Ester der Formel

(85) 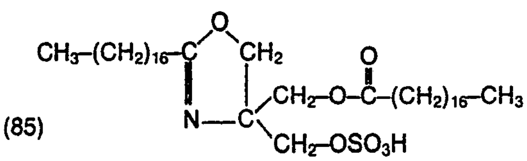

enthält. Schmelzpunkt: 66–71 °C, Säurezahl: 75.

Beispiel 56:

Man verfährt wie in Beispiel 53 angegeben, setzt jedoch 635 Teile des gemäss Beispiel 9 hergestellten Estergemisches (1 Mol), 98 Teile Maleinsäureanhydrid (1 Mol), 18 Teile p-Toluolsulfonsäure, 1 Teil Hydrochinon und 1500 Teile p-Xylol ein und hält das Reaktionsgemisch während 8 Stunden bei 110 °C. Man erhält 681 Teile eines ebenfalls aus Aceton umkristallisierten Estergemisches, welches als Hauptbestandteil den sauren Ester der Formel

(86) 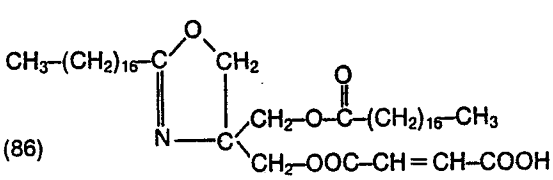

enthält. Schmelzpunkt: 53–55 °C, Säurezahl: 53.

Beispiel 57:

Man verfährt wie in Beispiel 50 angegeben, setzt jedoch 624 Teile des in Beispiel 22 verwendeten Gemisches aus 1,3- und 1,2-Glycerindistearat (1 Mol), 128 Teile Chlorsulfonsäure (1,1 Mol) und 666 Teile Dichlormethan ein, gibt die Chlorsulfonsäure innerhalb von 30 Minuten zu und kristallisiert das Rohprodukt aus Aceton um. Man erhält 616 Teile eines Estergemisches, das als Hauptbestandteil den sauren Ester der Formel

(87) 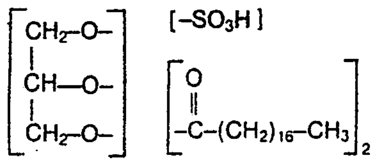

enthält. Schmelzpunkt: 54–56 °C, Säurezahl: 71.

Beispiel 58:

Man verfährt wie in Beispiel 50 angegeben, setzt jedoch 595 Teile des gemäss Beispiel 10 hergestellten Estergemisches (1 Mol), 128 Teile Chlorsulfonsäure (1,1 Mol) und 800 Teile Dichlormethan ein, gibt die Chlorsulfonsäure innerhalb von 30 Minuten zu und kristallisiert das Rohprodukt aus Aceton um. Man erhält als weisses Pulver 618 Teile eines Estergemisches, das als Hauptbestandteil den sauren Ester der Formel

(88) 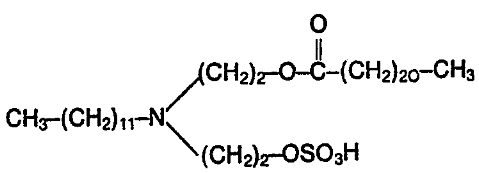

enthält. Schmelzpunkt: 65–70 °C, Säurezahl: 79.

Beispiel 59:

Man verfährt wie in Beispiel 53 angegeben, setzt jedoch 1090 Teile des gemäss Beispiel 11 hergestellten Estergemisches (1 Mol), 98 Teile Maleinsäureanhydrid (1 Mol), 20 Teile p-Toluolsulfonsäure und 2,2 Teile Hydrochinon in 3333 Teilen Toluol ein. Man erhält 1027 Teile eines ebenfalls aus Aceton umkristallisierten, wachsartigen Estergemisches, welches als Hauptbestandteil den sauren Ester der Formel

(89) 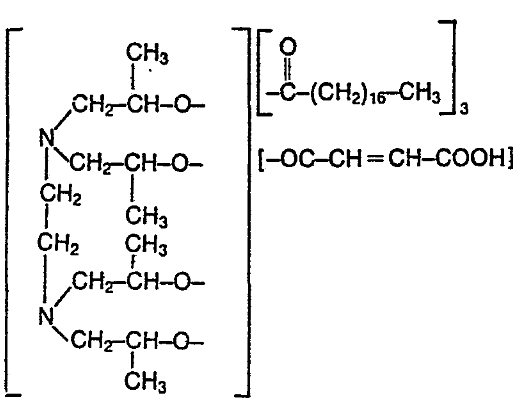

enthält. Schmelzpunkt: 37–38 °C, Säurezahl: 35,6.

Beispiel 60:

Man verfährt wie in Beispiel 50 angegeben, setzt jedoch 1090 Teile des gemäss Beispiel 11 hergestellten Estergemisches (1 Mol), 116 Teile Chlorsulfonsäure (1 Mol) und 2500 Teile Dichlormethan ein und kristallisiert das Rohprodukt aus Aceton um. Man erhält 1006 Teile eines Estergemisches als weisses Pulver, welches als Hauptbestandteil den sauren Ester der Formel

(90) 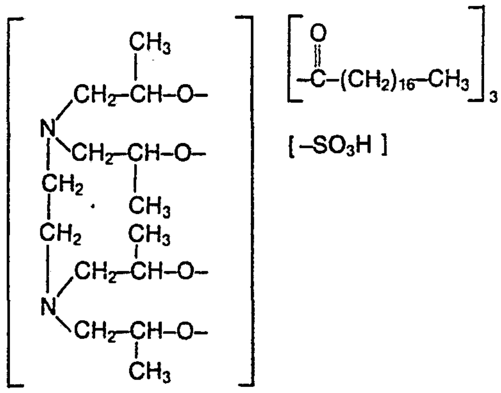

enthält. Schmelzpunkt: 65–70 °C, Säurezahl: 55.

Beispiel 61:

Man verfährt wie in Beispiel 50 angegeben, setzt jedoch 92,1 Teile des gemäss Beispiel 2 erhaltenen Diäthers (0,15 Mol), 17,5 Teile Chlorsulfonsäure (0,15 Mol) und 350 Teile Dichlormethan ein und kristallisiert das Rohprodukt aus Essigsäure-äthylester um. Man erhält 85,3 Teile einer sulfonierten Verbindung der Formel

(91) 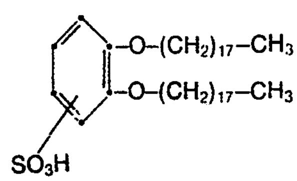

Schmelzpunkt: 78–82 °C, Säurezahl: 90.

Beispiel 62:

Man verfährt wie in Beispiel 50 angegeben, setzt jedoch 61,4 Teile des gemäss Beispiel 3 erhaltenen Diäthers (0,1 Mol), 11,56 Teile Chlorsulfonsäure (0,1 Mol) und 300 Teile Dichlormethan ein und kristallisiert das Rohprodukt aus Essigsäure-äthylester um. Man erhält 61,8 Teile einer sulfonierten Verbindung der Formel

(92) 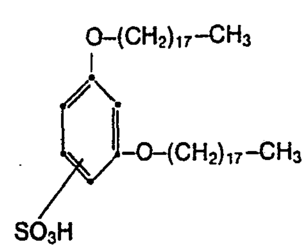

Schmelzpunkt: 71–73 °C, Säurezahl: 106.

Beispiel 63:

95,9 Teile des gemäss Beispiel 12 erhaltenen Estergemisches (0,15 Mol) und 21,3 Teile Phosphorpentoxyd (0,15 Mol) werden in 380 Teilen

Toluol gelöst. Diese Lösung wird auf 85 °C aufgeheizt und 6 Stunden bei dieser Temperatur unter Rühren gehalten. Anschliessend wird die trübe Reaktionslösung filtriert. Aus dem Filtrat wird das Lösungsmittel unter vermindertem Druck abdestilliert. Das als Destillationsrückstand vorliegende Rohprodukt wird aus Aceton umkristallisiert. Man erhält 103 Teile eines als beigefarbenes Pulver vorliegenden Estergemisches, das als Hauptbestandteil den sauren Ester der Formel

(93)

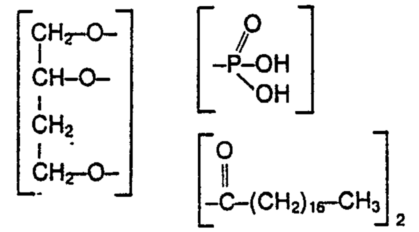

enthält. Schmelzpunkt: 48–50 °C, Säurezahl: 65.

Beispiel 64:

Man verfährt wie in Beispiel 53 angegeben, setzt jedoch 158,6 Teile des gemäss Beispiel 13 hergestellten Estergemisches (0,2 Mol), 19,6 Teile Maleinsäureanhydrid (0,2 Mol), 1,5 Teile p-Toluolsulfonsäure und 0,5 Teile Hydrochinon in 150 Teilen p-Xylol ein. Man erhält 109 Teile eines ebenfalls aus Aceton umkristallisierten, als weisses Pulver vorliegenden Estergemisches, das als Hauptbestandteil den sauren Triester der Formel

(94)

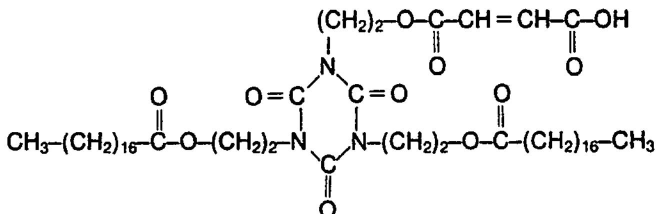

enthält. Schmelzpunkt: 65–67 °C, Säurezahl: 53.

Beispiel 65:

332 Teile der gemäss Beispiel 14 erhaltenen Verbindung der Formel (44) (0,3 Mol) werden in 1000 Teilen Isopropanol bei 60 °C gelöst. Zu dieser Lösung wird eine Lösung von 29,4 Teilen Maleinsäureanhydrid (0,3 Mol) in 500 Teilen Methyläthylketon gegeben, wobei die Temperatur des Reaktionsgemisches bei 60 °C gehalten wird. Das Reaktionsgemisch wird dann während 3 Stunden bei dieser Temperatur gehalten und dann auf 15 °C abgekühlt, wobei das Reaktionsprodukt ausfällt. Das Produkt wird abgenutscht und unter Vakuum bei 35 °C getrocknet. Man erhält 319 Teile eines farblosen Estergemisches, das als Hauptbestandteil die Verbindung der Formel

(95)

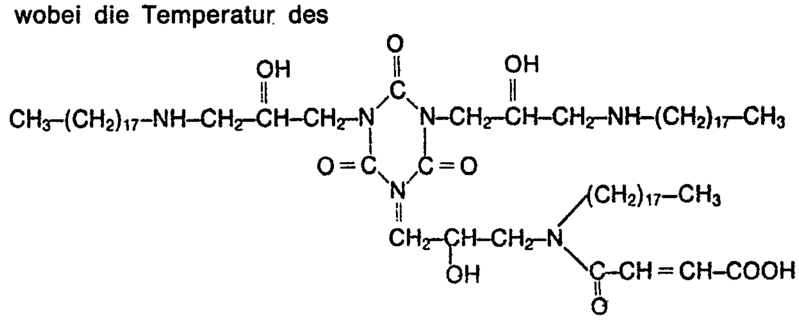

enthält. Schmelzpunkt: 62–65 °C, Säurezahl: 47.

Beispiel 66:

Man verfährt wie in Beispiel 22 angegeben, setzt jedoch 229,5 Teile der gemäss Beispiel 15 erhaltenen Verbindung der Formel (45) (0,2 Mol) gelöst in 1800 Teilen Tetrachlorkohlenstoff und eine Suspension von 19 Teilen Phosphorpentoxyd (0,13 Mol) in 500 Teilen Tetrachlorkohlenstoff ein und führt die Reaktion während 3 Stunden bei 50 °C durch. Man erhält 179 Teile eines ebenfalls aus Methyläthylketon auskristallisierten, hellbeigefarbenen Estergemisches, das als Hauptbestandteil den sauren Ester der Formel

(96)

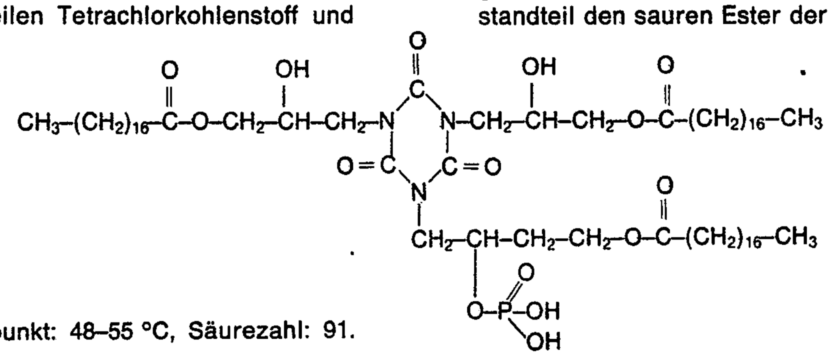

enthält. Schmelzpunkt: 48–55 °C, Säurezahl: 91.

Beispiel 67:

153,8 Teile der gemäss Beispiel 17 erhaltenen Verbindung der Formel (47) (0,1 Mol) werden mit 10 Teilen Bernsteinsäureanhydrid (0,1 Mol) vermischt, bei 180 °C geschmolzen und während 6 Stunden bei dieser Temperatur gehalten. Das erhaltene Rohprodukt wird aus Essigsäure-äthylester umkristallisiert und bei 45 °C unter vermindertem Druck getrocknet. Man erhält 121,1 Teile eines farblosen Estergemisches, das als Hauptbestandteil den sauren Ester der Formel

(97) 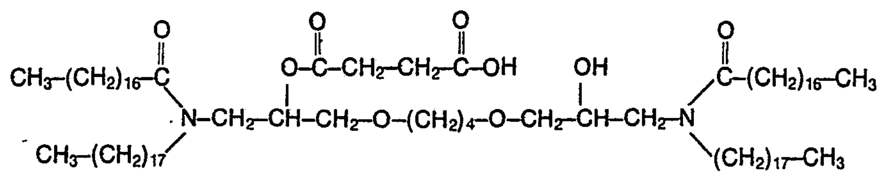

enthält. Schmelzpunkt: 62–65 °C, Säurezahl: 41.

Beispiel 68:

Man verfährt wie in Beispiel 23 angegeben, setzt jedoch 63 Teile Dimethylmaleinsäureanhydrid (0,5 Mol) ein. Man erhält 360 Teile eines als weissliches Pulver vorliegenden Rohproduktes, das aus Essigsäureäthylester umkristallisiert werden kann und als Hauptbestandteil das saure Amid der Formel

(98) 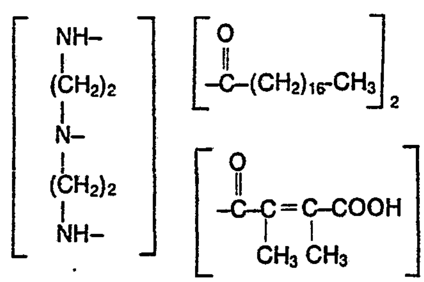

enthält.
Schmelzpunkt (umkristallisiertes Produkt): 66–72 °C
Säurezahl (umkristallisiertes Produkt): 67

Beispiel 69:

Man verfährt wie in Beispiel 23 angegeben, setzt jedoch 56 Teile Itaconsäureanhydrid (0,5 Mol) ein. Man erhält 360 Teile eines als cremefarbenes Pulver vorliegenden Rohproduktes, das aus Aceton umkristallisiert werden kann und als Hauptbestandteil das Umsetzungsprodukt der Formel

(99) 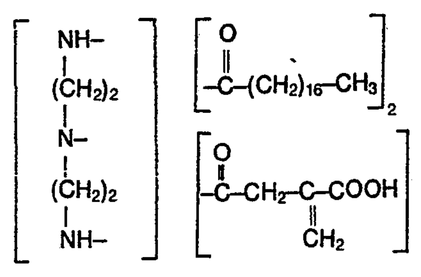

enthält.
Schmelzpunkt (umkristallisiertes Produkt): 79–83 °C
Säurezahl (umkristallisiertes Produkt): 87

Beispiel 70:

Man verfährt wie in Beispiel 23 angegeben, setzt jedoch 55 Teile Trimellithsäureanhydrid (0,25 Mol) ein. Man erhält 360 Teile eines als weisses Pulver vorliegenden Rohproduktes, das aus Essigsäureäthylester umkristallisiert werden kann und als Hauptbestandteil das Umsetzungsprodukt der Formel

(100)
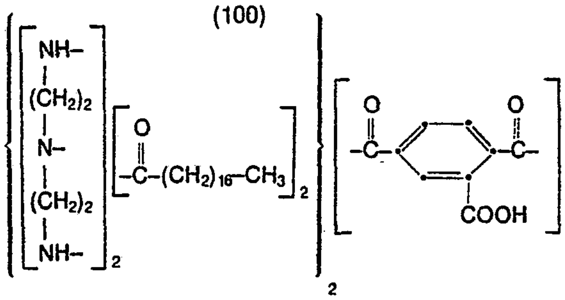

enthält.
Schmelzpunkt (umkristallisiertes Produkt): 124–130 °C
Säurezahl (umkristallisiertes Produkt): 80

Beispiel 71:

Man verfährt wie in Beispiel 23 angegeben, setzt jedoch 41 Teile Propansulton (0,5 Mol) ein. Man erhält 260 Teile eines als ockerfarbenes Pulver vorliegenden Rohproduktes, das aus Äthanol umkristallisiert werden kann und als Hauptbestandteil das Umsetzungsprodukt der Formel

(101) 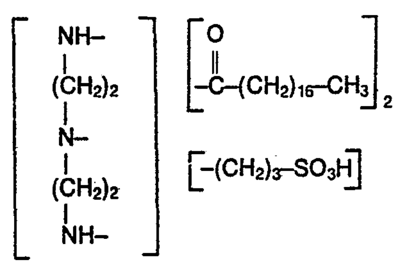

enthält.
Schmelzpunkt (umkristallisiertes Produkt): 96–99 °C
Säurezahl (umkristallisiertes Produkt): 49

Beispiel 72:

320 Teile des gemäss Beispiel 4 erhaltenen Amidgemisches (0,5 Mol) werden bei 100 °C geschmolzen. In diese Schmelze werden 82 Teile Bi-

cyclo(2,2,1)-hept-5-en-2,3-dicarbonsäureanhydrid (0,5 Mol) gegeben. Das Reaktionsgemisch wird unter Rühren gehalten, wobei sich nach 2 bis 3 Minuten das Anhydrid löst und die Temperatur des Reaktionsgemisches sich innerhalb von ca. 10 Minuten von selbst auf 115 bis 120 °C erhöht. Das Reaktionsgemisch wird während 30 Minuten bei 120 °C gehalten und dann auf Raumtemperatur (15–25 °C) abgekühlt. Man erhält 380 Teile eines hellbraun gefärbten Wachses, das als Hauptbestandteil das Umsetzungsprodukt der Formel

(102) 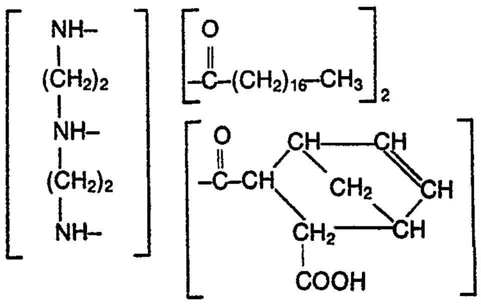

enthält. Schmelzpunkt: 63–64 °C, Säurezahl: 61.

Beispiel 73:

Zu 347 Teilen des gemäss Beispiel 18 erhaltenen Umsetzungsproduktes (0,5 Mol), das als Lösung in 160 Teilen Toluol bei 60 °C vorliegt, wird eine Lösung von 49 Teilen Maleinsäureanhydrid (0,5 Mol) in 40 Teilen Toluol innerhalb von 15 Minuten gegeben, wobei sich die Temperatur des Reaktionsgemisches von selbst auf 70 °C erhöht. Anschliessend wird das Reaktionsgemisch auf die Rückflusstemperatur von ca. 111 °C aufgeheizt, während 2 Stunden bei dieser Temperatur gehalten und dann auf 60 °C abgekühlt. Das Lösungsmittel wird hierauf aus dem Reaktionsgemisch unter vermindertem Druck abdestilliert. Als Destillationsrückstand erhält man 384 Teile eines als beigefarbenes Pulver vorliegenden Rohproduktes, das aus Dioxan umkristallisiert werden kann und als Hauptbestandteil das saure Amid der Formel

(103) 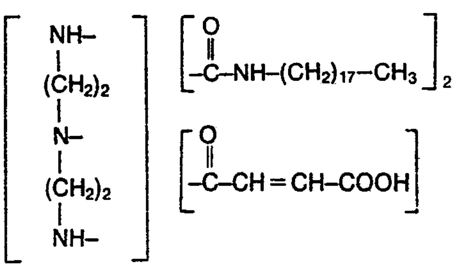

enthält.
Schmelzpunkt (umkristallisiertes Produkt): 112–130 °C
Säurezahl (umkristallisiertes Produkt): 64

Beispiel 74:

195 Teile des gemäss Beispiel 19 erhaltenen Produktgemisches (0,3 Mol) werden in 900 Teilen Toluol gelöst. Zu dieser Lösung wird eine Lösung von 30 Teilen (0,3 Mol) Bernsteinsäureanhydrid in 300 Teilen Methyläthylketon bei 50 °C gegeben. Das Reaktionsgemisch wird anschliessend auf 65 °C aufgeheizt und während 3 Stunden bei dieser Temperatur gehalten. Dann wird das Lösungsmittel aus dem Reaktionsgemisch unter vermindertem Druck abdestilliert. Das als Destillationsrückstand vorliegende Rohprodukt wird aus Essigsäureäthylester umkristallisiert. Man erhält 175 Teile eines als beige gefärbtes Pulver vorliegenden Amidgemisches, das als Hauptbestandteil das Umsetzungsprodukt der Formel

(104) 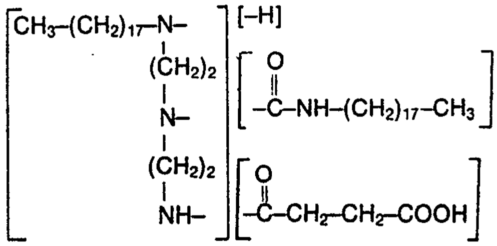

enthält. Schmelzpunkt: 55–58 °C, Säurezahl: 74.

Beispiel 75:

Man verfährt wie in Beispiel 74 angegeben, setzt jedoch 444 Teile Phthalsäureanhydrid (0,3 Mol) (anstelle von 30 Teilen Bernsteinsäureanhydrid) ein. Man erhält 192 Teile eines als beige gefärbtes Pulver vorliegenden Amidgemisches, das als Hauptbestandteil das Umsetzungsprodukt der Formel

(105) 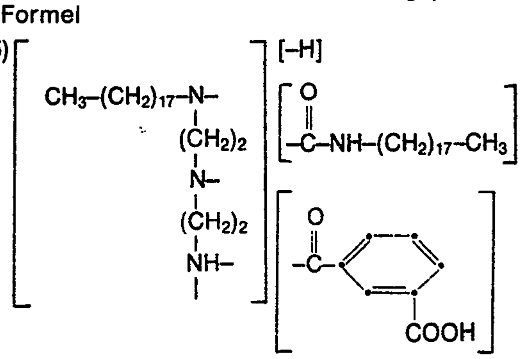

enthält. Schmelzpunkt: 65–70 °C, Säurezahl: 70.

Beispiel 76:

Man verfährt wie in Beispiel 74 angegeben, setzt jedoch 34,2 Teile Glutarsäureanhydrid (0,3 Mol) ein. Man erhält 166 Teile eines als beige gefärbtes Pulver vorliegenden Amidgemisches, das als Hauptbestandteil das Umsetzungsprodukt der Formel

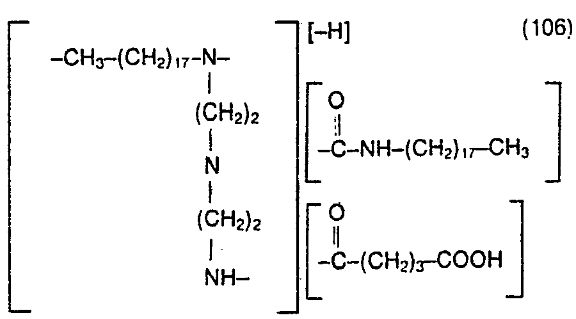 (106)

enthält. Schmelzpunkt: 53–56 °C, Säurezahl: 73.

Beispiel 77:

Man verfährt wie in Beispiel 74 angegeben, setzt jedoch 186,6 Teile des gemäss Beispiel 20 erhaltenen Produktgemisches (0,3 Mol) und 29,4 Teile Maleinsäureanhydrid (0,3 Mol) ein, wobei das Maleinsäureanhydrid in 300 Teilen Toluol (statt Methyläthylketon) gelöst wird. Man erhält 182 Teile eines als beige gefärbtes Pulver vorliegenden Amidgemisches, das als Hauptbestandteil das Umsetzungsprodukt der Formel

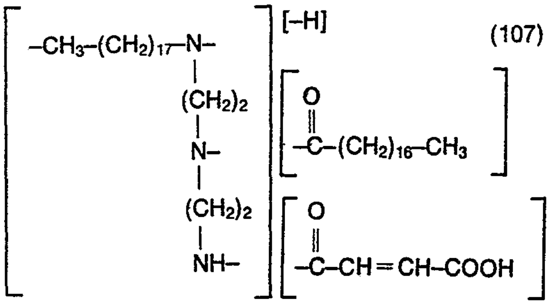

(107)

enthält. Schmelzpunkt: 62–67 °C. Säurezahl: 78.

Beispiel 78:

116 Teile Methantricarbonsäure-Triäthylester (0,5 Mol) und 406 Teile Octadecanol (1,5 Mol) werden auf 140 °C aufgeheizt und 17 Stunden bei dieser Temperatur gehalten, wobei die durch die Umesterungsreaktion freigesetzte, theoretische Menge Äthanol (1,5 Mol) aus dem Reaktionsgemisch abdestilliert wird. Das Reaktionsgemisch wird dann auf 20 °C abgekühlt und das Rohprodukt aus Aceton umkristallisiert. Man erhält 300 Teile eines als farbloses Pulver vorliegenden Umsetzungsproduktes der Formel

(108)

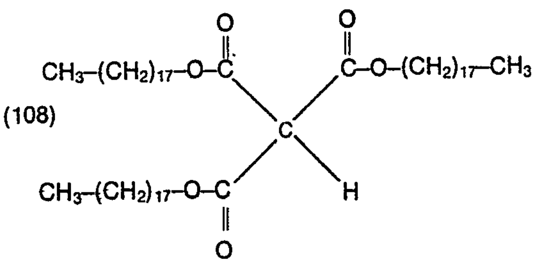

Schmelzpunkt: 50–54 °C.

Beispiel 79:

67,2 Teile des gemäss Beispiel 21 erhaltenen Zwischenproduktes (0,2 Mol) werden in 300 Teilen Dimethylformamid bei Raumtemperatur (15–25 °C) gelöst und mit 16 Teilen einer 50%igen Natriumhydroxydlösung (0,2 Mol) versetzt, wobei eine Lösung des entsprechenden Natriumsalzes entsteht. Nun werden zu dieser Lösung 16,8 Teile 1,6-n-Hexan-diisocyanat (0,1 Mol) innerhalb von 15 Minuten bei 30 °C gegeben. Anschliessend wird das Reaktionsgemisch während 4 Stunden bei 30 °C gehalten, mit 1000 Teilen Wasser verdünnt und mit einer wässerigen Essigsäurelösung auf den pH-Wert von 4,5 gestellt, wobei das Reaktionsprodukt als freie Säure ausfällt. Das Rohprodukt wird abfiltriert und aus Essigsäureäthylester umkristallisiert. Man erhält 61,3 Teile eines als weisses Pulver vorliegenden Umsetzungsproduktes der Formel

(109)

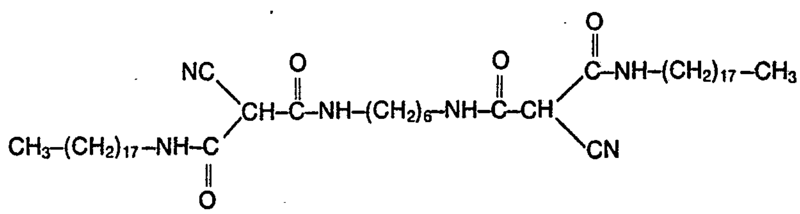

Schmelzpunkt: 116–120 °C.

Beispiel 80:

Man verfährt wie in Beispiel 79 angegeben, löst jedoch das gemäss Beispiel 21 erhaltene Zwischenprodukt in 200 Teilen Dimethylsulfoxyd (an Stelle von 300 Teilen Dimethylformamid) und setzt 17,4 Teile Toluylen-diisocyanat (0,1 Mol) als technisches Gemisch aus etwa 80% 1-Toluylen-2,4-diisocyanat und etwa 20% 1-Toluylen-2,6-diisocyanat (an Stelle von 16,8 Teilen 1,6-n-Hexandiisocyanat) ein. Man erhält 68 Teile eines als hellgelbes Pulver vorliegenden Umsetzungsproduktgemisches, das etwa 80% des Umsetzungsproduktes der Formel

(110)

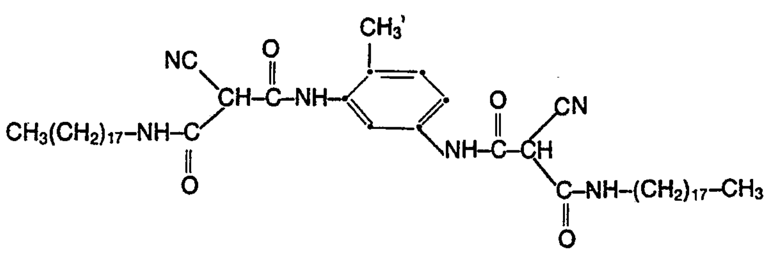

und etwa 20% des Umsetzungsproduktes der Formel

(111) 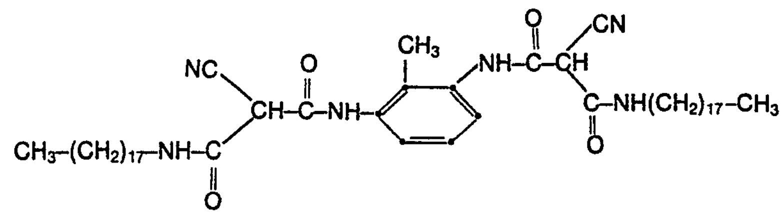

enthält. Schmelzpunkt: 116–120 °C.

Beispiel 81:

Man verfährt wie in Beispiel 35 angegeben, setzt jedoch 322 Teile Benzophenontetracarbonsäuredianhydrid (1 Mol) (an Stelle von 192 Teilen Trimellithsäureanhydrid) ein. Man erhält 823 Teile eines ebenfalls farblosen, wachsartigen Estergemisches, das als Hauptbestandteil den Diester der Formel

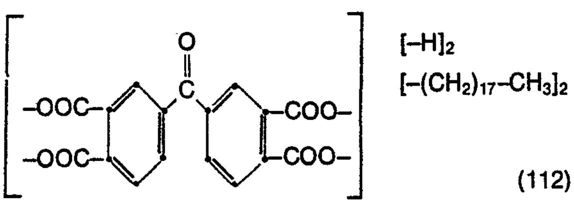 (112)

enthält. Schmelzpunkt: 46–47 °C, Säurezahl: 110.

Beispiel 82:

Man verfährt wie in Beispiel 35 angegeben, setzt jedoch 176 Teile Tricarballylsäure (1 Mol) (an Stelle von 192 Trimellithsäureanhydrid) ein. Man erhält 650 Teile eines farblosen, wachsartigen Estergemisches, das als Hauptbestandteil den Diester der Formel

(113) 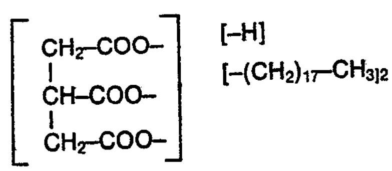

enthält. Schmelzpunkt: 43–45 °C, Säurezahl: 85.

Beispiel 83:

Man verfährt wie in Beispiel 35 angegeben, setzt jedoch 210 Teile Citronensäuremonohydrat (1 Mol) (an Stelle von 192 Teilen Trimellithsäureanhydrid), 270 Teile Octadecanol (1 Mol) und 521 Teile Dioctadecylamin (1 Mol) (an Stelle von 540 Teilen Octadecanol) ein. Man erhält 909 Teile eines gelblichen, wachsartigen Amid- und Estergemisches, das als Hauptbestandteil den Amidester der Formel

(114) 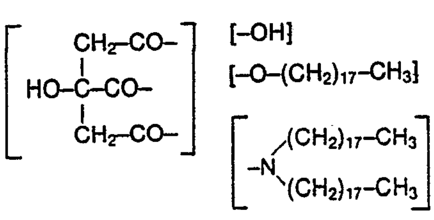

enthält. Schmelzpunkt: 54–67 °C, Säurezahl: 63.

Beispiel 84:

Man verfährt wie in Beispiel 35 angegeben, setzt jedoch 174 Teile trans-Aconitsäure (1 Mol) (an Stelle von 192 Teilen Trimellithsäureanhydrid) ein und führt die Reaktion in Gegenwart von 1 Teil Hydrochinon als Polymerisationsinhibitor durch, wobei die Reaktionsdauer bei 160 °C 30 Minuten (anstatt 60 Minuten) beträgt. Man erhält 650 Teile eines farblosen, wachsartigen Estergemisches, das als Hauptbestandteil den Diester der Formel

(115) 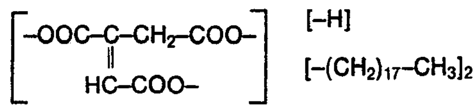

enthält. Schmelzpunkt: 42–43 °C, Säurezahl: 83.

Beispiel 85:

Man verfährt wie in Beispiel 35 angegeben, setzt jedoch 87 Teile trans-Aconitsäure (0,5 Mol) (an Stelle von 192 Teilen Trimellithsäureanhydrid) und 521 Teile Dioctadecylamin (1 Mol) (an Stelle von 540 Teilen Octadecanol) ein und führt die Reaktion in Gegenwart von 1 Teil Hydrochinon als Polymerisationsinhibitor durch. Man erhält 560 Teile (98% der Theorie) eines gelblichen, wachsartigen Amidgemisches, das als Hauptbestandteil das Diamid der Formel

(116) 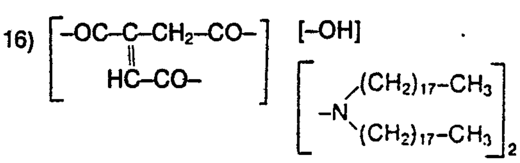

enthält. Schmelzpunkt: 52–57 °C, Säurezahl: 47

Beispiel 86:

Man verfährt wie in Beispiel 48 angegeben, setzt jedoch 170,4 Teile (an Stelle von 284 Teilen) Stearinsäure (0,6 Mol), 48,9 Teile N,N-Bis(2-hydroxyäthyl)-glycin (0,3 Mol) (an Stelle von 67 Teilen 2,2-Bis(hydroxymethyl)-propionsäure), 1,2 Teile (an Stelle von 2,5 Teilen) p-Toluolsulfonsäure und 170 Teile (an Stelle von 200 Teilen) p-Xylol ein, wobei 0,6 Mol (anstatt 1 Mol) Wasser azeotrop entfernt wird. Man erhält 208 Teile eines gelblichen, wachsartigen Estergemisches, welches als Hauptbestandteil den Diester der Formel

(117)

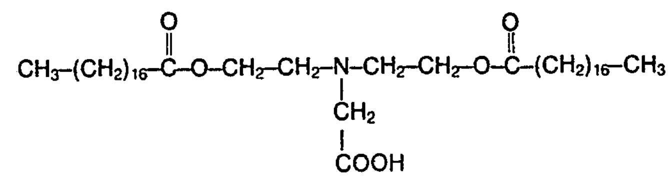

enthält. Schmelzpunkt: 64–68 °C, Säurezahl: 106

Beispiel 87:

95,6 Teile Nitrilotriessigsäure (0,5 Mol) und 522 Teile Dioctadecylamin (1 Mol) werden auf 160 °C aufgeheizt und 6 Stunden bei dieser Temperatur gehalten. Nach Abkühlen des Reaktionsgemisches auf 15 bis 25 °C erhält man 565 Teile eines ockerfarbenen, wachsartigen Amidgemisches, das als Hauptbestandteil das Diamid der Formel

(118)

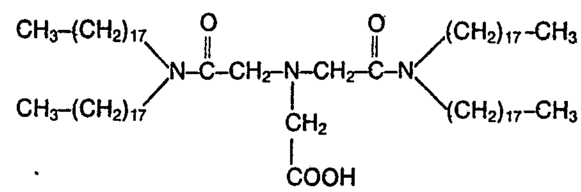

enthält. Schmelzpunkt: 44–51 °C, Säurezahl: 49

Applikationsbeispiele:

Beispiele 88 bis 109:

Eine Faserstoffsuspension aus gebleichtem Birkensulfatzellstoff und Kiefernsulfatzellstoff im Gewichtsverhältnis 1:1 in Wasser von 10° dH (deutsche Härtegrade), die ein Schopper-Riegler Mahlgrad von 35° und einen Feststoffgehalt von 0,5% aufweist, wird gegebenenfalls mit 20% des in der nachfolgenden Tabelle I angegebenen Füllmittels und hierauf mit 0,01% PERCOL 292® (kationisches, hochmolekulares (MG > $1 \cdot 10^7$) Polyacrylamid) als Hilfsmittel zum Zurückhalten feinster Zellstoffaserteilchen versetzt, wobei sich der ebenfalls in der Tabelle I angegebene pH-Wert der Faserstoffsuspension einstellt. Die Prozentangaben beziehen sich auf Trockensubstanz an Hilfs- und Füllmittel, bezogen auf den Feststoffgehalt der Faserstoffsuspension.

Die ebenfalls in der nachfolgenden Tabelle I angegebenen Formulierungen des Leimungsmittels werden hergestellt, indem die angegebenen Leimungsmittel in Pulverform oder auch als Reaktionsgemische, wie sie bei der Herstellung anfallen, mit jeweils dem nachstehend beschriebenen Retentionsmitteln in Gegenwart von entionisiertem Wasser und von Glasperlen mit einem Durchmesser von 2 mm bei Raumtemperatur (15 bis 25 °C) verrührt werden. Die erhaltenen Dispersionen sind giessbar, homogen und lagerstabil. Die Prozentangaben in den Formulierungen beziehen sich auf die Trockensubstanz an Leimungs- und Retentionsmittel, bezogen auf das Gesamtgewicht der Formulierung. Retentionsmittel Nr. 1 ist POLYMIN P® (Polyäthylenimin eines Molekulargewichts von 10 000 bis 100 000). Retentionsmittel Nr. 2 ist CATO 110® (kationisch modifizierte Stärke, die mit einem quaternären Ammoniumgruppen enthaltenden Propylenoxyd modifiziert ist und deren pH-Wert der 25%igen Anschlämmung in destilliertem Wasser bei 20 °C 4,2 bis 4,6 beträgt). Retentionsmittel Nr. 3 ist ein Kondensationsprodukt aus Dicyandiamid und Triäthylentetramin, das mit Epichlorhydrin weiter umgesetzt und gemäss Beispiel 2 der DE-A-2 710 061 hergestellt wird.

Nun wird die wässrige Formulierung des Leimungsmittels und des Retentionsmittels zur Faserstoffsuspension so gegeben, dass eine Einsatzmenge von 0,5% an Trockensubstanz des Leimungsmittels, bezogen auf den Feststoffgehalt der Faserstoffsuspension, entsteht. Anschliessend wird die Faserstoffsuspension in einem Labor-Blattbildner «Formette Dynamique» der Fa. Allimand, Grenoble, Frankreich, zu Papierblättern verarbeitet, die nach einer ersten Trocknung bei 130 °C während 3 Minuten ein Flächengewicht von 80 g/$m^2$ aufweisen. Das so erhaltene Papierblatt wird einer zusätzlichen Wärmebehandlung bei 140 °C während 3 Minuten unterworfen.

Beide Oberflächen der erhaltenen Papierblätter, d.h. die auf der Siebseite des Blattbildners erhaltene Oberfläche und die Gegen- oder Oberseite werden auf ihre Leimungseigenschaften geprüft. Zu diesem Zweck wird die Wasseraufnahme nach Cobb bei 30 Sekunden Einwirkungsdauer (WA $Cobb_{30}$) gemäss DIN 53 132 gemessen. Die Ergebnisse der WA $Cobb_{30}$-Messungen in g/$m^2$ der Siebseite (SS) und Oberseite (OS) vor und nach der Wärmebehandlung bei 140 °C und vor und nach einer Lagerung von einem Tag bei 20 °C sind in der nachfolgenden Tabelle I angegeben. Je geringer die Wasseraufnahme, desto besser ist die Leimung des Papiers. WA $Cobb_{30}$-Werte über 100 entsprechen einer völlig unbefriedigenden Leimung des Papiers.

Tabelle I

| Beispiel Nr. | Formulierung | Füllmittel | pH-Wert Suspension | WA Cobb$_{30}$ (g/m$^2$) nach Trocknung sofort SS | OS | nach 1 Tag Lagerung SS | OS | nach Wärmebehandl. sofort SS | OS | nach 1 Tag Lagerung SS | OS |
|---|---|---|---|---|---|---|---|---|---|---|---|
| 88 | 7% Leimungsmittel gem. Beisp. 50<br>3,5% Retentionsmittel Nr. 1 | Kreide | 8,4 | 21 | 11 | 17 | 11 | 16 | 12 | 14 | 11 |
| 89 | 7% Leimungsmittel gem. Beisp. 50<br>3,5% Retentionsmittel Nr. 2<br>3,5% Retentionsmittel Nr. 3 | Kreide | 8,3 | 26 | 15 | 24 | 13 | 14 | 11 | 15 | 13 |
| 90 | 7% Leimungsmittel gem. Beisp. 51<br>3,5% Retentionsmittel Nr. 2<br>3,5% Retentionsmittel Nr. 3 | Kreide | 7,8 | 51 | 19 | 46 | 19 | 17 | 30 | 13 | 15 |
| 91 | 7% Leimungsmittel gem. Beisp. 52<br>3,5% Retentionsmittel Nr. 1 | Kreide | 8,2 | 21 | 13 | 15 | 10 | 18 | 14 | 15 | 8 |
| 92 | 7% Leimungsmittel gem. Beisp. 53<br>3,5% Retentionsmittel Nr. 1 | Kreide | 8,4 | 84 | 28 | 15 | 15 | 47 | 20 | 43 | 12 |
| 93 | 7% Leimungsmittel gem. Beisp. 54<br>3,5% Retentionsmittel | Kreide | 9,0 | 24 | 13 | 22 | 11 | 19 | 12 | 17 | 13 |
| 94 | 7% Leimungsmittel gem. Beisp. 55<br>3,7% Retentionsmittel Nr. 1 | Kreide | 9,0 | 16 | 13 | 14 | 11 | 18 | 14 | 17 | 14 |
| 95 | 7% Leimungsmittel gem. Beisp. 56<br>3,5% Retentionsmittel Nr. 1 | Kreide | 8,4 | 18 | 13 | 15 | 9 | 16 | 12 | 13 | 9 |
| 96 | 7% Leimungsmittel gem. Beisp. 57<br>3,5% Retentionsmittel Nr. 1 | Kreide | 8,0 | 39 | 14 | 28 | 10 | 19 | 14 | 17 | 11 |
| 97 | 7% Leimungsmittel gem. Beisp. 58<br>3,5% Retentionsmittel Nr. 1 | Kreide | 8,8 | 38 | 22 | 37 | 23 | 37 | 21 | 30 | 23 |
| 98 | 7% Leimungsmittel gem. Beisp. 59<br>3,5% Retentionsmittel Nr. 1 | Kreide | 8,7 | 49 | 26 | 44 | 14 | 29 | 14 | 31 | 17 |
| 99 | 7% Leimungsmittel gem. Beisp. 60<br>3,5% Retentionsmittel Nr. 1 | Kreide | 8,5 | 20 | 13 | 23 | 13 | 15 | 13 | 17 | 16 |
| 100 | 7% Leimungsmittel gem. Beisp. 61<br>3,5% Retentionsmittel Nr. 1 | Kreide | 8,7 | 16 | 12 | 15 | 12 | 17 | 13 | 19 | 16 |
| 101 | 7% Leimungsmittel gem. Beisp. 62<br>3,5% Retentionsmittel Nr. 1 | Kreide | 8,8 | 20 | 13 | 22 | 11 | 20 | 16 | 21 | 18 |
| 102 | 7% Leimungsmittel gem. Beisp. 63<br>3,5% Retentionsmittel Nr. 1 | Kreide | 8,9 | 27 | 14 | 27 | 13 | 34 | 16 | 37 | 18 |
| 103 | 7% Leimungsmittel gem. Beisp. 64<br>3,5% Retentionsmittel Nr. 1 | Kreide | 8,3 | 37 | 16 | 38 | 17 | 29 | 18 | 32 | 18 |
| 104 | 7% Leimungsmittel gem. Beisp. 65<br>3,5% Retentionsmittel Nr. 1 | Kreide | 8,3 | 38 | 16 | 16 | 12 | 28 | 14 | 16 | 11 |
| 105 | 7% Leimungsmittel gem. Beisp. 66<br>3,5% Retentionsmittel Nr. 1 | Kreide | 8,4 | 25 | 13 | 25 | 13 | 25 | 15 | 23 | 14 |
| 106 | 7% Leimungsmittel gem. Beisp. 67<br>3,5% Retentionsmittel Nr. 1 | Kreide | 9,1 | 39 | 23 | 23 | 13 | 38 | 19 | 24 | 15 |
| 107 | 7% Leimungsmittel gem. Beisp. 50<br>3,5% Retentionsmittel Nr. 1 | – *) | 5,6 | 27 | 16 | 25 | 14 | 24 | 15 | 21 | 12 |
| 108 | 24% Leimungsmittel gem. Beisp. 54<br>4% Retentionsmittel Nr. 1 | Kreide | 8,2 | 56 | 26 | 36 | 14 | 22 | 16 | 21 | 13 |
| 109 | 7% Leimungsmittel gem. Beisp. 49<br>3,5% Retentionsmittel | Kreide | 8,7 | 23 | 14 | 23 | 13 | – | – | – | – |

*) kein Füllmittel

Beispiele 110 bis 113:

Man verfährt wie in Beispielen 88 bis 109 angegeben, setzt jedoch eine Formulierung aus 7% Leimungsmittel gemäss Beispiel 22 und 3,5% Retentionsmittel Nr. 1 ein. Zudem wird neben der holzschlifffreien Faserstoffsuspension auch eine Faserstoffsuspension eingesetzt, die gebleichten Birkensulfatzellstoff: gebleichten Kiefernsulfatzellstoff: gebleichter Holzschliff im Gewichtsverhältnis 1:1:2 enthält. Die Leimungsergebnisse sind in der nachfolgenden Tabelle II zusammengestellt.

Tabelle II

| Beispiel Nr. | Holzschliff* | Füllmittel | pH-Wert Suspension | WA $Cobb_{30}$ (g/m²) nach Trocknung sofort SS | OS | nach 1 Tag Lagerung SS | OS | nach 1 Woche Lagerung SS |
|---|---|---|---|---|---|---|---|---|
| 110 | – | Kreide | 7,5 | 15 | 13 | 15 | 12 | 16 |
| 111 | – | Kaolin | 6 | 17 | 13 | 18 | 13 | 31 |
| 112 | – | ** | 5,6 | 17 | 17 | 18 | 13 | 20 |
| 113 | + | ** | 6,6 | 16 | 14 | 12 | 10 | 10 |

* – holzschlifffreie Suspensionen
 + holzschliffhaltige Suspensionen
** kein Füllmittel

Beispiele 114 bis 117:

Man verfährt wie in Beispielen 88 bis 109 angegeben, setzt jedoch nur 0,3 statt 0,5% an Trockensubstanz des Leimungsmittels, bezogen auf den Feststoffgehalt der Faserstoffsuspension ein. Die Leimungsergebnisse sind in der nachfolgenden Tabelle III zusammengestellt.

Tabelle III

| Beispiel Nr. | Formulierung | Füllmittel | pH-Wert Suspension | WA $Cobb_{30}$ (g/m²) nach Trocknung sofort SS | OS | nach 1 Tag Lagerung SS | OS |
|---|---|---|---|---|---|---|---|
| 114 | 7% Leimungsmittel gemäss Beispiel 50<br>3,5% Retentionsmittel Nr. 1 | Kreide | 8,2 | 34 | 19 | 35 | 17 |
| 115 | 7% Leimungsmittel gemäss Beispiel 54<br>3,5% Retentionsmittel Nr. 1 | Kreide | 8,2 | 41 | 15 | 32 | 13 |
| 116 | 7% Leimungsmittel gemäss Beispiel 55<br>3,5% Retentionsmittel Nr. 1 | Kreide | 8,2 | 40 | 23 | 30 | 18 |
| 117 | 7% Leimungsmittel gemäss Beispiel 22<br>3,5% Retentionsmittel Nr. 1 | Kreide | 8,0 | 23 | 14 | 20 | 13 |

Beispiele 118 und 119:

Man verfährt wie in Beispielen 88 bis 109 angegeben, setzt jedoch die nachfolgenden Formulierungen des Leimungsmittels, das in Gegenwart von Wasser jeweils in geschmolzenem Zustand bei 80 °C emulgiert wird:

In Beispiel 118 angesetzte Formulierung:
7 % Leimungsmittel gemäss Beispiel 50
3,5% Retentionsmittel Nr. 1
0,7% Sorbitan-Monostearat (Öl-in-Wasser Emulgator)
0,7% eines Adduktes aus Äthylenoxyd und Sorbitan-monostearat (Wasser-in-Öl Emulgator).

In Beispiel 119 eingesetzte Formulierung:

30% Leimungsmittel gemäss Beispiel 57
15% Retentionsmittel Nr. 1

Beide Formulierungen sind homogen und lagerstabil.

Die Leimungsergebnisse sind in der nachfolgenden Tabelle IV zusammengestellt.

Tabelle IV

| Beispiel Nr. | Füllmittel | pH-Wert Suspension | WA $Cobb_{30}$ (g/m²) nach Trocknung sofort SS | OS | nach 1 Tag Lagerung SS | OS | nach Wärmebehandlung sofort SS | OS | nach 1 Tag Lagerung SS | OS |
|---|---|---|---|---|---|---|---|---|---|---|
| 118 | Kreide | 8,1 | 19 | 10 | 20 | 13 | 17 | 11 | 15 | 14 |
| 119 | Kreide | 8,4 | 58 | 17 | 54 | 19 | 31 | 17 | 29 | 18 |

Beispiele 120 bis 127:

Man verfährt wie in Beispielen 88 bis 109 angegeben, gibt jedoch das Leimungsmittel und das Retentionsmittel separat zur Faserstoffsuspension, wobei das Leimungsmittel in Pulverform mit einer wässrigen, 5%-igen Natrium- oder Kaliumhydroxydlösung in Gegenwart von Wasser und Glasperlen verrührt wird und selbstemulgierende, ebenfalls homogene und lagerstabile, in der nachfolgenden Tabelle V angegebene Formulierungen des Leimungsmittels entstehen. Die angegebenen Val% bedeuten die Anzahl Äquivalente an Natrium- oder Kaliumhydroxyd für 100 Äquivalente, bezogen auf die Anzahl negative Ladungen von sauren Gruppen der als Leimungsmittel jeweils eingesetzten sauren Ester. 10 Sekunden vor oder nach der Zugabe des Leimungsmittels wird die Fasersuspension mit jeweils 0,25% an Trockensubstanz des Retentionsmittels Nr. 1, bezogen auf den Feststoffgehalt der Faserstoffsuspension, versetzt. Die Leimungsergebnisse sind ebenfalls aus der Tabelle V zu entnehmen.

Tabelle V

| Beispiel Nr. | Reihenfolge der Zugabe des Retentionsmittels | Leimungsmittel Formulierung | Füllmittel | pH-Wert der Suspension | WA $Cobb_{30}$ nach Trocknung sofort | | nach 1 Tag Lagerung | |
|---|---|---|---|---|---|---|---|---|
| | | | | | SS | OS | SS | OS |
| 120 | vor Leimungsmittel | 7% Leimungsmittel gem. Beisp. 50<br>10 Val% Natriumhydroxyd | Kreide | 8,1 | 16 | 15 | 18 | 14 |
| 121 | nach Leimungsmittel | 7% Leimungsmittel gem. Beisp. 50<br>10 Val% Natriumhydroxyd | Kreide | 8,1 | 17 | 15 | 19 | 15 |
| 122 | vor Leimungsmittel | 7% Leimungsmittel gem. Beisp. 50<br>90 Val% Natriumhydroxyd | Kreide | 8,2 | 25 | 15 | 46 | 18 |
| 123 | nach Leimungsmittel | 7% Leimungsmittel gem. Beisp. 50<br>90 Val% Natriumhydroxyd | Kreide | 8,2 | 21 | 18 | 36 | 27 |
| 124 | vor Leimungsmittel | 7% Leimungsmittel gem. Beisp. 22<br>90 Val% Natriumhydroxyd | Kreide | 8,2 | 17 | 16 | 14 | 12 |
| 125 | nach Leimungsmittel | 15% Leimungsmittel gem. Beisp. 22<br>70 Val% Kaliumhydroxyd | Kreide | 8,3 | 30 | 17 | 17 | 12 |
| 126 | nach Leimungsmittel | 15% Leimungsmittel gem. Beisp. 22<br>70 Val% Kaliumhydroxyd | –* | 6,0 | 18 | 14 | 16 | 12 |
| 127 | nach Leimungsmittel | 15% Leimungsmittel gem. Beisp. 22<br>70 Val% Kaliumhydroxyd | – (Holzschliff)** | 7,0 | 19 | 14 | 11 | 18 |

* Kein Füllmittel

** Beim Beispiel 126 wird eine Faserstoffsuspension eingesetzt, die gebleichten Birkensulfatstoff: gebleichten Kiefersulfatzellstoff: gebleichter Holzschliff im Gewichtsverhältnis 1:1:2, jedoch kein Füllmittel enthält.

Beispiele 128 bis 145:

Man verfährt wie in Beispielen 88 bis 109 angegeben, gibt jedoch das Leimungsmittel und das Retentionsmittel separat zur Faserstoffsuspension, wobei sofern ein Füllmittel eingesetzt wird, das Leimungsmittel 10 Sekunden nach der Füllmittelzugabe zugesetzt wird. Die Retentionsmittelzugabe erfolgt 10 Sekunden nach der Leimungsmittelzugabe und die Zugabe an PERCOL 292® 10 Sekunden nach der Retentionsmittelzugabe. Die eingesetzte Faserstoffsuspension enthält 0,3% Alaun (als trockenes $Al_2(SO_4)_3$ berechnet) bezogen auf den Feststoffgehalt der Suspension und 1,5 g Natriumsulfat pro Liter Suspension. Zudem wird neben den holzschlifffreien Suspensionen auch eine Faserstoffsuspension eingesetzt, die gebleichten Birkensulfatzellstoff: gebleichten Kiefernsulfatzellstoff: gebleichter Holzschliff im Gewichtsverhältnis 1:1:2 enthält. Einem Teil dieser Faserstoffsuspensionen wird die in den nachfolgenden Tabellen VI und VII angegebenen Mengen Schwefelsäure oder Natriumhydroxyd (als Reinsubstanz bezogen auf Suspensionsfeststoffgehalt) versetzt, wobei die Schwefelsäure und das Natriumhydroxyd in Form verdünnter, wässriger Lösungen eingesetzt werden. Als Leimungsmittel wird eine 15,3%-ige weiterverdünnbare, homogene und stabile Formulierung des gemäss Beispiel 56 erhaltenen Estergemisches in Pulverform, das mit 65 Val% Ammoniak in Gegenwart von Wasser und Glasperlen dispergiert wird. Bezogen auf den Feststoffgehalt der Faserstoffsuspension werden 0,5% des angegebenen Leimungsmittels (berechnet als Trockensubstanz) und 0,25% Retentionsmittel Nr. 1 (ebenfalls als Trockensubstanz berechnet) eingesetzt. Die Leimungsergebnisse sind aus den Tabellen VI und VII zu entnehmen.

Tabelle VI
Holzschlifffreie Fasersuspensionen

| Beispiel Nr. | Füllmittel | Schwefelsäure Zusatz (%) | Natriumhydroxyd Zusatz (%) | pH-Wert Suspension | WA $Cobb_{30}$ ($g/m^2$) nach Trocknung sofort | | nach 1 Tag Lagerung | | nach 2 Wochen Lagerung |
|---|---|---|---|---|---|---|---|---|---|
| | | | | | SS | OS | SS | OS | SS |
| 128 | – | 0,74 | – | 3,4 | 39 | 15 | 35 | 14 | 27 |
| 129 | – | – | – | 6,0 | 17 | 15 | 16 | 13 | 17 |
| 130 | – | – | 1 | 9,7 | 18 | 15 | 17 | 13 | 18 |
| 131 | – | – | 4 | 10,7 | 27 | 20 | 27 | 13 | 35 |
| 132 | Kreide | – | – | 8,3 | 15 | 14 | 13 | 11 | 14 |
| 133 | Kaolin | – | – | 6,5 | 18 | 15 | 18 | 11 | 29 |
| 134 | Talk | – | – | 7,5 | 14 | 14 | 14 | 12 | 13 |
| 135 | Kaolin | – | 1,02 | 9,5 | 21 | 15 | 18 | 12 | 43 |
| 136 | Talk | – | 1,02 | 9,9 | 16 | 12 | 17 | 10 | 22 |

Tabelle VII
Holzschliffhaltige Faserstoffsuspensionen

| Beispiel Nr. | Füllmittel | Schwefelsäure Zusatz (%) | Natriumhydroxyd Zusatz (%) | pH-Wert Suspension | WA $Cobb_{30}$ ($g/m^2$) nach Trocknung sofort | | nach 1 Tag Lagerung | | nach 2 Wochen Lagerung |
|---|---|---|---|---|---|---|---|---|---|
| | | | | | SS | OS | SS | OS | SS |
| 137 | – | 0,74 | – | 3,2 | 32 | 13 | 15 | 11 | 13 |
| 138 | – | – | – | 5,9 | 23 | 12 | 13 | 11 | 14 |
| 139 | – | – | 1 | 7,6 | 19 | 12 | 12 | 10 | 12 |
| 140 | – | – | 4 | 10,1 | 29 | 14 | 17 | 12 | 18 |
| 141 | Kreide | – | – | 8,6 | 18 | 11 | 12 | 10 | 14 |
| 142 | Kaolin | – | – | 6,5 | 34 | 12 | 15 | 9 | 20 |
| 143 | Talk | – | – | 6,6 | 23 | 11 | 13 | 9 | 14 |
| 144 | Kaolin | – | 1,02 | 7,8 | 20 | 12 | 13 | 10 | 15 |
| 145 | Talk | – | 1,02 | 8,1 | 16 | 11 | 12 | 10 | 13 |

Beispiele 146 bis 150:

Man verfährt wie in Beispielen 88 bis 109 angegeben, gibt jedoch das Leimungsmittel und das Retentionsmittel separat zur Faserstoffsuspension und setzt eine Faserstoffsuspension ein, die Kiefernsulfatzellstoff: gebleichtem Holzschliff im Gewichtsverhältnis 1:4 enthält. 30% Kreide werden als Füllmittel zur Faserstoffsuspension gegeben. Die Leimungsmittelzugabe erfolgt 10 Sekunden nach der Kreidezugabe, die Retentionsmittelzugabe 10 Sekunden nach der Leimungsmittelzugabe und die Zugabe an PERCOL 292® 10 Sekunden nach der Retentionsmittelzugabe. Als Leimungsmittel wird eine 35%-ige, weiterverdünnbare, homogene und stabile Formulierung des gemäss Beispiel 56 erhaltenen Estergemisches in geschmolzener Form, das mit 18 Val% Natriumhydroxyd in Gegenwart von Wasser bei 80 °C emulgiert wird. Anstelle einer der Retentionsmittel Nr. 1 bis 3 wird das Retentionsmittel Nr. 4 oder Nr. 5 eingesetzt. Retentionsmittel Nr. 4 ist ein Umsetzungsprodukt aus Dicyandiamid, Formaldehyd, Ammoniumchlorid und Äthylendiamin, das gemäss Beispiel 1 der US-A-3 491 064 hergestellt wird. Retentionsmittel Nr. 5 ist RETAMINOL K® (Polyäthylenimin eines Molekulargewichtes von 20 000 bis 40 000). Die in der nachfolgenden Tabelle VIII angegebenen Mengen an Leimungs- und Retentionsmitteln werden eingesetzt, wobei diese Mengen und die Menge Füllmittel (30% Kreide) sich auf Trockensubstanz an Leimungs-, Retentions- und Füllmittel und auf den Feststoffgehalt der Faserstoffsuspension beziehen. Die Leimungsergebnisse sind ebenfalls aus der nachfolgenden Tabelle VIII zu entnehmen.

Tabelle VIII zu entnehmen.

| Beispiel Nr. | Einsatzmenge Leimungsmittel (%) | Retentionsmittel | Einsatzmenge Retentionsmittel (%) | pH-Wert Suspension | WA $Cobb_{30}$ ($g/m^2$) nach Trocknung sofort | | nach 1 Tag Lagerung | |
|---|---|---|---|---|---|---|---|---|
| | | | | | SS | OS | SS | OS |
| 146 | 0,5 | Nr. 4 | 1 | 8,4 | 63 | 15 | 55 | 19 |
| 147 | 1 | Nr. 5 | 0,02 | 7,9 | 40 | 11 | 38 | 9 |
| 148 | 1 | Nr. 5 | 0,25 | 8,0 | 14 | 10 | 11 | 8 |
| 149 | 0,2 | Nr. 5 | 0,5 | 8,2 | 46 | 20 | 38 | 13 |
| 150 | 1 | Nr. 5 | 0,5 | 8,2 | 13 | 10 | 11 | 7 |

Beispiele 151 bis 170:

Man verfährt wie in Beispielen 88 bis 109 angegeben unter Zugabe von 20% Kreide als Füllmittel, setzt jedoch Formulierungen aus 7% der in der nachfolgenden Tabelle IX angegebenen Leimungsmittel und 3,5% Retentionsmittel Nr. 1 ein, wobei ebenfalls giessbare, homogene und lagerstabile Dispersionen erhalten werden, die zur Faserstoffsuspension so gegeben werden, dass eine Einsatzmenge von 0,5 oder 1,0% an Trockensubstanz des Leimungsmittels, bezogen auf den Feststoffgehalt der Faserstoffsuspensionen, entsteht. Die Leimungsergebnisse sind aus der Tabelle IX zu entnehmen.

Tabelle IX

| Beispiel Nr. | Leimungsmittel | Einsatzmenge (%) | pH-Wert der Suspension | WA Cobb ($g/m^2$) nach Trocknung | | nach 1 Tag Lagerung | |
|---|---|---|---|---|---|---|---|
| | | | | SS | OS | SS | OS |
| 151 | Umsetzungsprodukt gemäss Beispiel 23 | 0,5 | 9,0 | 74 | 56 | 78 | 64 |
| 152 | Umsetzungsprodukt gemäss Beispiel 23 | 1,0 | 9,2 | 68 | 39 | 77 | 41 |
| 153 | Umsetzungsprodukt gemäss Beispiel 26 | 0,5 | 8,7 | 19 | 12 | 20 | 13 |
| 154 | Umsetzungsprodukt gemäss Beispiel 27 | 0,5 | 9,1 | 42 | 22 | 30 | 16 |
| 155 | Umsetzungsprodukt gemäss Beispiel 27 | 1,0 | 9,2 | 25 | 11 | 23 | 14 |
| 156 | Umsetzungsprodukt gemäss Beispiel 28 | 0,5 | 9,2 | 27 | 16 | 26 | 15 |
| 157 | Umsetzungsprodukt gemäss Beispiel 30 | 0,5 | 9,1 | 19 | 15 | 17 | 13 |
| 158 | Umsetzungsprodukt gemäss Beispiel 31 | 0,5 | 9,2 | 59 | 24 | 42 | 15 |
| 159 | Umsetzungsprodukt gemäss Beispiel 31 | 1,0 | 9,2 | 47 | 15 | 23 | 12 |
| 160 | Umsetzungsprodukt gemäss Beispiel 22 | 1,0 | 9,2 | 59 | 58 | 70 | 68 |
| 161 | Umsetzungsprodukt gemäss Beispiel 69 | 0,5 | 9,2 | 72 | 46 | 81 | 64 |
| 162 | Umsetzungsprodukt gemäss Beispiel 69 | 1,0 | 9,2 | 24 | 13 | 20 | 14 |
| 163 | Umsetzungsprodukt gemäss Beispiel 70 | 0,5 | 9,2 | 23 | 14 | 20 | 12 |
| 164 | Umsetzungsprodukt gemäss Beispiel 72 | 0,5 | 9,4 | 90 | 77 | 16 | 12 |
| 165 | Umsetzungsprodukt gemäss Beispiel 72 | 1,0 | 9,2 | 75 | 66 | 15 | 12 |
| 166 | Umsetzungsprodukt gemäss Beispiel 73 | 0,5 | 8,9 | 65 | 19 | 61 | 14 |
| 167 | Umsetzungsprodukt gemäss Beispiel 74 | 0,5 | 8,9 | 19 | 15 | 17 | 12 |
| 168 | Umsetzungsprodukt gemäss Beispiel 75 | 0,5 | 8,9 | 34 | 16 | 37 | 14 |
| 169 | Umsetzungsprodukt gemäss Beispiel 76 | 0,5 | 8,9 | 28 | 17 | 24 | 15 |
| 170 | Umsetzungsprodukt gemäss Beispiel 77 | 0,5 | 8,8 | 20 | 13 | 16 | 11 |

Ähnliche Ergebnisse werden erzielt, wenn man das Amidgemisch gemäss Beispiel 24, 25, 29, 68 oder 71 einsetzt.

Beispiele 171 bis 175:

Man verfährt wie in Beispielen 88 bis 109 angegeben unter Zugabe von 20% Kreide als Füllmittel, setzt jedoch Formulierungen aus 7% der in der nachfolgenden Tabelle X angegebenen Leimungsmittel und 3,5% Retentionsmittel Nr. 1 oder Nr. 2 ein, wobei ebenfalls giessbare, homogene und lagerstabile Dispersionen erhalten werden, die zur Faserstoffsuspension so gegeben werden, dass eine Einsatzmenge von 0,4 oder 0,5% an Trockensubstanz des Leimungsmittels, bezogen auf den Feststoffgehalt der Faserstoffsuspension, entsteht. Die Leimungsergebnisse sind aus der Tabelle X zu entnehmen.

Tabelle X

| Beispiel Nr. | Leimungsmittel | Retentionsmittel | Einsatzmenge Leimungsmittel % | pH-Wert der Suspension | WA $Cobb_{30}$ (g/m²) vor Wärmebehandlung sofort SS | OS | nach 1 Tag Lagerung SS | OS | nach Wärmebehandlung sofort SS | OS | nach 1 Tag Lagerung SS | OS |
|---|---|---|---|---|---|---|---|---|---|---|---|---|
| 171 | Umsetzungsprodukt gemäss Beispiel 33 | Nr. 2 | 0,5 | 7,4 | 31 | 20 | 37 | 17 | 15 | 12 | 15 | 9 |
| 172 | Umsetzungsprodukt gemäss Beispiel 34 | Nr. 1 | 0,5 | 8,4 | 21 | 13 | 19 | 12 | 18 | 12 | 19 | 9 |
| 173 | Umsetzungsprodukt gemäss Beispiel 78 | Nr. 1 | 0,5 | 8,0 | 49 | 17 | 40 | 17 | 21 | 14 | 18 | 12 |
| 174 | Umsetzungsprodukt gemäss Beispiel 79 | Nr. 2 | 0,4 | 7,9 | 36 | 22 | 14 | 14 | 16 | 15 | 15 | 12 |
| 175 | Umsetzungsproduktgemisch gemäss Beispiel 80 | Nr. 2 | 0,4 | 7,9 | 62 | 24 | 17 | 14 | 18 | 15 | 16 | 12 |

Beispiele 176 bis 201:

Man verfährt wie in Beispielen 88 bis 109 angegeben unter Zugabe von 20% Kreide als Füllmittel, setzt jedoch Formulierungen aus 7% der in der nachfolgenden Tabelle XI angegebenen Leimungsmittel und 3,5% Retentionsmittel ein, wobei ebenfalls giessbare, homogene und lagerstabile Dispersionen erhalten werden, die zur Faserstoffsuspension so gegeben werden, dass eine Einsatzmenge von 0,25, 0,35, 0,5 oder 1,0% an Trockensubstanz des Leimungsmittels, bezogen auf den Feststoffgehalt der Faserstoffsuspension, entsteht. Die Leimungsergebnisse sind aus der Tabelle XI zu entnehmen.

Beispiele 202 bis 206:

Man verfährt wie in Beispielen 88 bis 109 angegeben, gibt jedoch das Leimungsmittel und das Retentionsmittel separat zur Faserstoffsuspension, wobei 7% Leimungsmittel in flüssigem oder geschmolzenem Zustand bei 80 °C mit einer wässrigen, 5%-igen Ammoniaklösung in Gegenwart von Wasser zu einer selbstemulgierenden, ebenfalls giessbaren, homogenen und lagerstabilen Emulsion verrührt wird und in der nachfolgenden Tabelle XII angegebenen Formulierungen des Leimungsmittels entstehen. Die angegebenen Val% bedeuten die Anzahl Äquivalente an Ammoniak für 100 Äquivalente, bezogen auf die Anzahl vorhandener –COOH Gruppen der als Leimungsmittel jeweils eingesetzten Ester- oder Amidgemische. 10 Sekunden nach der Zugabe von 0,5% oder 1% an Trockensubstanz des Leimungsmittels wird die Faserstoffsuspension mit jeweils der halben Einsatzmenge, d.h. 0,25% oder 0,5% an Trockensubstanz des Retentionsmittels Nr. 1 versetzt, wobei sich die Einsatzmengen an Leimungs- und Retentionsmittel auf den Feststoffgehalt der Faserstoffsuspension beziehen. Die Leimungsergebnisse sind ebenfalls aus der Tabelle XII zu entnehmen.

Beispiele 207 bis 214:

Man verfährt wie in Beispielen 88 bis 109 angegeben, setzt jedoch die in der nachfolgenden Tabelle XIII angegebenen Füllmittel ein und gibt das Leimungsmittel und das Retentionsmittel separat zur Faserstoffsuspension, wobei 15% Leimungsmittel in Pulverform mit einer wässrigen, 5%-igen Ammoniaklösung in Gegenwart von Wasser und Glasperlen verrührt wird und selbstemulgierende, ebenfalls homogene und lagerstabile, in der nachfolgenden Tabelle XIII angegebenen Formulierungen des Leimungsmittels entstehen. Die angegebenen Val% bedeuten die Anzahl Äquivalente an Ammoniak für 100 Äquivalente, bezogen auf die Anzahl vorhandener –COOH Gruppen der als Leimungsmittel jeweils eingesetzten Ester- oder Amidgemische. 10 Sekunden nach der Zugabe von 0,4% an Trockensubstanz des Leimungsmittels wird die Faserstoffsuspension mit jeweils 0,2% an Trockensubstanz des Retentionsmittels Nr. 1 versetzt. Die Einsatzmenge an Füll-, Leimungs- und Retentionsmittel beziehen sich auf

Tabelle XI

| Beispiel Nr. | Leimungsmittel | Einsatzmenge (%) | pH-Wert der Suspension | WA $Cobb_{30}$ ($g/m^2$) nach Trocknung SS | nach Trocknung OS | nach 1 Tag Lagerung SS | nach 1 Tag Lagerung OS | nach 2 Wochen Lagerung SS |
|---|---|---|---|---|---|---|---|---|
| 176 | Estergemisch gemäss Beispiel 35 | 0,5 | 8,4 | 21 | 12 | 18 | 11 | 23 |
| 177 | Estergemisch gemäss Beispiel 36 | 0,5 | 8,1 | 22 | 12 | 19 | 12 | 22 |
| 178* | Estergemisch gemäss Beispiel 37 | 0,5 | 8,1 | 26 | 12 | 22 | 12 | 32 |
| 179 | Estergemisch gemäss Beispiel 38 | 0,5 | 8,9 | 16 | 12 | 15 | 11 | 22 |
| 180 | Amidgemisch gemäss Beispiel 39 | 0,5 | 8,3 | 16 | 11 | 15 | 10 | 14 |
| 181 | Amidgemisch gemäss Beispiel 40 | 0,5 | 8,7 | 19 | 11 | 15 | 11 | 15 |
| 182 | Amidgemisch gemäss Beispiel 41 | 0,5 | 8,1 | 75 | 41 | 78 | 45 | 89 |
| 183 | Amidgemisch gemäss Beispiel 41 | 1,0 | 8,5 | 52 | 14 | 44 | 14 | 49 |
| 184 | Amidgemisch gemäss Beispiel 43 | 0,5 | 8,8 | 87 | 27 | 74 | 32 | 75 |
| 185 | Amidgemisch gemäss Beispiel 43 | 1,0 | 8,5 | 34 | 11 | 40 | 13 | 22 |
| 186 | Amidgemisch gemäss Beispiel 44 | 0,5 | 8,5 | 21 | 12 | 19 | 10 | 17 |
| 187 | Estergemisch gemäss Beispiel 45 | 0,5 | 8,5 | 15 | 12 | 14 | 12 | 16 |
| 188 | Estergemisch gemäss Beispiel 45 | 0,35 | 8,3 | 22 | 13 | 21 | 12 | 22 |
| 189 | Estergemisch gemäss Beispiel 45 | 0,25 | 8,4 | 35 | 20 | 31 | 19 | 37 |
| 190 | Estergemisch gemäss Beispiel 46 | 0,5 | 8,5 | 20 | 12 | 16 | 11 | 17 |
| 191 | Estergemisch gemäss Beispiel 46 | 0,35 | 8,5 | 22 | 13 | 21 | 12 | 22 |
| 192 | Estergemisch gemäss Beispiel 46 | 0,25 | 8,6 | 28 | 15 | 28 | 14 | 31 |
| 193 | Amidgemisch gemäss Beispiel 47 | 0,5 | 8,8 | 31 | 15 | 21 | 11 | 21 |
| 194 | Estergemisch gemäss Beispiel 81 | 0,5 | 8,4 | 21 | 12 | 18 | 12 | 35 |
| 195 | Amidgemisch gemäss Beispiel 82 | 0,5 | 8,8 | 31 | 13 | 19 | 11 | 26 |
| 196 | Amidestergemisch gemäss Beispiel 83 | 0,5 | 8,8 | 30 | 15 | 18 | 11 | 22 |
| 197** | Estergemisch gemäss Beispiel 84 | 1,0 | 8,9 | 20 | 15 | 15 | 12 | 18 |
| 198 | Amidgemisch gemäss Beispiel 85 | 0,5 | 8,8 | 49 | 21 | 23 | 12 | 22 |
| 199** | Amidgemisch gemäss Beispiel 85 | 0,5 | 8,9 | 37 | 20 | 27 | 13 | 21 |
| 200 | Estergemisch gemäss Beispiel 86 | 0,5 | 8,3 | 44 | 13 | 42 | 12 | 51 |
| 201 | Amidgemisch gemäss Beispiel 87 | 1,0 | 8,7 | 36 | 15 | 26 | 12 | 19 |

* In Beispiel 178 wird die Formulierung des Leimungsmittels in geschmolzenem Zustand bei 80°C durchgeführt.

** In den Beispielen 197 und 199 wird die Formulierung des Leimungsmittels in Gegenwart von 0,7% eines Gemisches im Gewichtsverhältnis 1:1 aus Sorbitan-Monostearat und aus dessen Addukt mit Äthylenoxyd hergestellt.

Tabelle XII

| Beispiel Nr. | Leimungsmittel Formulierung | Einsatzmenge Leimungsmittel (%) | pH-Wert der Suspension | WA $Cobb_{30}$ ($g/m^2$) nach Trocknung SS | nach Trocknung OS | nach 1 Tag Lagerung SS | nach 1 Tag Lagerung OS | nach 2 Wochen Lagerung SS |
|---|---|---|---|---|---|---|---|---|
| 202 | 7%iges flüssiges Estergemisch gemäss Beispiel 39 100 Val% Ammoniak | 0,5 | 8,3 | 89 | 78 | 58 | 43 | 84 |
| 203 | 7% flüssiges Estergemisch gemäss Beispiel 39 100 Val% Ammoniak | 1,0 | 8,3 | 29 | 26 | 20 | 18 | 102 |
| 204 | 7% geschmolzenes Amidgemisch gemäss Beispiel 42 100 Val% Ammoniak | 0,5 | 8,2 | 18 | 13 | 17 | 13 | 25 |
| 205 | 7% geschmolzenes Amidgemisch gemäss Beispiel 42 200 Val% Ammoniak | 0,5 | 8,2 | 19 | 13 | 19 | 14 | 39 |
| 206 | 7% geschmolzenes Estergemisch gemäss Beispiel 48 100 Val% Ammoniak | 0,5 | 8,2 | 30 | 16 | 29 | 16 | 80 |

den Feststoffgehalt der Faserstoffsuspension. Dies gilt auch für die Alaunmenge. Die Leimungsergebnisse sind ebenfalls aus der Tabelle XIII zu entnehmen.

Tabelle XIII

| Beispiel Nr. | Leimungsmittel Formulierung | Füllmittel | pH-Wert der Suspension | WA Cobb$_{30}$ (g/m$^2$) nach Trocknung SS | OS | nach 1 Tag Lagerung SS | OS | nach 2 Wochen Lagerung SS | OS |
|---|---|---|---|---|---|---|---|---|---|
| 207 | 15% Estergemisch gemäss Beispiel 38 100 Val% Ammoniak | 20% Kreide (in Gegenwart von 1,5 g $Na_2SO_4$/l Flotte) | 8,5 | 28 | 12 | 28 | 12 | 47 | 16 |
| 208 | 15% Amidgemisch gemäss Beispiel 40 50 Val% Ammoniak | 20% Kreide (in Gegenwart von 1,5 g $Na_2SO_4$/l Flotte) | 8,5 | 26 | 13 | 22 | 11 | 41 | 13 |
| 209 | 15% Estergemisch gemäss Beispiel 38 100 Val% Ammoniak | 20% Kreide (in Gegenwart von 1,5% $Al_2(SO_4)_3$) | 8,0 | 22 | 14 | 20 | 12 | 44 | 17 |
| 210 | 15% Amidgemisch gemäss Beispiel 40 50 Val% Ammoniak | 20% Kreide (in Gegenwart von 1,5% $Al_2(SO)_4)_3$) | 8,0 | 17 | 14 | 16 | 13 | 23 | 15 |
| 211 | 15% Estergemisch gemäss Beispiel 38 100 Val% Ammoniak | 20% Kaolin | 5,5 | 44 | 18 | 39 | 18 | 100 | 94 |
| 212 | 15% Amidgemisch gemäss Beispiel 40 50 Val% Ammoniak | 20% Kaolin | 5,5 | 30 | 20 | 25 | 24 | 109 | 96 |
| 213* | 15% Estergemisch gemäss Beispiel 38 100 Val% Ammoniak | –* | 6,5 | 29 | 20 | 15 | 11 | 19 | 14 |
| 214* | 15% Amidgemisch gemäss Beispiel 40 50 Val% Ammoniak | –* | 6,5 | 69 | 44 | 17 | 12 | 53 | 23 |

* In den Beispielen 213 und 214 wird eine Faserstoffsuspension eingesetzt, die gebleichten Birkensulfatzellstoff: gebleichten Kiefersulfatzellstoff: Holzschliff im Gewichtsverhältnis 1:1:2, jedoch kein Füllmittel enthält.

Ähnliche Ergebnisse werden auch erzielt, wenn man das Retentionsmittel zuerst zur Faserstoffsuspension gibt und das Leimungsmittel 10 Sekunden nach der Retentionsmittelzugabe gibt.

Beispiele 215 bis 218:

Man verfährt wie in Beispielen 88 bis 109 angegeben, gibt jedoch das Leimungsmittel und das Retentionsmittel separat zur Faserstoffsuspension und setzt kein PERCOL 292® als Hilfsmittel ein. Als Füllmittel werden 20% Kreide zur Faserstoffsuspension gegeben. Die Leimungsmittelzugabe erfolgt 10 Sekunden nach der Kreidezugabe und die Retentionsmittelzugabe 10 Sekunden nach der Leimungsmittelzugabe. Als Leimungsmittel werden die in der nachfolgenden Tabelle XIV angegebenen Emulsionen oder Dispersionen eingesetzt. Anstelle einer der Retentionsmittel Nr. 1 bis 3 wird das Retentionsmittel Nr. 6 oder 7 eingesetzt. Retentionsmittel Nr. 6 ist ein Epichlorhydrin-Addukt einem Umsetzungsprodukt aus Dicyandiamid, Diäthylentriamin und Dimethyladipat, das gemäss Beispiel 7 der GB-A-1 125 486 hergestellt wird. Retentionsmittel Nr. 7 ist ein Copolymerisat aus Adipinsäure und Dimethylamino-hydroxypropyldiäthylentriamin eines Molekulargewichts von 1000 bis 10 000. Die in der nachfolgenden Tabelle XIV angegebenen Mengen an Leimungs- und Retentionsmitteln werden eingesetzt, wobei diese Mengen und die Menge Füllmittel (20% Kreide) sich auf Trockensubstanz an Leimungs-, Retentions- und Füllmittel und auf den Feststoffgehalt der Faserstoffsuspension beziehen. Die Leimungsergebnisse sind ebenfalls aus der nachfolgenden Tabelle XIV zu entnehmen.

Tabelle XIV

| Beispiel Nr. | Leimungsmittel | Einsatzmenge Leimungsmittel (%) | Retentionsmittel | Einsatzmenge Retentionsmittel (%) | pH-Wert Suspension | WA $Cobb_{30}$ ($g/m^2$) nach Trocknung sofort | | nach 1 Tag Lagerung | |
|---|---|---|---|---|---|---|---|---|---|
| | | | | | | SS | OS | SS | OS |
| 215 | gemäss Beispiel 35* | 0,5 | Nr. 6 | 0,25 | 8,4 | 34 | 13 | 18 | 10 |
| 216 | gemäss Beispiel 45** | 0,5 | Nr. 6 | 1,0 | 8,5 | 39 | 15 | 30 | 11 |
| 217 | gemäss Vorschrift Beispiel 56*** | 0,5 | Nr. 6 | 1,0 | 8,5 | 17 | 12 | 14 | 11 |
| 218 | gemäss Beispiel 56*** | 0,5 | Nr. 7 | 1,0 | 8,8 | 20 | 15 | 16 | 12 |

* als weiterverdünnbare, homogene, stabile Formulierung des Leimungsmittels, das in geschmolzener Form mit 58 Val% Natriumhydroxyd in Gegenwart von Wasser bei 80° C emulgiert wird.

** als weiterverdünnbare, homogene, stabile Formulierung des Leimungsmittels, das in geschmolzener Form mit 89 Val% Natriumhydroxyd in Gegenwart von Wasser bei 80° C emulgiert wird.

*** als weiterverdünnbare, homogene, stabile Formulierung des Leimungsmittels, das in Pulverform mit 68 Val% Ammoniak in Gegenwart von Wasser und Glasperlen bei Raumtemperatur (15 bis 25° C) dispergiert wird.

Beispiel 219 und Vergleichsversuch I:

Man verfährt wie in Beispielen 88 bis 109 angegeben, gibt jedoch das Leimungsmittel und das Retentionsmittel separat zur Faserstoffsuspension und setzt kein PERCOL 292® als Hilfsmittel ein. Als Füllmittel werden 20% Kreide zur Faserstoffsuspension gegeben. Die Leimungsmittelzugabe erfolgt 10 Sekunden nach der Kreidezugabe und die Retentionsmittelzugabe 10 Sekunden nach der Leimungsmittelzugabe. Als Leimungsmittel wird eine 35%ige, weiterverdünnbare, homogene und stabile Formulierung des gemäss Beispiel 35 erhaltenen Estergemisches in geschmolzener Form, das mit 58 Val.% Natriumhydroxyd in Gegenwart von Wasser bei 80 °C emulgiert wird. Anstelle einer der Retentionsmittel Nr. 1 bis 3 wird das Retentionsmittel Nr. 8 eingesetzt. Retentionsmittel Nr. 8 ist ein Epichlorhydrin-Addukt eines Poly-N-Methyldiallylamins, das gemäss Beispiel 1 der US-A-4 279 794 hergestellt wird. Die in der nachfolgenden Tabelle XV angegebenen Mengen an Leimungs- und Retentionsmitteln werden eingesetzt, wobei diese Mengen und die Menge Füllmittel (20% Kreide) sich auf Trockensubstanz an Leimungs-, Retentions- und Füllmittel und auf den Feststoffgehalt der Faserstoffsuspension beziehen. Die Leimungsergebnisse sind aus der nachfolgenden Tabelle XV zu entnehmen.

Zur Bestimmung des Weissgrades werden zudem die Remissionswerte gemäss Standardtest T 452 des TAPPI (Technical Association of the Pulp and Paper Industry) gemessen. Je höher die Remissionswerte liegen, desto besser ist der Weissgrad des Papiers.

Tabelle XV

| Nr. Beispiel bzw. Vergleichsversuch | Einsatzmenge Leimungsmittel (%) | Einsatzmenge Retentionsmittel (%) | pH-Wert Suspension | WA $Cobb_{30}$ ($g/m^2$) nach Trocknung sofort | | nach 1 Tag Lagerung | | Weissgrad (Remissionswerte) |
|---|---|---|---|---|---|---|---|---|
| | | | | SS | OS | SS | OS | |
| Beispiel 219 | 0,5 | 0,25 | 8,8 | 17 | 12 | 16 | 11 | 67,9 |
| Vergleichsversuch I | 0 | 0 | – | – | 161 | – | 169 | 67,1 |

Beispiele 220 bis 222 und Vergleichsversuch II:

Man verfährt wie in Beispielen 88 bis 109 angegeben, gibt jedoch das Leimungsmittel und das Retentionsmittel separat zur Faserstoffsuspension und setzt zusätzlich als optische Aufheller 0,075% Diäthanolamin-Salz des 4,4'-Bis[2-(di-β-hydroxyäthylamino)-4-(p-sulfophenylamino)-1,3,5-triazin(6)-yl-amino]-stilben-2,2'-disulfonsäure ein. Als Füllmittel werden 20% Kreide zur Faser-

Tabelle XVI

| Nr. Beispiel bzw. Vergleichsversuch | Einsatzmenge Leimungsmittel (%) | Leimungsmittel | Einsatzmenge Retentionsmittel (%) | pH-Wert Suspension | WA $Cobb_{30}$ (g/m$^2$) nach Trocknung sofort | | nach 1 Tag Lagerung | | nach 2 Wochen Lagerung | Weissgrad (Remissionswerte) |
|---|---|---|---|---|---|---|---|---|---|---|
| | | | | | SS | OS | SS | OS | SS | |
| Beispiel 220 | 0,5 | gem. Beispiel 35* | 0,25 | 9,0 | 22 | 14 | 21 | 12 | 22 | 73,0 |
| Beispiel 221 | 0,5 | gem. Beispiel 45** | 0,25 | 9,0 | 26 | 14 | 24 | 12 | 26 | 72,5 |
| Beispiel 222 | 0,5 | gem. Beispiel 56*** | 0,25 | 9,0 | 21 | 12 | 18 | 10 | 17 | 72,3 |
| Vergleichsversuch II | 0 | – | 0 | – | – | 153 | – | 146 | 147 | 74,4 |

* als weiterverdünnbare, homogene, stabile Formulierung des Leimungsmittels, das in geschmolzener Form mit 58 Val% Natriumhydroxyd in Gegenwart von Wasser bei 80° C emulgiert wird.

** als weiterverdünnbare, homogene, stabile Formulierung des Leimungsmittels, das in geschmolzener Form mit 89 Val% Natriumhydroxyd in Gegenwart von Wasser bei 80° C emulgiert wird.

*** als weiterverdünnbare, homogene, stabile Formulierung des Leimungsmittels, das in Pulverform mit 68 Val% Ammoniak in Gegenwart von Wasser und Glasperlen bei Raumtemperatur (15 bis 25° C) dispergiert wird.

stoffsuspension gegeben. Die Kreidezugabe erfolgt 1 Minute nach der Aufhellerzugabe, die Leimungsmittelzugabe 10 Sekunden nach der Kreidezugabe, die Retentionsmittelzugabe 10 Sekunden nach der Leimungsmittelzugabe und die Zugabe an PERCOL 292® 10 Sekunden nach der Retentionsmittelzugabe. Als Leimungsmittel werden die in der nachfolgenden Tabelle XVI angegebenen Emulsionen oder Dispersionen eingesetzt. Anstelle einer der Retentionsmittel Nr. 1 bis 3 wird das Retentionsmittel Nr. 8 eingesetzt. Die in der nachfolgenden Tabelle XVI angegebenen Mengen an Leimungs- und Retentionsmitteln werden eingesetzt, wobei diese Mengen, die Menge Aufheller (0,075%) und die Menge Füllmittel (20% Kreide) sich auf Trockensubstanz an Aufheller und an Leimungs-, Retentions- und Füllmittel und auf den Feststoffgehalt der Faserstoffsuspension beziehen. Die Leimungsergebnisse sind ebenfalls aus der nachfolgenden Tabelle XVI zu entnehmen.

Zur Bestimmung des Weissgrades werden zudem die Remissionswerte gemäss Standardtest T 452 der TAPPI gemessen.

Vergleichsversuche III bis V

Man verfährt wie in Beispielen 146 bis 150 angegeben, setzt jedoch im Versuch III 0,5% Retentionsmittel Nr. 5 ohne Leimungsmittel, im Versuch IV 0,5% des angegebenen Leimungsmittels ohne Retentionsmittel und im Versuch V weder Leimungsmittel noch Retentionsmittel ein. Nur eine schlechte Leimung des Papiers wird erhalten, wie aus den in der nachfolgenden Tabelle XVII angegebenen WA Cobb-Werte über 100 ersichtlich ist.

Tabelle XVII

| Vergleichs-versuch Nr. | pH-Wert Suspension | WA $Cobb_{30}$ $(g/m^2)$ nach Trocknung sofort SS | OS | nach 1 Tag Lagerung SS | OS |
|---|---|---|---|---|---|
| III | 8,1 | 191 | 154 | 162 | 148 |
| IV | 8,0 | 173 | 146 | 150 | 124 |
| V | 8,1 | 240 | 196 | 197 | 176 |

**Patentansprüche**

1. Verfahren zum Leimen von Papier oder Karton, bei welchem man zu wässrigen, cellulosehaltigen, gegebenenfalls füllmittelhaltigen Faserstoffsuspensionen mindestens
(A) ein Leimungsmittel
und
(B) ein polymeres, kationisches Retentionsmittel in beliebiger Reihenfolge oder gleichzeitig hinzugibt, dadurch gekennzeichnet, dass das Leimungsmittel als anionische oder acide, gegebenenfalls in Salzform vorliegende Gruppe mindestens eine saure Phosphat-, Sulfat-, Carboxyl-, Methylen- oder Methingruppe und mindestens zwei hydrophobe, nur Kohlenstoff- und Wasserstoffatome enthaltende Substituenten mit je 5 bis 22 Kohlenstoffatomen enthält, wobei mindestens einer der hydrophoben Substituenten mindestens 8 Kohlenstoffatome aufweist und mindestens zwei der nächst benachbarten hydrophoben Substituenten miteinander über ein Verbindungsglied verknüpft sind, das mindestens 1 Kohlenstoffatom und 2 Heteroatome aufweist.

2. Verfahren nach Anspruch 1, dadurch gekennzeichnet, dass man als Komponente (A) ein Leimungsmittel einsetzt, dessen hydrophobe Substituenten je 8 bis 22 Kohlenstoffatome aufweisen.

3. Verfahren nach einem der Ansprüche 1 oder 2, dadurch gekennzeichnet, dass man als Komponente (A) ein Leimungsmittel einsetzt, das 1 bis 6 saure Phosphat-, Sulfat- oder Carboxylgruppen und 2 bis 10 hydrophobe Substituenten enthält, wobei mindestens zwei der nächst benachbarten hydrophoben Substituenten über 1 bis 5 Verbindungsglieder mit je 4 bis 15 Kohlenstoffatomen und mindestens zwei Sauerstoff- und/oder Stickstoffatomen verknüpft sind.

4. Verfahren nach Anspruch 3, dadurch gekennzeichnet, dass man als Komponente (A) ein Leimungsmittel einsetzt, das durch Umsetzung von mindestens

(a) einem aliphatischen Alkohol mit 3 bis 26 Kohlenstoffatomen und 2 bis 6 Hydroxyl- oder Hydroxy-$C_1$–$C_4$-alkylgruppen, der gegebenenfalls 1 bis 5 Stickstoffatome und bei Anwesenheit von 2 Hydroxylgruppen gegebenenfalls einen $C_6$–$C_{22}$-Fettaminrest aufweist, einem heterocyclischen Alkohol oder Glycid mit 3 Stickstoffatomen im Heteroring und 3 Hydroxy-$C_1$–$C_4$-alkyl- oder Glycidgruppen, einem Alkandioldiglycid mit 2 bis 6 Kohlenstoffatomen im Alkanrest, einem Di- oder Triphenol oder einem Dihydroxynaphthalin, mit

(b) einer gegebenenfalls ungesättigten Fettsäure, deren Halogenide oder einem primären oder sekundären Fettamin mit 6 bis 22 Kohlenstoffatomen im Fettrest und hierauf mit

(c) einer mehrbasischen anorganischen oder organischen Säure mit 2 bis 18 Kohlenstoffatomen oder deren Anhydriden
erhältlich ist.

5. Verfahren nach Anspruch 4, dadurch gekennzeichnet, dass man als Komponente (A) ein Leimungsmittel einsetzt, das durch Umsetzung von

(a) 1,5-, 1,8-, 2,3- oder 2,7-Dihydroxynaphthalin,
Hydroxyhydrochinon, Pyrogallol, Phloroglucin, Hydrochinon, Brenzcatechin, Resorcin,

Tris(hydroxyäthyl)-isocyanurat,
Isocyanursäuretriglycid,
Butan-1,4-dioldiglycid, Glycerin,
Butan-1,2,4-triol, Pentaerythrit, Sorbit,
Sorbitan, Triäthanolamin,
einem $C_8$–$C_{22}$-Fettamin-diäthoxylat oder einem Trialkanolamin,
N,N,N′,N′-Tetrakis(2-hydroxypropyl)-äthylendiamin oder
Tris(hydroxymethyl)-aminomethan, mit

(b) einer Fettsäure mit 16 bis 20 Kohlenstoffatomen, einem Alkyl- oder Alkenylhalogenid, einem Mono- oder Dialkylamin oder einem Mono- oder Dialkenylamin mit je 16 bis 20 Kohlenstoffatomen im Alkyl- oder Alkenylrest und hierauf mit

(c) Schwefeltrioxyd, Schwefelsäure, Chlorsulfonsäure, Phosphorsäure, Phosphorpentoxyd, Phthalsäureanhydrid, Bernsteinsäureanhydrid oder Maleinsäureanhydrid
erhältlich ist.

6. Verfahren nach einem der Ansprüche 1 oder 2, dadurch gekennzeichnet, dass man als Komponente (A) ein Leimungsmittel einsetzt, das 1 bis 4 saure Phosphor-, Sulfat- oder Carboxylgruppen und 2 bis 5 hydrophobe Substituenten enthält, wobei mindestens zwei der nächst benachbarten hydrophoben Substituenten über 1 bis 5 Verbindungsglieder mit je 1 bis 15 Kohlenstoffatomen und je 2 bis 6 Stickstoffatomen verknüpft sind.

7. Verfahren nach Anspruch 6, dadurch gekennzeichnet, dass man als Komponente (A) ein Leimungsmittel einsetzt, welches durch Umsetzung von mindestens

(a′) einem gegebenenfalls mit N–$C_6$–$C_{22}$-Alkenyl- oder Alkyl monosubstituierten Polyalkylenpolyamin mit 3 bis 6 Stickstoffatomen und 4 bis 40 Kohlenstoffatomen, mit

(b′) einer Fettsäure oder einem Alkyl- oder Alkenylisocyanat mit 6 bis 22 Kohlenstoffatomen, und hierauf mit

(c′) dem Anhydrid einer mehrbasischen, anorganischen oder organischen Säure mit 2 bis 18 Kohlenstoffatomen, einer α- oder β-Halogencarbonsäure mit 2 bis 6 Kohlenstoffatomen oder einem Sulton
erhältlich ist.

8. Verfahren nach Anspruch 7, dadurch gekennzeichnet, dass man als Komponente (A) ein Leimungsmittel einsetzt, das durch Umsetzung von mindestens

(a′) N,N′-Bis(3-aminopropyl)-1,4-diaminobutan, N-(3-aminopropyl)-1,4-diamino-butan,
1,2-Bis(3-aminopropyl-amino)-äthan,
Pentaäthylen-hexamin, Tetraäthylenpentamin,
Triäthylentetramin, Dipropylen-triamin oder
Diäthylentriamin, mit

(b′) Palmitin-, Stearin-, Öl- oder Behensäure, oder Octadecisocyanat, und hierauf mit

(c′) 2-Chlorbuttersäure,
3- oder 2-Chlorpropionsäure, Bromessigsäure,
Chloressigsäure oder deren Alkalimetallsalzen,
Propansulton, Benzophenontetracarbonsäure,
1,8-Naphthalsäure, Tri- oder Pyromellithsäure,
Norbornendicarbonsäure,
Hexa- oder Tetrahydrophthalsäure-, Phthal-,
Bernstein-, Glutar-, Malein-, Dimethylmalein-,
Citracon- oder Itaconsäureanhydrid
erhältlich ist.

9. Verfahren nach einem der Ansprüche 1 oder 2, dadurch gekennzeichnet, dass man als Komponente (A) ein Leimungsmittel einsetzt, das 1 oder 2 acide Methylen- oder Methingruppen und 2 oder 3 hydrophobe Substituenten enthält, wobei mindestens zwei der nächst benachbarten hydrophoben Substituenten über 1 oder 2 Verbindungsglieder mit je 1 bis 15 Kohlenstoffatomen und je 2 bis 4 Stickstoff- und/oder Sauerstoffatomen verknüpft sind und die Methylen- oder Methingruppen als zwei- oder dreiwertige Reste $-CO-CH_2-CO-$ oder $-CO-\overset{|}{C}H-CO-$ vorliegen.

10. Verfahren nach Anspruch 9, dadurch gekennzeichnet, dass man als Komponente (A) ein Leimungsmittel einsetzt, welches durch Umsetzung von

($a_1''$) Malonsäure, einem Malonsäuredihalogenid, Acetondicarbonsäure oder einem $C_1$–$C_4$-Alkylester der Malonsäure, Acetondicarbonsäure oder Methantricarbonsäure, mit

($b_1''$) einem Fettalkohol
oder von

($a_2''$) Cyanessigsäure oder deren $C_1$–$C_4$-Alkylester, mit

($b_2''$) einem Fettamin und hierauf mit

($c''$) einem $C_4$–$C_{12}$-Alkylen-, $C_6$–$C_{14}$-Cycloalkylen- oder $C_6$–$C_{14}$-Arylendiisocyanat
erhältlich ist.

11. Verfahren nach Anspruch 10, dadurch gekennzeichnet, dass man als Komponente (A) ein Leimungsmittel einsetzt, das durch Umsetzung von ($a_1''$) Malonsäuredichlorid, dem Dimethyl- oder Diäthylester der Malonsäure oder der Acetondicarbonsäure oder dem Trimethyl- oder Triäthylester der Methantricarbonsäure, mit

($b_1''$) einem gegebenenfalls ungesättigten, aliphatischen Alkohol mit 8 bis 22 Kohlenstoffatomen
erhältlich ist.

12. Verfahren nach Anspruch 10, dadurch gekennzeichnet, dass man als Komponente (A) ein Leimungsmittel einsetzt, das durch Umsetzung von

($a_2''$) Cyanessigsäure-methylester oder -äthylester, mit

($b_2''$) einem Mono- oder Dialkylamin oder einem Mono- oder Dialkenylamin mit 8 bis 22 Kohlenstoffatomen im Alkyl- oder Alkenylrest und hierauf mit

($c''$) Butylendiisocyanat, Dodecylendiisocyanat,
Decylen-1,10-diisocyanat,
Hexylen-1,6-diisocyanat,
Cyclohexyl- oder Dicyclohexyldiisocyanat,
Naphthylen-1,5-diisocyanat,
Diphenyl-methan-4,4′-diisocyanat,
Phenylen-1,4-diisocyanat oder
Toluylen-2,4- oder -2,6-diisocyanat
erhältlich ist.

13. Verfahren nach einem der Ansprüche 1 oder 2, dadurch gekennzeichnet, dass man als Komponente (A) ein Leimungsmittel einsetzt, das 1 bis 6 Gruppen $-COO^{\ominus}$ oder $-COOH$ als anionische oder

acide Gruppen und 2 bis 10 hydrophobe Substituenten enthält, wobei mindestens zwei der nächst benachbarten hydrophoben Substituenten über 1 bis 5 Verbindungsglieder mit je 4 bis 15 Kohlenstoffatomen und je 1 Sauerstoff- und 1 Stickstoffatom oder je 2 Sauerstoff- oder Stickstoffatomen verknüpft sind.

14. Verfahren nach Anspruch 13, dadurch gekennzeichnet, dass man als Komponente (A) ein Leimungsmittel einsetzt, welches durch Umsetzung von mindestens

(a''') einer aliphatischen oder aromatischen Carbonsäure mit mindestens 3 Carboxylgruppen, einer Polyalkylenpolyamino-polyessigsäure mit 4 bis 6 Carboxylgruppen oder einer aliphatischen Monocarbonsäure oder Aminomonocarbonsäure mit 2 Hydroxylgruppen, mit

(b''') Fettalkoholen und/oder Fettaminen, oder, sofern eine Monocarbonsäure eingesetzt wird, Fettsäuren

erhältlich sind.

15. Verfahren nach Anspruch 14, dadurch gekennzeichnet, dass man als Komponente (A) ein Leimungsmittel einsetzt, das durch Umsetzung von

(a''') Hemimellith-, Trimellith-, Trimesin-, Prehnit-, Mellophan-, Pyromellith-, Mellithsäure, Benzolpentacarbonsäure, Benzophenon-tri- bis -hexacarbonsäure oder deren Anhydride, Tricarballyl-, Aconit- oder Citronensäure, Bis(2-carboxyäthyl)-carboxymethylamin, Nitrilotriproprionsäure, Nitrilotriessigsäure, Glycerinsäure, 1,2-Dihydroxybuttersäure, 1,3- und 2,3-Dihydroxyvaleriansäure, 2,2-Bis(hydroxymethyl)-propionsäure, N,N-Bis-(hydroxyäthyl)-β-alanin oder N,N-Bis(hydroxyäthyl)-glycin, mit

(b''') einem gegebenenfalls ungesättigten, aliphatischen Alkohol mit 8 bis 22 Kohlenstoffatomen und/oder einem Mono- oder Dialkylamin oder einem Mono- oder Dialkenylamin mit jeweils 8 bis 22 Kohlenstoffatomen im Alkyl- oder Alkenylrest oder, sofern eine Monocarbonsäure als Komponente (a''') verwendet wird, einer Fettsäure mit 8 bis 22 Kohlenstoffatomen

erhältlich ist.

16. Verfahren nach einem der Ansprüche 1 bis 15, dadurch gekennzeichnet, dass man als Komponente (A) ein Leimungsmittel einsetzt, das mindestens teilweise als Salz vorliegt und durch zusätzliche Umsetzung mit einem Alkalimetallhydroxyd, Ammoniak oder einem Alkyl- oder Alkanolamin mit höchstens 6 Kohlenstoffatomen, erhältlich ist.

17. Verfahren nach einem der Ansprüche 1 bis 16, dadurch gekennzeichnet, dass man 0,02 bis 3 Gewichtsprozent Leimungsmittel (A) und 0,02 bis 3 Gewichtsprozent Retentionsmittel (B), bezogen jeweils auf Trockensubstanz an (A) und an (B) und auf den Feststoffgehalt der Faserstoffsuspension, einsetzt.

**Claims**

1. A process for sizing paper or cardboard, in which at least

(A) a sizing agent

and

(B) a polymeric cationic retention aid

are added in any order or simultaneously to an aqueous cellulose-containing fibre dispersion which can, if desired, also contain a filler, wherein the sizing agent contains as an anionic or acidic group which can be present in salt form at least one acid phosphate, sulfate, carboxyl, methylene or methine group and at least two hydrophobic substituents which contain only carbon and hydrogen atoms and have 5 to 22 carbon atoms each, at least one of the hydrophobic substituents having at least 8 carbon atoms and at least two of the most closely adjacent hydrophobic substituents being bonded to each other via a linking member which has at least 1 carbon atom and 2 hetero atoms.

2. A process according to Claim 1, wherein component (A) is a sizing agent whose hydrophobic substituents each have 8 to 22 carbon atoms.

3. A process according to either one of Claims 1 or 2, wherein component (A) is a sizing agent which contains 1 to 6 acid phosphate, sulfate or carboxyl groups and 2 to 10 hydrophobic substituents, at least two of the most closely adjacent hydrophobic substituents being bonded to each other via 1 to 5 linking members each having 4 to 15 carbon atoms and at least two oxygen and/or nitrogen atoms.

4. A process according to Claim 3, wherein component (A) is a sizing agent which is obtainable by reaction of at least

(a) an aliphatic alcohol having 3 to 26 carbon atoms and 2 to 6 hydroxyl or hydroxy-$C_1$–$C_4$-alkyl groups which can have 1 to 5 nitrogen atoms and, in the presence of 2 hydroxyl groups, can have a $C_6$–$C_{22}$-fatty amine radical, a heterocyclic alcohol or glycide having 3 nitrogen atoms in the hetero ring and 3-hydroxy-$C_1$–$C_4$-alkyl or glycide groups, an alkanediol diglycide having 2 to 6 carbon atoms in the alkane radical, a di- or triphenol or a dihydroxy-naphthalene with

(b) a saturated or unsaturated fatty acid, halides thereof or a primary or secondary fatty amine having 6 to 22 carbon atoms in the fatty acid radical and thereafter with

(c) a polybasic inorganic or organic acid having 2 to 18 carbon atoms or anhydrides thereof.

5. A process according to Claim 4, wherein component

(A) is a sizing agent which is obtainable by reaction of

(a) 1,5-, 1,8-, 2,3- or 2,7-dihydroxy-naphthalene,

hydroxyhydroquinone, pyrogallol, phloroglucinol, hydroquinone, pyrocatechol, resorcinol,

tris(hydroxyethyl) isocyanurate, isocyanuric triglycide,

butane-1,4-diol diglycide, glycerol,

butane-1,2,4-triol, pentaerythritol, sorbitol,

sorbitan, triethanolamine,

a $C_8$–$C_{22}$-fatty amine diethoxylate or a trialkanolamine,

N,N,N',N'-tetrakis(2-hydroxypropyl)ethylenediamine or
tris(hydroxymethyl)aminomethane with

(b) a fatty acid having 16 to 20 carbon atoms, an alkyl or alkenyl halide, a mono- or dialkylamine or a mono- or dialkenylamine each having 16 to 20 carbon atoms in the alkyl or alkenyl radical and thereafter with

(c) sulfur trioxide, sulfuric acid, chlorosulfonic acid, phosphoric acid, phosphorus pentoxide, phthalicanhydride, succinic anhydride or maleic anhydride.

6. A process according to either of Claims 1 or 2, wherein component (A) is a sizing agent which contains 1 to 4 acid phosphorus, sulfate or carboxyl groups and 2 to 5 hydrophobic substituents, at least two of the most closely adjacent hydrophobic substituents being bonded to each other via 1 to 5 linking members each having 1 to 15 carbon atoms and 2 to 6 nitrogen atoms.

7. A process according to Claim 6, wherein component (A) is a sizing agent which is obtainable via reaction of at least

(a′) a polyalkylenepolyamine which can be monosubstituted by N-$C_6$-$C_{22}$-alkenyl or alkyl and has 3 to 6 nitrogen atoms and 4 to 40 carbon atoms which

(b′) a fatty acid or an alkyl or alkenyl isocyanate having 6 to 22 carbon atoms, and thereafter with

(c′) the anhydride of a polybasic inorganic or organic acid having 2 to 18 carbon atoms, an α- or β-halocarboxylic acid having 2 to 6 carbon atoms or a sultone.

8. A process according to Claim 7, wherein component (A) is a sizing agent which is obtainable by reaction of at least

(a′) N,N′-bis(3-aminopropyl)-1,4-diaminobutane,
N-(3-aminopropyl)-1,4-diaminobutane,
1,2-bis(3-aminopropylamino)-ethane,
pentaethylenehexamine, tetraethylenepentamine,
triethylenetetramine, dipropylenetriamine or
diethylenetriamine with

(b′) palmitic, stearic, oelic or behenic acid or octadecyl isocyanate, and thereafter with

(c′) 2-chlorobutyric acid,
3- or 2-chloropropionic acid,
bromoacetic acid,
chloroacetic acid or alkali metal salts thereof,
propanesultone, benzophenonetetracarboxylic acid,
1,8-naphthalic acid, trimellitic acid,
pyromellitic acid, norbornenedicarboxylic acid,
hexahydrophthalic acid,
tetrahydrophthalic acid or phalic,
succinic, glutaric, maleic, dimethylmaleic,
citraconic or itaconic anhydride.

9. A process according to either of Claims 1 or 2, wherein component (A) is a sizing agent which contains 1 or 2 acidic methylene or methine groups and 2 or 3 hydrophobic substituents, at least two of the most closely adjacent hydrophobic substituents being bonded to each other via 1 or 2 linking members having 1 to 15 carbon atoms each and 2 to 4 hydrogen and/or oxygen atoms each and the methylene or methine groups being present as divalent or trivalent radicals of the formula $-CO-CH_2-CO-$ or $-CO-\overset{|}{C}H-CO-$.

10. A process according to Claim 9, wherein component

(A) is a sizing agent which is obtainable by reaction of

($a_1''$) malonic acid, a malonyl dihalide, acetonedicarboxylic acid or a $C_1$-$C_4$-alkyl ester of malonic acid, acetonedicarboxylic or methanetricarboxylic acid with

($b_1''$) a fatty alcohol

or of

($a_2''$) cyanoacetic acid or a $C_1$-$C_4$-alkyl ester thereof with

($b_2''$) a fatty amine and thereafter with

($c''$) a $C_4$-$C_{12}$-alkylene diisocyanate, $C_6$-$C_{14}$-cycloalkylene diisocyanate or $C_6$-$C_{14}$-arylene diisocyanate.

11. A process according to Claim 10, wherein component

(A) is a sizing agent which is obtainable by reaction of

($a_1''$) malonyl dichloride, the dimethyl or diethyl ester of malonic acid or of acetone dicarboxylic acid or the trimethyl or triethyl ester of methanetricarboxylic acid with

($b_1''$) a saturated or unsaturated aliphatic alcohol having 8 to 22 carbon atoms

12. A process according to Claim 10, wherein component

(A) is a sizing agent which is obtainable by reaction of

($a_2''$) methyl or ethyl cyanoacetate with

($b_2''$) a mono- or dialkylamine or a mono- or dialkenylamine each having 8 to 22 carbon atoms in the alkyl or alkenyl radical and thereafter with

($c''$) butylene diisocyanate,
dodecylene diisocyanate, decylene, 1,10-diisocyanate,
hexylene 1,6-diisocyanate, cyclohexyl or dicyclohexyl diisocyanate,
naphthalene 1,5-diisocyanate,
diphenylmethane 4,4′-diisocyanate,
phenylene 1,4-diisocyanate or toluylene 2,4- or 2,6-diisocyanate.

13. A process according to either of Claims 1 or 2, wherein component (A) is a sizing agent which contains 1 to 6 $-COO^{\ominus}$ or $-COOH$ groups as anionic or acidic groups and 2 to 10 hydrophobic substituents, at least two of the most closely adjacent hydrophobic substituents being bonded to each other via 1 to 5 linking members having 4 to 15 carbon atoms each and 1 oxygen and 1 nitrogen atom each or 2 oxygen or 2 nitrogen atoms each.

14. A process according to Claim 13, wherein component

(A) is a sizing agent which is obtainable by reaction of at least

($a'''$) an aliphatic or aromatic carboxylic acid having at least 3 carboxyl groups, a polyalkylenepolyaminopolyacetic acid having 4 to 6 carboxyl groups or an aliphatic monocarboxylic acid or aminomonocarboxylic acid having 2 hydroxyl groups with

(b‴) fatty alcohols and/or fatty amines or, if a monocarboxylic acid is used, fatty acids.

15. A process according to Claim 14, wherein component

(A) is a sizing agent obtainable by reaction of

(a‴) hemimellitic, trimellitic, trimesinic, prehnitic, mellophanic, pyromellitic, mellitic or benzenepentacarboxylic acid, benzophenonetricarboxylic to benzophenone-hexacarboxylic acid or anhydrides thereof, tricarballylic, aconitic or citric acid, bis(2-carboxyethyl)-carboxymethylamine, nitrilotripropionic acid, nitrilotriacetic acid, glyceric acid, 1,2-dihydroxy-butyric acid, 1,3- and 2,3-hydroxyvaleric acid, 2,2-bis(hydroxymethyl)-propionic acid, N,N-bis-(hydroxyethyl)-β-alanine or N,N-bis(hydroxyethyl)-glycine with

(b‴) a saturated or unsaturated aliphatic alcohol having 8 to 22 carbon atoms and/or a mono- or dialkylamine or a mono- or dialkenylamine having in each case 8 to 22 carbon atoms in the alkyl or alkenyl radical or, if a monocarboxylic acid is used as component (a‴), a fatty acid having 8 to 22 carbon atoms.

16. A process according to any one of Claims 1 to 15, wherein component (A) is a sizing agent which is at least partly present in salt form and is obtainable by additional reaction with an alkali metal hydroxide, ammonia or an alkylamine or alkanolamine having at most 6 carbon atoms.

17. A process according to any one of Claims 1 to 16, wherein 0.02 to 3 percent by weight of sizing agent (A) and 0.02 to 3 percent by weight of retention aids (B), in each case based on dry substance in (A) and in (B) and on the solids content of the fibre dispersion are used.

**Revendications**

1. Procédé pour l'encollage du papier ou du carton, dans lequel on ajoute, dans un ordre quelconque ou simultanément, à des suspensions aqueuses de matières fibreuses cellulosiques, contenant éventuellement des charges, au moins

(A) un agent d'encollage et

(B) un agent de rétention polymère cationique, caractérisé par le fait que l'agent d'encollage contient, en tant que groupe anionique ou acide, se trouvant éventuellement sous forme de sel, au moins un groupe acide phosphate, sulfate, carboxyle, méthylène ou méthine, et au moins deux substituants hydrophobes, ne contenant que des atomes de carbone et d'hydrogène, ayant chacun de 5 à 22 atomes de carbone, au moins un des substituants hydrophobes comportant au moins 8 atomes de carbone, et au moins deux des substituants hydrophobes immédiatement voisins étant reliés entre eux par un chaînon de liaison qui comporte au moins 1 atome de carbone et 2 hétéroatomes.

2. Procédé selon la revendication 1, caractérisé par le fait que l'on utilise, en tant que composant (A), un agent d'encollage dont les substituants hydrophobes comportent chacun de 8 à 22 atomes de carbone.

3. Procédé selon l'une des revendications 1 ou 2, caractérisé par le fait que l'on utilise, en tant que composant (A), un agent d'encollage qui contient de 1 à 6 groupes phosphate acide, sulfate acide ou carboxyle et de 2 à 10 substituants hydrophobes, au moins deux des substituants hydrophobes immédiatement voisins étant reliés par 1 à 5 chaînons de liaisons ayant chacun de 4 à 15 atomes de carbone et au moins 2 atomes d'oxygène et/ou d'azote.

4. Procédé selon la revendication 3, caractérisé par le fait que l'on utilise, en tant que composant (A), un agent d'encollage qui peut être obtenu par réaction d'au moins

(a) un alcool aliphatique ayant de 3 à 26 atomes de carbone et de 2 à 6 groupes hydroxyle ou hydroxyalkyle en $C_1$–$C_4$, qui comporte éventuellement 1 à 5 atomes d'azote et, dans le cas de la présence de 2 groupes hydroxyle, éventuellement un reste d'amine grasse en $C_6$–$C_{22}$; un alcool ou glycide hétérocyclique comportant 3 atomes d'azote dans l'hétérocycle et 3 groupes hydroxyalkyle en $C_1$–$C_4$ ou glycide; un alcanediol-diglycide ayant de 2 à 6 atomes de carbone dans le radical alcane; un di- ou triphénol ou un dihydroxynaphtalène; avec

(b) un acide gras, éventuellement insaturé, des halogénures de celui-ci ou une amine grasse primaire ou secondaire ayant de 6 à 22 atomes de carbone dans le radical aliphatique; et ensuite avec

(c) un polyacide minéral ou organique ayant de 2 à 18 atomes de carbone, ou des anhydrides de celui-ci.

5. Procédé selon la revendication 4, caractérisé par le fait que l'on utilise, en tant que composant (A), un agent d'encollage qui peut être obtenu par réaction

(a) du 1,5-, 1,8-, 2,3- ou 2,7-dihydroxy-naphtalène,
de l'hydroxyhydroquinone, du pyrogallol,
du phloroglucinol, de l'hydroquinone,
du pyrocatéchol, du résorcinol,
du tris(hydroxyéthyl)-isocyanurate,
de l'isocyanurotriglycide,
du butane-1,4-dioldiglycide, du glycérol,
du butane-1,2,4-triol, du pentaérythritol,
du sorbitol, du sorbitanne,
de la triéthanolamine,
d'une trialcanolamine ou d'un diéthoxylate d'amine grasse en $C_6$–$C_{22}$,
de la N,N,N′,N′-tétrakis(2-hydroxypropyl)-éthylènediamine ou du tris(hydroxyméthyl)-aminométhane, avec

(b) un acide gras ayant de 16 à 20 atomes de carbone, un halogénure d'alkyle ou d'alcényle, une mono- ou dialkylamine ou une mono- ou dialcénylamine ayant chacune de 16 à 20 atomes de carbone dans le radical alkyle ou alcényle, et ensuite avec

(c) l'anhydride sulfurique, l'acide sulfurique, l'acide chlorosulfonique, l'acide phosphorique, le

pentoxyde de phosphore, l'anhydride phtalique, l'anhydride succinique ou l'anhydride maléique.

6. Procédé selon l'une des revendications 1 ou 2, caractérisé par le fait que l'on utilise, en tant que composant (A), un agent d'encollage qui contient de 1 à 4 groupes acides phosphate, sulfate ou carboxyle, et 2 à 5 substituants hydrophobes, au moins 2 des substituants hydrophobes immédiatement voisins étant reliés par 1 à 5 chaînons de liaison ayant chacun de 1 à 15 atomes de carbone et chacun 2 à 6 atomes d'azote.

7. Procédé selon la revendication 6, caractérisé par le fait que l'on utilise, en tant que composant (A), un agent d'encollage qui peut être obtenu par réaction d'au moins

(a') une polyalkylènepolyamine ayant de 3 à 6 atomes d'azote et de 4 à 40 atomes de carbone, éventuellement substituée par un seul groupe N-alkyle en $C_6$–$C_{22}$ ou N-alkylène en $C_6$–$C_{22}$, avec

(b') un acide gras ou un alkyl- ou alkylène-isocyanate ayant de 6 à 22 atomes de carbone, et ensuite avec

(c') l'anhydride d'un polyacide organique ou minéral ayant de 2 à 18 atomes de carbone, d'un acide α- ou β-halogénocarboxylique ayant de 2 à 6 atomes de carbone, ou une sultone.

8. Procédé selon la revendication 7, caractérisé par le fait que l'on utilise, en tant que composant (A), un agent d'encollage qui peut être obtenu par réaction d'au moins

(a') le N,N'-bis-(3-aminopropyl)-1,4-diaminobutane,
le N-(3-aminopropyl)-1,4-diaminobutane,
le 1,2-bis(3-aminopropylamino)éthane,
la pentaéthylène-hexamine,
la tétraéthylène-pentamine,
la triéthylènetétramine,
la dipropylènetriamine ou la diéthylène-triamine, avec

(b') l'acide palmitique, stéarique, oléique ou béhénique, ou l'octadécylisocyanate, et ensuite avec

(c') l'acide 2-chlorobutyrique,
l'acide 3- ou 2-chloropropionique,
l'acide bromacétique,
l'acide chloracétique ou leurs sels alcalins,
la propanesultone,
l'acide benzophénonetétracarboxylique,
l'acide 1,8-naphtalique,
l'acide tri- ou pyromellitique,
l'acide norbornènedicarboxylique,
l'anhydride d'acide hexa- ou tétra-hydrophtalique,
phtalique, succinique, glutarique, maléique, diméthylmaléique, citraconique ou itaconique.

9. Procédé selon l'une des revendications 1 ou 2, caractérisé par le fait que l'on utilise, en tant que composant (A), un agent d'encollage qui contient 1 ou 2 groupes méthylène ou méthine acides et 2 ou 3 substituants hydrophobes, au moins deux des substituants hydrophobes immédiatement voisins étant reliés par 1 ou 2 chaînons de liaison ayant chacun 1 à 15 atomes de carbone et chacun 2 à 4 atomes d'azote et/ou atomes d'oxygène, et les groupes méthylène ou méthine se trouvant sous forme de restes bi- ou trivalents

$$-CO-CH_2-CO- \text{ ou } -CO-\overset{|}{C}H-CO-.$$

10. Procédé selon la revendication 9, caractérisé par le fait que l'on utilise, en tant que composant (A), un agent d'encollage qui peut être obtenu par réaction

($a_1''$) de l'acide malonique, d'un dihalogénure de l'acide malonique, de l'acide acétonedicarboxylique ou d'un ester alkylique en $C_1$–$C_4$ de l'acide malonique ou acétonedicarboxylique, ou de l'acide méthanetricarboxylique, avec

($b_1''$) un alcool gras,

ou

($a_2''$) de l'acide cyanoacétique ou d'un ester alkylique en $C_1$–$C_4$ de celui-ci, avec

($b_2''$) une amine grasse, et ensuite avec

($c''$) un alkylène($C_4$–$C_{12}$)-, cycloalkylène($C_6$–$C_{14}$)- ou arylène($C_6$–$C_{14}$)-diisocyanate.

11. Procédé selon la revendication 10, caractérisé par le fait que l'on utilise, en tant que composant (A), un agent d'encollage qui peut être obtenu par réaction

($a_1''$) du dichlorure de l'acide malonique, de l'ester diméthylique ou diéthylique de l'acide malonique ou de l'acide acétonedicarboxylique, ou de l'ester triméthylique ou triéthylique de l'acide méthanetricarboxylique, avec

($b_1''$) un alcool aliphatique, éventuellement insaturé, ayant de 8 à 22 atomes de carbone.

12. Procédé selon la revendication 10, caractérisé par le fait que l'on utilise, en tant que composant (A), un agent d'encollage qui peut être obtenu par réaction

($a_2''$) du cyanoacétate de méthyle ou d'éthyle avec

($b_2''$) une mono- ou dialkylamine ou une mono- ou dialcénylamine ayant de 8 à 22 atomes de carbone dans le reste alkyle ou alcényle, et ensuite avec

($c''$) le butylènediisocyanate,
dodécylènediisocyanate,
décylène-1,10-diisocyanate,
hexylène-1,6-diisocyanate,
cyclohexyl- ou dicyclohexyl-diisocyanate,
naphtylène-1,5-diisocyanate,
diphénylméthane-4,4'-diisocyanate,
phénylène-1,4-diisocyanate ou tolylène-2,4- ou -2,6-diisocyanate.

13. Procédé selon l'une des revendications 1 ou 2, caractérisé par le fait que l'on utilise, en tant que composant (A), un agent d'encollage qui contient 1 à 6 groupes $-COO^{\ominus}$ ou $-COOH$ en tant que groupes anioniques ou acides, et 2 à 10 substituants hydrophobes, au moins deux des substituants hydrophobes immédiatement voisins étant reliés par 1 à 5 chaînons de liaison ayant chacun 4 à 15 atomes de carbone et chacun 1 atome d'oxygène et 1 atome d'azote, ou chacun 2 atomes d'oxygène ou d'azote.

14. Procédé selon la revendication 13, caractérisé par le fait que l'on utilise, en tant que composant (A), un agent d'encollage qui peut être obtenu par réaction d'au moins

(a''') un acide carboxylique aliphatique ou aromatique comportant au moins 3 groupes carboxyle, un acide polyalkylènepolyamino-polyacétique ayant de 4 à 6 groupes carboxyle, ou un acide monocarboxylique ou aminomonocarboxylique aliphatique ayant 2 groupes hydroxyle, avec

(b''') des alcools gras et/ou des amines grasses, ou dans la mesure où on utilise un acide monocarboxylique, des acides gras.

15. Procédé selon la revendication 14, caractérisé par le fait que l'on utilise, en tant que composant (A), un agent d'encollage qui peut être obtenu par réaction de

(a''') l'acide hémimellitique, trimellitique, trimésique, prehnique, mellophanique, pyromellitique, mellitique, benzènepentacarboxylique, benzophéno-tri- à hexa-carboxylique ou leurs anhydrides,
l'acide tricarballylique, aconitique ou citrique,
la bis(2-carboxyéthyl)-carboxyméthylamine,
l'acide nitrilotripropionique,
l'acide nitrilotriacétique, l'acide glycérique,
l'acide 1,2-dihydroxybutyrique,
l'acide 1,3- ou 2,3-dihydroxyvalérianique,
l'acide 2,2-bis(hydroxyméthyl)-propionique,
la N,N-bis(hydroxyéthyl)-β-alanine ou la N,N-bis(hydroxyéthyl)-glycine, avec

(b''') un alcool aliphatique éventuellement insaturé, ayant de 8 à 22 atomes de carbone, et/ou une mono- ou dialkylamine ou une mono- ou dialcénylamine ayant chacune de 8 à 22 atomes de carbone dans le radical alkyle ou alcényle, ou dans la mesure où on utilise un acide monocarboxylique en tant que composant (a'''), un acide gras ayant de 8 à 22 atomes de carbone.

16. Procédé selon l'une des revendications 1 à 15, caractérisé par le fait que l'on utilise, en tant que composant (A), un agent d'encollage qui se trouve au moins en partie sous forme de sel et qui peut être obtenu par réaction additionnelle avec un hydroxyde d'un métal alcalin, l'ammoniac ou une alkyl- ou alcanolamine ayant 6 atomes de carbone au maximum.

17. Procédé selon l'une des revendications 1 à 16, caractérisé par le fait que l'on utilise de 0,02 à 3% en poids d'agent d'encollage (A) et de 0,02 à 3% en poids d'agent de rétention (B), exprimé en tant que matière sèche, respectivement, de (A) et de (B), et par rapport à la teneur en matières solides de la suspension de matières fibreuses.